# EUROPEAN PATENT APPLICATION

(11) **EP 2 883 953 A1**
(43) Date of publication of application: **17.06.2015**
(21) Application number: 13196924.8
(22) Date of filing: 12.12.2013
(51) Int. Cl.: C12N 9/22, C12N 15/10

(54) **An atypical naturally split intein engineered for highly efficient protein modification**

(71) Applicant: Westfälische Wilhelms-Universität Münster, 48149 Münster (DE); YEDA RESEARCH AND DEVELOPMENT CO. LTD., Rehovot 76100 (IL)
(72) Inventor: Mootz, Henning Dieter, 48149 Münster (DE); Thiel, Ilka, 48149 Münster (DE); Volkmann, Gerrit, 48149 Münster (DE); Pietrokovsi, Shmuel, 7610001 Rehovot (IL); Bachmann, Anne-Lena, 48149 Münster (DE)
(74) Representative: Viering, Jentschura & Partner Patent- und Rechtsanwälte

(57) **Abstract**

The present invention relates to an isolated polypeptide comprising at least one intein or at least one intein fragment of said intein, wherein said intein is a naturally split intein with a N-terminal intein fragment split after 14-60 amino acids from the intein's N-terminal end and methods of using the same.

SEQUENCE LISTING

<110>  Westfälische-Wilhelms-Universität Münster

<120>  atypical naturally split intein engineered for highly efficient
       protein modification

<130>  P55084

<160>  196

<170>  PatentIn version 3.5

<210>  1
<211>  129
<212>  PRT
<213>  Artificial Sequence

<220>
<223>  AceL-TerL-11

<400>  1

Cys Val Tyr Gly Asp Thr Met Val Glu Thr Glu Asp Gly Lys Ile Lys
1               5                   10                  15

Ile Glu Asp Leu Tyr Lys Arg Leu Ala Met Phe Arg Thr Asn Thr Asn
            20                  25                  30

Asn Ile Lys Ile Leu Ser Pro Asn Gly Phe Ser Asn Phe Asn Gly Ile
        35                  40                  45

Gln Lys Val Glu Arg Asn Leu Tyr Gln His Ile Ile Phe Asp Asp Asp
    50                  55                  60

Thr Glu Ile Lys Thr Ser Ile Asn His Pro Phe Gly Lys Asp Lys Ile
65                  70                  75                  80

Leu Ala Arg Asp Val Lys Val Gly Asp Tyr Leu Asn Ser Lys Lys Val
                85                  90                  95

Leu Tyr Asn Glu Leu Val Asn Glu Asn Ile Phe Leu Tyr Asp Pro Ile
            100                 105                 110

Asn Val Glu Lys Glu Ser Leu Tyr Ile Thr Asn Gly Val Val Ser His
        115                 120                 125

Asn

<210>  2
<211>  24
<212>  PRT
<213>  Artificial Sequence

<220>
<223>  IntN WT AceL-TerL-3

<400>  2

Cys Val Asp Gly Asn Thr Ile Val Glu Thr Glu Asp Gly Lys Ile Lys
1               5                   10                  15

Ile Glu Asp Leu Tyr Lys Lys Leu
            20

<210>  3
<211>  24
<212>  PRT
<213>  Artificial Sequence

<220>
<223>  IntN WT AceL-Terl4

<400>  3

Cys Val Asp Gly Asn Thr Ile Val Glu Thr Glu Asp Gly Lys Ile Lys
1               5                   10                  15

Ile Glu Asp Leu Tyr Lys Lys Met
            20

<210>  4
<211>  24
<212>  PRT
<213>  Artificial Sequence

<220>
<223>  IntN WT AceL-Terl5

<400>  4

Cys Val Asp Gly Asn Thr Ile Val Glu Thr Glu Asp Gly Lys Ile Lys
1               5                   10                  15

Ile Glu Asp Leu Tyr Lys Lys Leu
            20

<210>  5
<211>  25
<212>  PRT
<213>  Artificial Sequence

<220>
<223>  AceL-TerL-11 IntN

<400>  5

Cys Val Tyr Gly Asp Thr Met Val Glu Thr Glu Asp Gly Lys Ile Lys
1               5                   10                  15

Ile Glu Asp Leu Tyr Lys Arg Leu Ala
            20                  25

<210>  6
<211>  104
<212>  PRT
<213>  Artificial Sequence

<220>
<223>  AceL-TerL-11 IntC

<400>  6

Met Phe Arg Thr Asn Thr Asn Asn Ile Lys Ile Leu Ser Pro Asn Gly
1               5                   10                  15

Phe Ser Asn Phe Asn Gly Ile Gln Lys Val Glu Arg Asn Leu Tyr Gln
            20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Ser Lys Lys Val Leu Tyr Asn Glu Leu Val Asn Glu Asn
65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100

<210>  7
<211>  660
<212>  PRT
<213>  Artificial Sequence

<220>
<223>  MBP-AceL-TerLcis-FKBP-His6, pIT063

<400>  7

Met Glu Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala

290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly Ile
    370                 375                 380

Glu Gly Arg Ile Ser Glu Phe Glu Phe Glu Cys Val Tyr Gly Asp Thr
385                 390                 395                 400

Met Val Glu Thr Glu Asp Gly Lys Ile Lys Ile Glu Asp Leu Tyr Lys
                405                 410                 415

Arg Leu Ala Met Gly Met Phe Arg Thr Asn Thr Asn Asn Ile Lys Ile
            420                 425                 430

Leu Ser Pro Asn Gly Phe Ser Asn Phe Asn Gly Ile Gln Lys Val Glu
        435                 440                 445

Arg Asn Leu Tyr Gln His Ile Ile Phe Asp Asp Asp Thr Glu Ile Lys
    450                 455                 460

Thr Ser Ile Asn His Pro Phe Gly Lys Asp Lys Ile Leu Ala Arg Asp
465                 470                 475                 480

Val Lys Val Gly Asp Tyr Leu Asn Ser Lys Lys Val Leu Tyr Asn Glu
                485                 490                 495

Leu Val Asn Glu Asn Ile Phe Leu Tyr Asp Pro Ile Asn Val Glu Lys
            500                 505                 510

Glu Ser Leu Tyr Ile Thr Asn Gly Val Val Ser His Asn Cys Glu Phe
        515                 520                 525

Leu Ser Arg Asn Asn Gly Asn Gly Asn Gly Thr Arg Gly Val Gln Val
    530                 535                 540

Glu Thr Ile Ser Pro Gly Asp Gly Arg Thr Phe Pro Lys Arg Gly Gln
545                 550                 555                 560

Thr Cys Val Val His Tyr Thr Gly Met Leu Glu Asp Gly Lys Lys Phe
                565                 570                 575

Asp Ser Ser Arg Asp Arg Asn Lys Pro Phe Lys Phe Met Leu Gly Lys
            580                 585                 590

Gln Glu Val Ile Arg Gly Trp Glu Glu Gly Val Ala Gln Met Ser Val
        595                 600                 605

Gly Gln Arg Ala Lys Leu Thr Ile Ser Pro Asp Tyr Ala Tyr Gly Ala
    610                 615                 620

Thr Gly His Pro Gly Ile Ile Pro Pro His Ala Thr Leu Val Phe Asp
625                 630                 635                 640

Val Glu Leu Leu Lys Leu Glu Thr Ser Tyr Gly Ser Arg Ser His His
                645                 650                 655

His His His His
            660

<210>  8
<211>  665
<212>  PRT
<213>  Artificial Sequence

<220>
<223>  MBP-AceL-TerLcis-FKBP-His6 pIT064

<400>  8

Met Glu Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp

85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
        340                 345                 350

Ser Gly Arg Gln Thr Val Asp Glu Ala Leu Lys Asp Ala Gln Thr Asn
    355                 360                 365

Ser Ser Ser Asn Asn Asn Asn Asn Asn Asn Asn Asn Asn Leu Gly Ile
    370                 375                 380

Glu Gly Arg Ile Ser Glu Phe Glu Phe Glu Cys Val Tyr Gly Asp Thr
385                 390                 395                 400

Met Val Glu Thr Glu Asp Gly Lys Ile Lys Ile Glu Asp Leu Tyr Lys
                405                 410                 415

Arg Leu Ala Met Gly Ser Gly Gly Ser Gly Met Phe Arg Thr Asn Thr
        420                 425                 430

Asn Asn Ile Lys Ile Leu Ser Pro Asn Gly Phe Ser Asn Phe Asn Gly
    435                 440                 445

Ile Gln Lys Val Glu Arg Asn Leu Tyr Gln His Ile Ile Phe Asp Asp
    450                 455                 460

Asp Thr Glu Ile Lys Thr Ser Ile Asn His Pro Phe Gly Lys Asp Lys
465                 470                 475                 480

Ile Leu Ala Arg Asp Val Lys Val Gly Asp Tyr Leu Asn Ser Lys Lys
                485                 490                 495

Val Leu Tyr Asn Glu Leu Val Asn Glu Asn Ile Phe Leu Tyr Asp Pro
        500                 505                 510

Ile Asn Val Glu Lys Glu Ser Leu Tyr Ile Thr Asn Gly Val Val Ser
    515                 520                 525

His Asn Cys Glu Phe Leu Ser Arg Asn Asn Gly Asn Gly Asn Gly Thr
    530                 535                 540

Arg Gly Val Gln Val Glu Thr Ile Ser Pro Gly Asp Gly Arg Thr Phe
545                 550                 555                 560

Pro Lys Arg Gly Gln Thr Cys Val Val His Tyr Thr Gly Met Leu Glu
                565                 570                 575

Asp Gly Lys Lys Phe Asp Ser Ser Arg Asp Arg Asn Lys Pro Phe Lys
        580                 585                 590

Phe Met Leu Gly Lys Gln Glu Val Ile Arg Gly Trp Glu Glu Gly Val
        595                 600                 605

Ala Gln Met Ser Val Gly Gln Arg Ala Lys Leu Thr Ile Ser Pro Asp
    610                 615                 620

Tyr Ala Tyr Gly Ala Thr Gly His Pro Gly Ile Ile Pro Pro His Ala
625                 630                 635                 640

Thr Leu Val Phe Asp Val Glu Leu Leu Lys Leu Glu Thr Ser Tyr Gly
                645                 650                 655

Ser Arg Ser His His His His His His
            660                 665

<210>  9
<211>  660
<212>  PRT
<213>  Artificial Sequence

<220>
<223>  pIT065:inaktiv(N129A,C+1A

<400>  9

Met Glu Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly Ile
    370                 375                 380

Glu Gly Arg Ile Ser Glu Phe Glu Phe Glu Cys Val Tyr Gly Asp Thr
385                 390                 395                 400

Met Val Glu Thr Glu Asp Gly Lys Ile Lys Ile Glu Asp Leu Tyr Lys
                405                 410                 415

Arg Leu Ala Met Gly Met Phe Arg Thr Asn Thr Asn Asn Ile Lys Ile
            420                 425                 430

Leu Ser Pro Asn Gly Phe Ser Asn Phe Asn Gly Ile Gln Lys Val Glu
        435                 440                 445

Arg Asn Leu Tyr Gln His Ile Ile Phe Asp Asp Asp Thr Glu Ile Lys
    450                 455                 460

Thr Ser Ile Asn His Pro Phe Gly Lys Asp Lys Ile Leu Ala Arg Asp
465                 470                 475                 480

Val Lys Val Gly Asp Tyr Leu Asn Ser Lys Lys Val Leu Tyr Asn Glu
                485                 490                 495

Leu Val Asn Glu Asn Ile Phe Leu Tyr Asp Pro Ile Asn Val Glu Lys
            500                 505                 510

Glu Ser Leu Tyr Ile Thr Asn Gly Val Val Ser His Ala Ala Glu Phe
        515                 520                 525

Leu Ser Arg Asn Asn Gly Asn Gly Asn Gly Thr Arg Gly Val Gln Val
    530                 535                 540

Glu Thr Ile Ser Pro Gly Asp Gly Arg Thr Phe Pro Lys Arg Gly Gln
545                 550                 555                 560

Thr Cys Val Val His Tyr Thr Gly Met Leu Glu Asp Gly Lys Lys Phe
                565                 570                 575

Asp Ser Ser Arg Asp Arg Asn Lys Pro Phe Lys Phe Met Leu Gly Lys
            580                 585                 590

Gln Glu Val Ile Arg Gly Trp Glu Glu Gly Val Ala Gln Met Ser Val
        595                 600                 605

Gly Gln Arg Ala Lys Leu Thr Ile Ser Pro Asp Tyr Ala Tyr Gly Ala
    610                 615                 620

Thr Gly His Pro Gly Ile Ile Pro Pro His Ala Thr Leu Val Phe Asp
625                 630                 635                 640

Val Glu Leu Leu Lys Leu Glu Thr Ser Tyr Gly Ser Arg Ser His His
                645                 650                 655

His His His His
            660

<210>  10
<211>  665
<212>  PRT
<213>  Artificial Sequence

<220>
<223>  pIT066:inaktiv(N129A,C+1A)

<400>  10

Met Glu Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
        180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly Ile
    370                 375                 380

Glu Gly Arg Ile Ser Glu Phe Glu Phe Glu Cys Val Tyr Gly Asp Thr
385                 390                 395                 400

Met Val Glu Thr Glu Asp Gly Lys Ile Lys Ile Glu Asp Leu Tyr Lys
                405                 410                 415

Arg Leu Ala Met Gly Ser Gly Gly Ser Gly Met Phe Arg Thr Asn Thr
            420                 425                 430

Asn Asn Ile Lys Ile Leu Ser Pro Asn Gly Phe Ser Asn Phe Asn Gly
        435                 440                 445

Ile Gln Lys Val Glu Arg Asn Leu Tyr Gln His Ile Ile Phe Asp Asp
    450                 455                 460

Asp Thr Glu Ile Lys Thr Ser Ile Asn His Pro Phe Gly Lys Asp Lys
465                 470                 475                 480

Ile Leu Ala Arg Asp Val Lys Val Gly Asp Tyr Leu Asn Ser Lys Lys
                485                 490                 495

Val Leu Tyr Asn Glu Leu Val Asn Glu Asn Ile Phe Leu Tyr Asp Pro
            500                 505                 510

Ile Asn Val Glu Lys Glu Ser Leu Tyr Ile Thr Asn Gly Val Val Ser
        515                 520                 525

His Ala Ala Glu Phe Leu Ser Arg Asn Asn Gly Asn Gly Asn Gly Thr
    530                 535                 540

Arg Gly Val Gln Val Glu Thr Ile Ser Pro Gly Asp Gly Arg Thr Phe
545                 550                 555                 560

Pro Lys Arg Gly Gln Thr Cys Val Val His Tyr Thr Gly Met Leu Glu
                565                 570                 575

Asp Gly Lys Lys Phe Asp Ser Ser Arg Asp Arg Asn Lys Pro Phe Lys
            580                 585                 590

Phe Met Leu Gly Lys Gln Glu Val Ile Arg Gly Trp Glu Glu Gly Val
        595                 600                 605

Ala Gln Met Ser Val Gly Gln Arg Ala Lys Leu Thr Ile Ser Pro Asp
    610                 615                 620

Tyr Ala Tyr Gly Ala Thr Gly His Pro Gly Ile Ile Pro Pro His Ala
625                 630                 635                 640

Thr Leu Val Phe Asp Val Glu Leu Leu Lys Leu Glu Thr Ser Tyr Gly
                645                 650                 655

Ser Arg Ser His His His His His His
            660                 665

<210>  11

<211>  25
<212>  PRT
<213>  Artificial Sequence

<220>
<223>  M1N

<400>  11

Cys Val Tyr Gly Asp Thr Met Val Glu Thr Glu Asp Gly Lys Ile Lys
1               5                   10                  15

Ile Glu Asp Leu Tyr Lys Arg Leu Thr
            20                  25

<210>  12
<211>  25
<212>  PRT
<213>  Artificial Sequence

<220>
<223>  M3N

<400>  12

Cys Val Ser Gly Asp Thr Met Val Glu Thr Glu Asp Gly Lys Ile Lys
1               5                   10                  15

Ile Glu Asp Leu Tyr Lys Arg Leu Ala
            20                  25

<210>  13
<211>  104
<212>  PRT
<213>  Artificial Sequence

<220>
<223>  M1C

<400>  13

Met Phe Arg Thr Asn Thr Asn Asn Ile Lys Ile Leu Ser Pro Asn Gly
1               5                   10                  15

Phe Ser Asn Phe Asp Gly Ile Gln Lys Val Glu Arg Asn Gln Tyr Gln
            20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Ser Lys Lys Val Leu Tyr Asn Glu Leu Val Asn Glu Asn
65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100

<210>  14
<211>  104
<212>  PRT
<213>  Artificial Sequence

<220>
<223>  M2C

<400>  14

Met Phe Arg Thr Asn Thr Asn Asn Ile Lys Ile Leu Gly Pro Asn Gly
1               5                   10                  15

Phe Ser Asn Phe Ile Gly Ile Gln Lys Val Glu Arg Asp Gln Tyr Gln
            20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Ser Lys Lys Val Leu Tyr Asn Glu Leu Val Asn Glu Asn
65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100

<210>  15
<211>  104
<212>  PRT
<213>  Artificial Sequence

<220>
<223>  M3C

<400>  15

Met Phe Arg Thr Asn Thr Asn Asn Ile Lys Ile Leu Ser Pro Asn Gly
1               5                   10                  15

Phe Ser Asn Phe Asn Gly Ile Gln Lys Val Glu Arg Asn Leu Tyr Gln

                20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Gly Lys Lys Val Leu Tyr Asn Glu Leu Val Asn Glu Asn
65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100

<210>  16
<211>  104
<212>  PRT
<213>  Artificial Sequence

<220>
<223>  M4C

<400>  16

Met Phe Arg Thr Asn Thr Asn Asn Ile Lys Ile Leu Ser Pro Asn Gly
1               5                   10                  15

Phe Ser Asn Phe Ile Gly Ile Gln Lys Val Glu Arg Asn Gln Tyr Gln
            20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Ser Lys Lys Val Leu Tyr Asn Glu Leu Val Asn Glu Asn
65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100

<210>  17
<211>  104

<212>  PRT
<213>  Artificial Sequence

<220>
<223>  M5C

<400>  17

Met Phe Arg Thr Asn Thr Asn Asn Ile Lys Ile Leu Ser Pro Asn Gly
1               5                   10                  15

Phe Ser Asn Phe Asp Gly Ile Gln Lys Val Glu Arg Asn Leu Tyr Gln
            20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Ser Lys Lys Val Leu Tyr Asn Glu Leu Val Asn Glu Asn
65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100


<210>  18
<211>  104
<212>  PRT
<213>  Artificial Sequence

<220>
<223>  M6C

<400>  18

Met Phe Arg Thr Asn Thr Asn Asn Ile Lys Ile Leu Ser Pro Asn Gly
1               5                   10                  15

Phe Ser Asn Phe Asn Gly Ile Gln Lys Val Glu Arg Asn Gln Tyr Gln
            20                  25                  30

His Ile Ile Phe Asp Asp Asp Thr Glu Ile Lys Thr Ser Ile Asn His
        35                  40                  45

Pro Phe Gly Lys Asp Lys Ile Leu Ala Arg Asp Val Lys Val Gly Asp
    50                  55                  60

Tyr Leu Asn Ser Lys Lys Val Leu Tyr Asn Glu Leu Val Asn Glu Asn

65                  70                  75                  80

Ile Phe Leu Tyr Asp Pro Ile Asn Val Glu Lys Glu Ser Leu Tyr Ile
                85                  90                  95

Thr Asn Gly Val Val Ser His Asn
            100

<210>  19
<211>  312
<212>  DNA
<213>  Artificial Sequence

<220>
<223>  DNA M1C

<400>  19
atgtttcgta ccaacaccaa caacattaaa attctgagcc cgaacggctt tagtaacttt      60

gacggcattc agaaagtgga acgtaaccag tatcagcata ttatttttga tgatgatacc     120

gaaattaaaa ccagcattaa ccatccgttt ggcaaagata aaattctggc gcgtgatgtg     180

aaagtgggcg attatctgaa cagcaaaaaa gtgctgtata acgaactggt gaacgaaaac     240

atttttctgt atgatccgat taacgtggaa aaagaaagcc tgtatattac caacggcgtg     300

gtgagccata ac                                                         312

<210>  20
<211>  312
<212>  DNA
<213>  Artificial Sequence

<220>
<223>  DNA  M2C

<400>  20
atgtttcgta ccaacaccaa caacattaaa attctgggcc cgaacggctt tagcaacttt      60

atcggcattc agaaagtgga acgtgaccag tatcagcata ttatttttga tgatgatacc     120

gaaattaaaa ccagcattaa ccatccgttt ggcaaagata aaattctggc gcgtgatgtg     180

aaagtgggcg attatctgaa cagcaaaaaa gtgctgtata acgaactggt gaacgaaaac     240

atttttctgt atgatccgat taacgtggaa aaagaaagcc tgtatattac caacggcgtg     300

gtgagccata ac                                                         312

<210>  21
<211>  312
<212>  DNA
<213>  Artificial Sequence

<220>
<223>  DNA M3C

<400>  21
atgtttcgta ccaacaccaa caacattaaa attctgagcc cgaacggctt tagcaacttt      60

aacggcattc agaaagtgga acgtaacctg tatcagcata ttatttttga tgatgatacc     120

gaaattaaaa ccagcattaa ccatccgttt ggcaaagata aaattctggc gcgtgatgtg     180

aaagtgggcg attatctgaa cggcaaaaaa gtgctgtata acgaactggt gaacgaaaac     240

atttttctgt atgatccgat taacgtggaa aaagaaagcc tgtatattac caacggcgtg     300

gtgagccata ac                                                         312


<210>  22
<211>  312
<212>  DNA
<213>  Artificial Sequence

<220>
<223>  DNA  M4C

<400>  22
atgtttcgta ccaacaccaa caacattaaa attctgagcc cgaacggctt tagcaacttt      60

atcggcattc agaaagtgga acgtaaccag tatcagcata ttatttttga tgatgatacc     120

gaaattaaaa ccagcattaa ccatccgttt ggcaaagata aaattctggc gcgtgatgtg     180

aaagtgggcg attatctgaa cagcaaaaaa gtgctgtata acgaactggt gaacgaaaac     240

atttttctgt atgatccgat taacgtggaa aaggaaagcc tgtatattac caacggcgtg     300

gtgagccata ac                                                         312


<210>  23
<211>  312
<212>  DNA
<213>  Artificial Sequence

<220>
<223>  DNA  M5C

<400>  23
atgtttcgta ccaacaccaa caacattaaa attctgagcc cgaacggctt tagcaacttt      60

gacggcattc agaaagtgga acgtaacctg tatcagcata ttatttttga tgatgatacc     120

gaaattaaaa ccagcattaa ccatccgttt ggcaaagata aaattctggc gcgtgatgtg     180

aaagtgggcg attatctgaa cagcaaaaaa gtgctgtata acgaactggt gaacgaaaac     240

atttttctgt atgatccgat taacgtggaa aaagaaagcc tgtatattac caacggcgtg     300

gtgagccata ac                                                         312


<210>  24
<211>  5
<212>  PRT
<213>  Artificial Sequence

<220>
<223>  IntN WTAceL-TerL-11 flanking sequence

<400>  24

Gln Thr Glu Phe Glu
1               5


<210>  25
<211>  5
<212>  PRT
<213>  Artificial Sequence

<220>
<223>  IntC WTAceL-TerL-11 flanking sequence

<400>  25

Cys Glu Phe Leu Gly
1               5


<210>  26
<211>  152
<212>  PRT
<213>  Artificial Sequence

<220>
<223>  GS033_TerA-6intein

<400>  26

Ser Ile Ser Gln Glu Ser Tyr Ile Asn Ile Glu Val Asn Gly Lys Val
1               5                   10                  15

Glu Thr Ile Lys Ile Gly Asp Leu Tyr Lys Lys Leu Ser Phe Asn Glu
            20                  25                  30

Arg Lys Phe Asn Glu Met Lys Leu Pro Glu Ser Val Val Lys Asn Asn
        35                  40                  45

Ile Asn Leu Lys Ile Glu Thr Pro Tyr Gly Phe Glu Asn Phe Tyr Gly
    50                  55                  60

Val Asn Lys Ile Lys Lys Asp Lys Tyr Ile His Leu Glu Phe Thr Asn
65                  70                  75                  80

Gly Glu Lys Leu Lys Cys Ser Leu Asp His Pro Leu Ser Thr Ile Asp
                85                  90                  95

Gly Ile Val Lys Ala Lys Asp Leu Asp Lys Tyr Thr Glu Val Tyr Thr
            100                 105                 110

Lys Phe Gly Gly Cys Phe Leu Lys Lys Ser Lys Val Ile Asn Glu Ser
        115                 120                 125

Ile Glu Leu Tyr Asp Ile Val Asn Ser Gly Leu Lys His Leu Tyr Tyr
    130                 135                 140

Ser Asn Asn Ile Ile Ser His Asn
145                 150

<210>  27
<211>  115
<212>  PRT
<213>  Artificial Sequence

<220>
<223>  Wild type IntC of GS033_TerA-6

<400>  27

Met Lys Leu Pro Glu Ser Val Val Lys Asn Asn Ile Asn Leu Lys Ile
1               5                   10                  15

Glu Thr Pro Tyr Gly Phe Glu Asn Phe Tyr Gly Val Asn Lys Ile Lys
            20                  25                  30

Lys Asp Lys Tyr Ile His Leu Glu Phe Thr Asn Gly Glu Lys Leu Lys
        35                  40                  45

Cys Ser Leu Asp His Pro Leu Ser Thr Ile Asp Gly Ile Val Lys Ala
    50                  55                  60

Lys Asp Leu Asp Lys Tyr Thr Glu Val Tyr Thr Lys Phe Gly Gly Cys
65                  70                  75                  80

Phe Leu Lys Lys Ser Lys Val Ile Asn Glu Ser Ile Glu Leu Tyr Asp
                85                  90                  95

Ile Val Asn Ser Gly Leu Lys His Leu Tyr Tyr Ser Asn Asn Ile Ile
            100                 105                 110

Ser His Asn
        115

<210>  28
<211>  37
<212>  PRT
<213>  Artificial Sequence

<220>
<223>  Wild type IntN of GS033_TerA-6

<400>  28

Ser Ile Ser Gln Glu Ser Tyr Ile Asn Ile Glu Val Asn Gly Lys Val

1               5                   10                  15

Glu Thr Ile Lys Ile Gly Asp Leu Tyr Lys Lys Leu Ser Phe Asn Glu
            20                  25                  30

Arg Lys Phe Asn Glu
        35

<210>  29
<211>  37
<212>  PRT
<213>  Artificial Sequence

<220>
<223>  IntN of GS033_TerA-6

<400>  29

Cys Ile Ser Gln Glu Ser Tyr Ile Asn Ile Glu Val Asn Gly Lys Val
1               5                   10                  15

Glu Thr Ile Lys Ile Gly Asp Leu Tyr Lys Lys Leu Ser Phe Asn Glu
            20                  25                  30

Arg Lys Phe Asn Glu
        35

<210>  30
<211>  28
<212>  PRT
<213>  Artificial Sequence

<220>
<223>  IntN of GS033_TerA-6 (minus 9aa)

<400>  30

Ser Ile Ser Gln Glu Ser Tyr Ile Asn Ile Glu Val Asn Gly Lys Val
1               5                   10                  15

Glu Thr Ile Lys Ile Gly Asp Leu Tyr Lys Lys Leu
            20                  25

<210>  31
<211>  28
<212>  PRT
<213>  Artificial Sequence

<220>
<223>  IntN of GS033_TerA-6 (minus 9aa synthetic)

<400>  31

Ser Ile Ser Gln Glu Ser Tyr Ile Asn Ile Glu Val Asn Gly Lys Val
1               5                   10                  15

Glu Thr Ile Lys Ile Gly Asp Leu Tyr Lys Lys Leu
            20                  25

<210>  32
<211>  34
<212>  PRT
<213>  Artificial Sequence

<220>
<223>  IntN of GS033_TerA-6 (minus 3aa)

<400>  32

Ser Ile Ser Gln Glu Ser Tyr Ile Asn Ile Glu Val Asn Gly Lys Val
1               5                   10                  15

Glu Thr Ile Lys Ile Gly Asp Leu Tyr Lys Lys Leu Ser Phe Asn Glu
            20                  25                  30

Arg Lys

<210>  33
<211>  25
<212>  PRT
<213>  Artificial Sequence

<220>
<223>  IntNAceL-TerL-11 with C1S substitution

<400>  33

Ser Val Ser Gly Asp Thr Met Val Glu Thr Glu Asp Gly Lys Ile Lys
1               5                   10                  15

Ile Glu Asp Leu Tyr Lys Arg Leu Ala
            20                  25

<210>  34
<211>  5
<212>  PRT
<213>  Artificial Sequence

<220>
<223>  IntN GS033_TerA-6 flanking sequence

<400>  34

Lys Val Glu Phe Glu
1               5

<210>  35
<211>  5
<212>  PRT

<213>  Artificial Sequence

<220>
<223>  Intc GS033_TerA-6 flanking sequence

<400>  35

Cys Glu Phe Leu Gly
1               5


<210>  36
<211>  128
<212>  DNA
<213>  Artificial Sequence

<220>
<223>  DNA  IntN  GS033_TerA-6

<400>  36
aggttgagtt tgagtcaatt tctcaagaat cttacataaa tatcgaagtt aatggtaagg      60

tcgaaacaat taaaattggc gatttatata aaaaactttc atttaacgaa agaaaattta     120

atgagtga                                                              128


<210>  37
<211>  360
<212>  DNA
<213>  Artificial Sequence

<220>
<223>  DNA  IntC  GS033_TerA-6

<400>  37
atgaaattac cagaatctgt agtaaaaaac aatatcaact taaaaataga aactccatat      60

ggatttgaga atttttatgg agtaaataaa ataaagaagg ataagtatat acatttagaa     120

tttaccaatg gtgaaaaact aaagtgctct ttagatcatc cattatcaac aattgatgga     180

attgtaaaag caaaagattt agacaaatat acagaagtat atacaaaatt tggtggatgc     240

tttctaaaaa aatcaaaagt tattaatgaa tcaatagaat tatatgatat tgtaaactcg     300

ggactaaagc atttatatta ttcaaataat ataatatctc acaactgcga attcttaggg     360


<210>  38
<211>  36
<212>  PRT
<213>  Artificial Sequence

<220>
<223>  IntN TerA-1_CP21-BP

<400>  38

Cys Cys Leu Glu Asn Thr Arg Val Gln Val Arg Asn Lys Tyr Thr Asn
1               5                   10                  15

Lys Ile Glu Thr Leu Thr Ile Lys Glu Leu Tyr Ala Arg Leu Gln Glu
            20                  25                  30

Leu Lys Lys Ser
        35

<210>  39
<211>  104
<212>  PRT
<213>  Artificial Sequence

<220>
<223>  IntC TerA-1_CP21-BP

<400>  39

Met Ser Glu Ile Gln Asp Ile Asn Pro Tyr Glu Ile Leu Thr Pro Gln
1               5                   10                  15

Gly Phe Lys Pro Phe Val Asp Ile Ile Lys Ser Ile Gln Thr Thr Gly
            20                  25                  30

Ile Thr Ile Thr Leu Glu Asp Ser Arg Glu Ile Ser Val Thr Leu Asp
        35                  40                  45

His Lys Phe Lys His Leu Asn Asp Tyr Lys Glu Ala Lys Tyr Phe Lys
    50                  55                  60

Val Gly Asp Lys Leu Gln Cys Ser Lys Ile Ile Lys Ile Glu Asn Ile
65                  70                  75                  80

Glu Gly Glu Phe Tyr Glu Pro Leu Glu Val Gln Asp His Glu Tyr Ile
                85                  90                  95

Ala Asn Asp Phe Ile Asn His Asn
            100

<210>  40
<211>  5
<212>  PRT
<213>  Artificial Sequence

<220>
<223>  IntN TerA-1_CP21-BP flanking sequence

<400>  40

Phe Arg Gly Phe Ser
1               5

<210>  41
<211>  5
<212>  PRT

<213>  Artificial Sequence

<220>
<223>  IntC TerA-1_CP21-BP flanking sequence

<400>  41

Cys Asn Ile Ile Val
1               5


<210>  42
<211>  126
<212>  DNA
<213>  Artificial Sequence

<220>
<223>  DNA IntN TerA-1_CP21-BP

<400>  42
tttaggggat tcagttgctg tctcgaaaat actcgagtgc aggtaagaaa taaatatact      60

aataaaatag aaacgcttac cataaaggaa ttgtatgcta ggttacaaga actcaaaaaa     120

tcttaa                                                                126


<210>  43
<211>  327
<212>  DNA
<213>  Artificial Sequence

<220>
<223>  DNA IntC TerA-1_CP21-BP

<400>  43
gtgagtgaaa tccaagatat aaatccatat gaaatattaa caccacaagg atttaaacct      60

tttgttgata tcattaaatc aattcaaaca actggcataa caataacttt agaggattca     120

agagagatat cagttacatt agatcacaaa tttaaacact taaatgatta taaagaagcc     180

aaatatttta aagtaggtga taaattacag tgttcaaaaa ttattaaaat tgaaaatatt     240

gaaggtgaat tttatgaacc tttagaagtt caagatcacg agtatatagc caacgacttt     300

ataaatcata attgtaatat aatcgtt                                         327


<210>  44
<211>  31
<212>  PRT
<213>  Artificial Sequence

<220>
<223>  IntN  CP81-BP_TerA

<400>  44

Cys Val Ala Gly Asp Thr Lys Ile Thr Val Arg Asn Lys Lys Thr Gly
1               5                   10                  15

Val Ile Glu Asp Ile Thr Met Glu Glu Leu Tyr Asn Arg Ile Gly
            20                  25                  30

<210> 45
<211> 100
<212> PRT
<213> Artificial Sequence

<220>
<223> IntC CP81-BP_TerA

<400> 45

Met Tyr Glu Val Leu Thr Pro Asn Gly Phe Ser Asp Phe Asp Asp Ile
1               5                   10                  15

Ser Arg Glu Lys Lys Asp Val Tyr Lys Val Ile Thr Glu Asp Asp Phe
            20                  25                  30

Ile Lys Val Thr Lys Gly His Lys Phe Glu Thr Pro Asn Gly Phe Lys
        35                  40                  45

Gln Leu Lys His Leu Lys Ile Asn Asp Leu Ile Lys Tyr Lys Asn Lys
    50                  55                  60

Phe Ser Lys Ile Val Leu Ile Asp Tyr Val Gly Val Glu Tyr Val Tyr
65                  70                  75                  80

Asp Leu Ile Asn Val His Lys Asn Asn Glu Tyr Tyr Thr Asn Asn Phe
                85                  90                  95

Val Ser His Asn
            100

<210> 46
<211> 5
<212> PRT
<213> Artificial Sequence

<220>
<223> IntN CP81-BP_TerA flanking sequence

<400> 46

Ile Phe Ile Asp Glu
1               5

<210> 47
<211> 5
<212> PRT
<213> Artificial Sequence

<220>
<223> Intc CP81-BP_TerA flanking sequence

<400>  47

Cys Ala Phe Ile Asp
1               5


<210>  48
<211>  111
<212>  DNA
<213>  Artificial Sequence

<220>
<223>  DNA  IntN CP81-BP_TerA

<400>  48
atttttattg atgaatgtgt agctggtgac acaaaaatta cagttagaaa taagaaaaca       60

ggtgtcattg aagatataac aatggaagag ttatataaca gaataggata a               111


<210>  49
<211>  315
<212>  DNA
<213>  Artificial Sequence

<220>
<223>  DNA IntC CP81-BP_TerA

<400>  49
atgtatgaag tactaacacc aaatggattt agtgattttg atgatatatc aagagaaaaa       60

aaagatgtat ataaagtaat aacagaagat gattttataa aagtaacaaa aggtcataaa      120

tttgaaacac ctaatggttt taaacaatta aaacatctta aaattaatga tttaataaaa      180

tataaaaata aattttcaaa aattgtttta atagattatg ttggagtaga atatgtatat      240

gatttaatta atgtacataa aaataacgag tattatacaa ataattttgt ttcacacaat      300

tgtgcgttta tagat                                                       315


<210>  50
<211>  33
<212>  PRT
<213>  Artificial Sequence

<220>
<223>  IntN AceL-1_ClpC-1

<400>  50

Cys Phe Ser Lys Lys Thr Ser Ile Lys Leu Arg Asn Lys Lys Thr Gly
1               5                   10                  15


Asp Leu Glu Glu Ile Asp Ile Ser Asp Leu Ile Tyr Glu Leu His Ile
            20                  25                  30


Ser

<210>  51
<211>  125
<212>  PRT
<213>  Artificial Sequence

<220>
<223>  IntC AceL-1_ClpC-1

<400>  51

Met Ile Lys Leu Tyr Asn Lys Lys Gln Asn Lys Lys Phe Thr Lys Ser
1               5                   10                  15

Tyr Asp Leu Gly Asp Tyr Gln Ile Leu Thr Asp Ser Gly Tyr Ile Gly
            20                  25                  30

Leu Val Ser Leu His Glu Thr Ile Pro Tyr Glu Val Trp Lys Leu Lys
        35                  40                  45

Leu Ser Asn Gly Tyr Glu Leu Glu Cys Ala Asp Asp His Ile Ile Phe
    50                  55                  60

Asp Asn Glu Met Asn Glu Ile Phe Val Lys Asn Leu Glu Leu Gly Asp
65                  70                  75                  80

Arg Val Lys Val Asp Asp Gly Tyr Ala Val Val Ile Glu Leu Val Asn
                85                  90                  95

Thr Gly Leu Leu Glu Ser Met Tyr Asp Phe Glu Leu Val Glu Asp Ser
            100                 105                 110

Asn Arg Arg Tyr Tyr Thr Asn Gly Ile Leu Ser His Asn
        115                 120                 125


<210>  52
<211>  5
<212>  PRT
<213>  Artificial Sequence

<220>
<223>  IntN AceL-1_ClpC-1 flanking sequence

<400>  52

Ser Gly Val Gly Lys
1               5


<210>  53
<211>  5
<212>  PRT
<213>  Artificial Sequence

<220>
<223>  IntC AceL-1_ClpC-1flanking sequence

<400>  53

Thr Glu Leu Ala Lys
1               5


<210>  54
<211>  117
<212>  DNA
<213>  Artificial Sequence

<220>
<223>  DNA  IntN AceL-1_ClpC-1

<400>  54
agcggggttg gtaaatgttt ttctaaaaaa acatcaataa aattaaggaa taaaaaaact      60

ggtgatttag aagaaattga tatttctgat ctaatatatg aactacacat tagctaa        117


<210>  55
<211>  390
<212>  DNA
<213>  Artificial Sequence

<220>
<223>  DNA IntC AceL-1_ClpC-1

<400>  55
atgataaaat tatataataa aaaacaaaat aaaaaattca ccaaatctta tgatttgggt      60

gattaccaaa tactaactga tagtggatat attggtttgg tctcattaca tgagacaata     120

ccatatgaag tttggaaatt gaaattatct aatggatatg aattagagtg tgctgatgat     180

catattattt ttgataatga aatgaatgag atatttgtaa agaatctaga attaggagac     240

agagtaaaag tagatgatgg atatgctgtt gttatagaat tagtaaatac tggtctatta     300

gaaagtatgt atgattttga gttagtagaa gattcaaata gaaggtatta tacaaatggt     360

attttatcac acaacacaga actggctaaa                                      390


<210>  56
<211>  30
<212>  PRT
<213>  Artificial Sequence

<220>
<223>  IntN AceL-1_ClpC-2

<400>  56

Cys Val Ser Pro Asn Thr Lys Ile Lys Ile Arg Asn Ser Ser Thr Gly
1               5                   10                  15


Glu Ile Ser Glu Val Thr Ile Ala Glu Phe Asn Lys Met Ile
            20                  25                  30

<210>  57
<211>  115
<212>  PRT
<213>  Artificial Sequence

<220>
<223>  IntC AceL-1_ClpC-2

<400>  57

Met Lys Lys Ile Val Lys Ser Val Ser Val Glu Gly Phe Glu Val Leu
1               5                   10                  15

Ser Asp Asn Gly Trp Val Pro Ile Lys Asn Val His Thr Thr Val Pro
            20                  25                  30

Tyr Glu Leu Tyr Asn Leu Arg Thr Ala Asn Gly Leu Arg Leu Glu Cys
        35                  40                  45

Ala Asp Asn His Ile Val Phe Thr Ser Lys Leu Lys Glu Val Tyr Val
    50                  55                  60

Lys Asp Leu Asn Val Asp Asp Lys Ile Met Thr Glu Asp Gly Val Ser
65                  70                  75                  80

Leu Val Ser Ser Ile Glu Lys Thr Lys Ala Lys Val Thr Met Tyr Asp
                85                  90                  95

Leu Glu Val Asp Ser Glu Asp His Arg Tyr Tyr Thr Asp Gly Ile Leu
            100                 105                 110

Ser His Asn
        115


<210>  58
<211>  5
<212>  PRT
<213>  Artificial Sequence

<220>
<223>  IntN AceL-1_ClpC-2 flanking sequence

<400>  58

Ala Gly Val Gly Lys
1               5


<210>  59
<211>  5
<212>  PRT
<213>  Artificial Sequence

<220>
<223>  IntCAceL-1_ClpC-2 flanking sequence

<400>  59

Thr Ser Leu Ile Glu
1               5


<210>  60
<211>  108
<212>  DNA
<213>  Artificial Sequence

<220>
<223>  DNA I IntN AceL-1_ClpC-2

<400>  60
gcaggagtag gtaaatgcgt tagtcctaat acgaagatta agattaggaa cagtagcact      60

ggagaaattt cagaagttac gatagcggaa ttcaataaga tgatttaa                  108


<210>  61
<211>  360
<212>  DNA
<213>  Artificial Sequence

<220>
<223>  DNA IntC AceL-1_ClpC-2

<400>  61
atgaaaaaaa ttgttaagag tgtaagtgta gaaggatttg aggtactctc tgataatgga      60

tgggtaccaa ttaaaaatgt acataccact gtaccctacg aactctataa cctccgtaca     120

gccaacggtt tgcggttaga atgtgcagac aatcatatcg tgtttacttc taagctaaaa     180

gaggtatatg ttaaagactt aaatgttgac gataagatta tgactgagga tggagtatct     240

ttagtatcat caattgaaaa gactaaagct aaagtaacga tgtatgatct tgaagtagat     300

agtgaagatc atcgttacta tactgacggt attctttcac ataacacttc tctaatagaa     360


<210>  62
<211>  34
<212>  PRT
<213>  Artificial Sequence

<220>
<223>  IntN AceL-1_RadA1-1

<400>  62

Cys Val His Pro Asn Thr Leu Val Lys Ile Lys Ile Asp Ser Thr Gly
1               5                   10                  15


Glu Glu Arg Thr Ile Thr Val Lys Asp Leu His Glu Leu Ile Lys Ser
            20                  25                  30

Val Lys

<210> 63
<211> 115
<212> PRT
<213> Artificial Sequence

<220>
<223> IntC AceL-1_RadA1-1

<400> 63

Met Lys Arg Lys Phe Ile Glu Ser Ile Ser Ala Asp Asn Ile Ser Ile
1               5                   10                  15

Met Thr Asp Thr Gly Trp Glu Lys Val Lys Gly Ser His Val Thr Ile
            20                  25                  30

Glu Tyr Lys Val Phe Asn Leu Val Thr Asp Arg Leu Ser Leu Gln Cys
        35                  40                  45

Ala Asp Asp His Ile Val Phe Lys Glu Asp Phe Ser Glu Val Phe Val
    50                  55                  60

Lys Asp Leu Glu Val Gly Asp Leu Ile Gln Thr Val Asn Gly Leu Glu
65                  70                  75                  80

Ser Val Thr Glu Val Tyr Glu Thr Asp Asp Leu Val Asn Met His Asp
                85                  90                  95

Leu Glu Ile Asp Ser Lys Asn His Arg Tyr Tyr Thr Asp Gly Ile Leu
            100                 105                 110

Ser His Asn
        115

<210> 64
<211> 5
<212> PRT
<213> Artificial Sequence

<220>
<223> IntN AceL-1_RadA1-1 flanking sequence

<400> 64

Pro Gly Val Gly Lys
1               5

<210> 65
<211> 5
<212> PRT

<213>  Artificial Sequence

<220>
<223>  IntC AceL-1_RadA1-1 flanking sequence

<400>  65

Thr Thr Leu Leu Leu
1               5


<210>  66
<211>  120
<212>  DNA
<213>  Artificial Sequence

<220>
<223>  DNA IntN AceL-1_RadA1-1

<400>  66
ccaggagttg gtaaatgcgt ccatccaaat acattggtaa aaatcaaaat tgattctact      60

ggtgaggagc gtactattac agtcaaagac ctccacgaac taattaaatc tgtaaaatga      120


<210>  67
<211>  360
<212>  DNA
<213>  Artificial Sequence

<220>
<223>  DNA IntC AceL-1_RadA1-1

<400>  67
atgaaacgta aatttataga aagtatttct gcagacaata tcagcatcat gacagatact      60

ggttgggaaa aagttaaagg tagtcacgtt acaattgagt ataaagtatt caaccttgtc      120

actgacaggt tatcactaca atgtgcagat gatcatatcg tttttaaaga ggacttctca      180

gaggtctttg taaaggacct tgaggttggt gatttaatac aaacagtaaa cggtttagaa      240

tcagttactg aagtatatga aacagacgac ttggtaaata tgcacgattt agaaattgat      300

tctaaaaacc ataggtatta tactgatgga attctttcac ataatactac attattattg      360


<210>  68
<211>  34
<212>  PRT
<213>  Artificial Sequence

<220>
<223>  IntN AceL-1_TerL-10

<400>  68

Cys Val Ser Gly Asp Thr Lys Val Thr Leu Lys Asp Asn Asp Thr Gly
1               5                   10                  15


Lys Ile Ile Asn Val Asn Ile Glu Glu Met Val Ser Val Ser Ser Leu
            20                  25                  30

Asp Val

<210> 69
<211> 105
<212> PRT
<213> Artificial Sequence

<220>
<223> IntC AceL-1_TerL-10

<400> 69

Met Glu Val Gly Lys Met Ser Lys Ser Tyr Lys Val Leu Ser Pro Ser
1               5                   10                  15

Gly Phe Val Asp Phe Ala Gly Ile Gln Lys Ile Thr Arg Ser Lys Tyr
            20                  25                  30

Arg His Phe Ile Phe Asp Asp Gly Thr Glu Ile Lys Cys Ser Leu Asn
        35                  40                  45

His Arg Phe Gly Glu Glu Glu Ile Val Ala Ser Thr Leu His His Gly
    50                  55                  60

Thr Glu Leu Gln Gly Lys Lys Ile Leu Tyr Ala Glu Asp Val Glu Asp
65                  70                  75                  80

Asp Ile Asp Leu Tyr Asp Leu Leu Asn Val Ala Asn Gly Asn Leu Tyr
                85                  90                  95

Tyr Thr Asn Gly Leu Val Ser His Asn
            100                 105

<210> 70
<211> 5
<212> PRT
<213> Artificial Sequence

<220>
<223> IntN AceL-1_TerL-10 flanking sequence

<400> 70

Asn Thr Glu Phe Glu
1               5

<210> 71
<211> 5
<212> PRT
<213> Artificial Sequence

<220>
<223>  IntC AceL-1_TerL-10 flanking sequence

<400>  71

Cys Glu Phe Leu Gly
1               5


<210>  72
<211>  120
<212>  DNA
<213>  Artificial Sequence

<220>
<223>  DNA IntN AceL-1_TerL-10

<400>  72
aacacggagt ttgagtgtgt ttctggtgat acaaaggtta ctctcaaaga caatgataca      60

ggaaagatta ttaatgtaaa tattgaagaa atggtgagtg tgagttcttt ggatgtataa     120


<210>  73
<211>  330
<212>  DNA
<213>  Artificial Sequence

<220>
<223>  DNA IntC AceL-1_TerL-10

<400>  73
atggaagttg gaaagatgtc taaaagttat aaagtgttat caccatcagg gtttgtggat      60

tttgctggta ttcaaaaaat aacacgcagc aaatatcgac attttatttt tgatgatggc     120

acagaaatca aatgttcgtt aaatcataga tttggtgaag aggaaatagt agcctcaaca     180

ctccatcacg gcacagagct tcagggtaaa aaaatactgt atgcagaaga tgttgaggat     240

gatattgatt tatatgattt gttaaatgtt gccaatggaa atctttacta caccaacgga     300

ttagtatcac acaattgtga gttccttggc                                      330


<210>  74
<211>  55
<212>  PRT
<213>  Artificial Sequence

<220>
<223>  IntN AceL-1_TerL-2

<400>  74

Cys Phe Phe Phe Asn Thr Ile Ile Ser Val Glu Thr Asn Asn Gln Gln
1               5                   10                  15


Tyr Glu Thr Arg Ile Gly Ile Leu Tyr Tyr Ser Met Val Ser Lys Glu
            20                  25                  30

Arg Asn Leu Thr Ile Leu Glu Lys Ile Lys Ile Lys Leu Tyr Asp Leu
        35                  40                  45

Leu Phe Ile Leu Glu Lys His
    50                  55

<210>  75
<211>  150
<212>  PRT
<213>  Artificial Sequence

<220>
<223>  IntC AceL-1_TerL-2

<400>  75

Met Leu Arg Ile Phe Lys Arg Cys Leu Ile Tyr Leu Ile Lys Lys Met
1               5                   10                  15

Ile Glu Phe Ile Glu Leu Tyr Glu Tyr Lys Lys Ile Ser Leu Asp Glu
            20                  25                  30

Cys Asp Ile Asn Lys Lys Ile Leu Asn Ser Ile Ser Leu Met Asp Leu
        35                  40                  45

Lys Val Glu Thr Asp Thr Gly Tyr Glu Thr Ser Ser Asn Ile His Ile
    50                  55                  60

Thr Gln Pro Phe Lys His Tyr Asn Ile Glu Thr Val Asp Gly Tyr Glu
65                  70                  75                  80

Ile Ile Cys Ala Asp Asn His Ile Leu Phe Asp Glu Glu Phe Asn Glu
                85                  90                  95

Val Phe Thr Lys Asp Leu Lys Ile Gly Asp Leu Ile Lys Thr Lys Asn
            100                 105                 110

Gly Asn Ser Val Ile Lys Ser Ile Tyr Ile Asp Thr His Lys Ser Ser
        115                 120                 125

Met Phe Asp Leu Thr Ile Asp His Pro Asn His Arg Phe Tyr Thr Asn
    130                 135                 140

Gly Ile Leu Ser His Asn
145                 150

<210>  76
<211>  5
<212>  PRT
<213>  Artificial Sequence

<220>
<223>  IntNAceL-1_TerL-2 flanking sequence

<400>  76

Arg Gln Val Gly Lys
1               5


<210>  77
<211>  5
<212>  PRT
<213>  Artificial Sequence

<220>
<223>  IntCAceL-1_TerL-2flanking sequence

<400>  77

Thr Ile Ser Ser Ser
1               5


<210>  78
<211>  183
<212>  DNA
<213>  Artificial Sequence

<220>
<223>  DNA  IntN AceL-1_TerL-2

<400>  78
aggcaagttg gaaaatgttt ttttttcaat acaattatat ccgttgaaac caataatcag      60

caatatgaaa ctagaatagg aattctttat tattcaatgg tttcaaagga aagaaattta     120

actattttag agaaaattaa aataaaatta tatgatttat tattcatttt agaaaaacat     180

taa                                                                   183


<210>  79
<211>  465
<212>  DNA
<213>  Artificial Sequence

<220>
<223>  DNA  IntC AceL-1_TerL-2

<400>  79
atgcttagaa tttttaaaag gtgtttaatt tatttaatta aaaaaatgat tgaatttatt      60

gaattatatg aatataaaaa aatctcatta gatgagtgtg atataaataa aaaaatatta     120

aactcaatat ctcttatgga tttaaaggta gagactgata caggatatga aacatcatct     180

aatatacaca taacacaacc atttaaacac tataatattg aaacggttga tggttatgaa     240

ataatatgtg ctgataatca tatattattt gatgaggaat tcaatgaagt atttacaaag     300

gatttaaaaa taggagattt aattaaaaca aaaaatggca acagtgttat caagagtatt     360

tatatagaca cacataagtc atccatgttt gacctaacaa tagaccatcc aaaccacagg     420

ttctatacaa atggtatact ttcacataat acgatatcat cttct                    465

<210>  80
<211>  24
<212>  PRT
<213>  Artificial Sequence

<220>
<223>  IntN AceL-1_TerL-3

<400>  80

Cys Val Asp Gly Asn Thr Ile Val Glu Thr Glu Asp Gly Lys Ile Lys
1               5                   10                  15

Ile Glu Asp Leu Tyr Lys Lys Leu
            20

<210>  81
<211>  104
<212>  PRT
<213>  Artificial Sequence

<220>
<223>  IntC AceL-1_TerL-3

<400>  81

Met Phe Ile Thr Asn Thr Asp Asn Ile Lys Ile Leu Ser Pro Ser Gly
1               5                   10                  15

Phe Ser Asn Phe Asn Gly Ile Gln Lys Val Glu Arg Asn Leu Tyr Gln
            20                  25                  30

His Ile Ile Phe Asp Asp Glu Ser Glu Ile Lys Thr Ser Ile Asn His
        35                  40                  45

Pro Phe Gly Lys Asn Lys Ile Leu Ala Arg Asn Val Lys Val Gly Asp
    50                  55                  60

Tyr Leu Ser Ser Lys Lys Val Leu Tyr Asn Glu Leu Val Asn Glu Lys
65                  70                  75                  80

Ile Phe Leu Tyr Asp Pro Ile Asn Val Glu Lys Glu Asn Leu Tyr Ile
                85                  90                  95

Thr Asn Gly Val Val Ser His Asn
            100

<210>  82
<211>  5
<212>  PRT

<213>  Artificial Sequence

<220>
<223>  IntN AceL-1_TerL-3 flanking sequence

<400>  82

Gln Gln Glu Phe Glu
1               5


<210>  83
<211>  5
<212>  PRT
<213>  Artificial Sequence

<220>
<223>  IntC AceL-1_TerL-3 flanking sequence

<400>  83

Cys Glu Phe Leu Gly
1               5


<210>  84
<211>  90
<212>  DNA
<213>  Artificial Sequence

<220>
<223>  DNA  IntN  AceL-1_TerL-3

<400>  84
caacaagagt ttgaatgtgt tgacggtaat acgatagtcg aaacggaaga tggtaaaata        60

aaaatagaag atttatataa aaaattgtga                                          90


<210>  85
<211>  327
<212>  DNA
<213>  Artificial Sequence

<220>
<223>  DNA IntCAceL-1_TerL-3

<400>  85
atgtttataa ctaatacaga taatataaaa atattaagtc caagtggatt ttctaatttt        60

aatggtattc aaaaggttga aagaaacctt tatcaacaca ttatctttga tgatgaatct       120

gaaataaaaa cttctattaa ccaccctttt ggtaaaaata aaatattagc aagaaatgta       180

aaagtaggag attatttaag tagtaaaaaa gtattatata atgagttggt taatgaaaaa       240

atatttttat atgaccctat aaatgtagaa aaagaaaact tatatattac taacggtgtt       300

gtttctcata attgtgagtt tttaggt                                             327


<210>  86
<211>  24

<212>  PRT
<213>  Artificial Sequence

<220>
<223>  IntN AceL-1_TerL-4

<400>  86

Cys Val Asp Gly Asn Thr Ile Val Glu Thr Glu Asp Gly Lys Ile Lys
1               5                   10                  15

Ile Glu Asp Leu Tyr Lys Lys Met
            20

<210>  87
<211>  104
<212>  PRT
<213>  Artificial Sequence

<220>
<223>  IntC AceL-1_TerL-4

<400>  87

Met Phe Arg Thr Asn Thr Asp Asn Ile Lys Ile Leu Ser Pro Ser Gly
1               5                   10                  15

Phe Ser Ile Phe Asn Gly Ile Gln Lys Val Glu Arg Asp Leu Tyr Gln
            20                  25                  30

His Ile Ile Phe Asp Asp Lys Ser Glu Ile Lys Thr Ser Ile Asn His
        35                  40                  45

Pro Phe Gly Lys Asp Lys Ile Leu Ala Arg Asn Ile Lys Val Gly Asp
    50                  55                  60

Tyr Leu Asn Ser Lys Lys Val Leu Tyr Asn Glu Leu Val Ala Glu Lys
65                  70                  75                  80

Ile Thr Leu Tyr Asp Pro Ile Asn Val Glu Lys Glu Asn Leu Tyr Ile
                85                  90                  95

Thr Asn Gly Val Ile Ser His Asn
            100

<210>  88
<211>  5
<212>  PRT
<213>  Artificial Sequence

<220>
<223>  IntN AceL-1_TerL4 flanking sequence

<400>  88

Gln Gln Glu Phe Glu
1               5

<210>  89
<211>  5
<212>  PRT
<213>  Artificial Sequence

<220>
<223>  IntC AceL-1_TerL-4 flanking sequence

<400>  89

Cys Glu Phe Leu Gly
1               5

<210>  90
<211>  90
<212>  DNA
<213>  Artificial Sequence

<220>
<223>  DNA  IntN  AceL-1_TerL-4

<400>  90
caacaagagt ttgagtgtgt ggatggaaat acgatagtcg aaacggaaga tggcaaaata      60

aaaatagaag atttatataa aaaaatgtga                                       90

<210>  91
<211>  327
<212>  DNA
<213>  Artificial Sequence

<220>
<223>  DNA IntC AceL-1_TerL-4

<400>  91
atgtttagaa caaatacaga taatataaaa attttaagtc caagtgggtt ttctattttt      60

aatggcattc aaaaggttga aagagacctc tatcaacata ttatctttga tgataaatct     120

gaaataaaga cttctatcaa ccaccccttt ggtaaagata aaatattagc gagaaatata     180

aaggttggtg attatttaaa tagtaagaaa gttttatata atgagttggt cgccgaaaag     240

attactttat atgatcctat aaatgtagaa aaagaaaatt tatatatcac taacggtgtt     300

atttctcata attgtgagtt tttaggt                                         327

<210>  92
<211>  24
<212>  PRT
<213>  Artificial Sequence

<220>
<223>  IntN AceL-1_TerL-5

<400>  92

Cys Val Asp Gly Asn Thr Ile Val Glu Thr Glu Asp Gly Lys Ile Lys
1               5                   10                  15

Ile Glu Asp Leu Tyr Lys Lys Leu
            20

<210>  93
<211>  104
<212>  PRT
<213>  Artificial Sequence

<220>
<223>  IntC AceL-1_TerL-5

<400>  93

Met Phe Arg Thr Asn Thr Asp Asn Ile Lys Ile Leu Ser Pro Ser Gly
1               5                   10                  15

Phe Ser Asn Phe Asn Gly Ile Gln Lys Val Glu Arg Asp Leu Tyr Gln
            20                  25                  30

His Ile Ile Phe Asp Asp Lys Ser Glu Ile Lys Thr Ser Ile Asn His
        35                  40                  45

Pro Phe Gly Lys Asp Lys Ile Leu Ala Arg Asn Ile Lys Val Gly Asp
    50                  55                  60

Tyr Leu Asn Ser Lys Lys Val Leu Tyr Asn Glu Leu Val Asn Glu Lys
65                  70                  75                  80

Ile Thr Leu Tyr Asp Pro Ile Asn Val Glu Lys Glu Asn Leu Tyr Ile
                85                  90                  95

Thr Asn Gly Val Ile Ser His Asn
            100

<210>  94
<211>  5
<212>  PRT
<213>  Artificial Sequence

<220>
<223>  IntN AceL-1_TerL5 flanking sequence

<400>  94

Gln Gln Glu Phe Glu
1               5

<210>  95

<211>  5
<212>  PRT
<213>  Artificial Sequence

<220>
<223>  IntC AceL-1_TerL-5 flanking sequence

<400>  95

Cys Glu Phe Leu Gly
1               5


<210>  96
<211>  90
<212>  DNA
<213>  Artificial Sequence

<220>
<223>  DNA  IntN  AceL-1_TerL-5

<400>  96
caacaagagt ttgaatgtgt tgacggtaat acgatagttg aaacggaaga tggtaaaata      60

aaaatagaag atttatataa aaaattatag                                       90


<210>  97
<211>  327
<212>  DNA
<213>  Artificial Sequence

<220>
<223>  DNA  IntC AceL-1_TerL-5

<400>  97
atgtttagaa ccaatacaga taatataaaa atattaagtc caagtggatt ttctaatttt      60

aacggcattc aaaaggttga aagagacctc tatcaacata ttatctttga tgataagtct     120

gaaataaaaa cttctattaa ccaccctttt ggtaaagata aaatattagc gagaaatata     180

aaagtaggag attatttaaa tagtaagaag gttttatata atgagttggt taatgaaaaa     240

attactttat atgaccctat aaatgtagaa aaagaaaact tatatattac taacggtgtt     300

atttctcata attgtgagtt tttaggt                                         327


<210>  98
<211>  27
<212>  PRT
<213>  Artificial Sequence

<220>
<223>  IntN AceL-1_UvsW-3-1

<400>  98

Cys Arg Thr Tyr Asp Ser Thr Met Asp Ile Asp Val Gly Asn Ser Asp
1               5                   10                  15

Phe Ala Glu Tyr Leu Leu Asn Asn Ser Lys Lys
            20                  25

<210>  99
<211>  135
<212>  PRT
<213>  Artificial Sequence

<220>
<223>  IntC AceL-1_UvsW-3-1

<400>  99

Met Lys Phe Asn Ile Pro Ile Gly Glu Leu Ala Glu Ser Ile Ala Lys
1               5                   10                  15

Tyr Lys Gly Val Leu Leu Asn Asp Asn Cys Glu Ile Asn Ile Lys Asp
            20                  25                  30

Leu Asp Cys Lys Val Asn Thr Pro Ser Gly Thr Ala Thr Ile Asn Ile
        35                  40                  45

Ile Ile Lys Lys Glu Lys Leu Glu Gly Ile Lys Leu Leu Leu Ala Asn
    50                  55                  60

Gly Val Glu Ile Lys Cys Ala Asn Lys His Ile Leu Arg Tyr Asn Asn
65                  70                  75                  80

Ala Asp Val Phe Ala Asp Ser Leu Ala Ile Gly Asp Ser Val Glu Thr
                85                  90                  95

Ile Asn Gly Asn Val Lys Val Ser Ser Ile Asn Asn Ile Asp Asp Thr
            100                 105                 110

Thr Phe Tyr Asp Ile Gly Ile Asp Ala Pro Tyr Leu Tyr Tyr Asp Ala
        115                 120                 125

Asp Gly Val Leu His His Asn
    130                 135

<210>  100
<211>  5
<212>  PRT
<213>  Artificial Sequence

<220>
<223>  IntN AceL-1_UvsW-3-1 flanking sequence

<400>  100

Thr Gly Ala Gly Lys
1               5

<210>  101
<211>  5
<212>  PRT
<213>  Artificial Sequence

<220>
<223>  IntC AceL-1_UvsW-3-1 flanking sequence

<400>  101

Thr Ile Thr Thr Ala
1               5


<210>  102
<211>  99
<212>  DNA
<213>  Artificial Sequence

<220>
<223>  DNA  IntN AceL-1_UvsW-3-1

<400>  102
actggtgcag gcaaatgtcg aacttatgat tctacaatgg atatagatgt aggtaattct      60

gattttgctg aatatttgct aaataatagt aagaaatag                             99


<210>  103
<211>  420
<212>  DNA
<213>  Artificial Sequence

<220>
<223>  DNA IntC AceL-1_UvsW-3-1

<400>  103
atgaaattta acataccaat aggggaacta gcagagtcga tcgcgaagta caaaggagta      60

ctattaaacg ataactgcga aattaatatt aaagatcttg attgtaaagt taatacacca     120

tcaggaactg ctactattaa tattataatt aaaaaagaaa agttagaagg cataaaacta     180

ttacttgcaa atggtgtaga aataaagtgt gctaataagc atatattaag atataataat     240

gcagacgtat ttgcagattc attagcaatt ggcgactcgg tagaaactat taacgggaat      300

gttaaggtta gtagtattaa caatattgac gatactacat tttacgatat cggaatagat     360

gcaccgtact tatattatga tgcagacgga gtattacatc ataatacaat taccacagca     420


<210>  104
<211>  37
<212>  PRT
<213>  Artificial Sequence

<220>
<223>  IntN AceL-1_gp41-1

<400>  104

Cys Phe Phe Ser Asp Gly Glu Ile Asn Thr Arg Asn Ile Ser Asn Lys
1               5                   10                  15

Glu Ile Lys Ser Ile Lys Ile Gly Lys Ile Phe Thr Asn Ile Ser Lys
            20                  25                  30

Gly His Thr Asn Ile
        35

<210>  105
<211>  148
<212>  PRT
<213>  Artificial Sequence

<220>
<223>  IntC AceL-1_gp41-1

<400>  105

Met Leu Asp Asn Tyr Glu Ile Ile Glu Ala Asp Ser Leu Leu Glu Gly
1               5                   10                  15

Lys Tyr Asp Arg Pro Leu Tyr Asp Lys Phe Ile Glu Ala Tyr Glu Val
            20                  25                  30

Asp Asn Leu Glu Val Asp Thr Pro Asn Gly Trp Ile Lys Ile Glu Gly
        35                  40                  45

Ile Gly Lys Thr Ile Glu Phe Tyr Glu Trp Glu Ile Gln Thr Ser Gly
    50                  55                  60

Gly Lys His Leu Ile Cys Ala Asp Lys His Leu Leu Tyr Arg Cys Asp
65                  70                  75                  80

Asn Met Asn Phe Tyr Asn Lys Lys Cys Asp Ile Thr Glu Ile Tyr Cys
                85                  90                  95

Gln Asp Leu Asn Ile Gly Asp Phe Ile Met Thr Lys Asp Gly Pro Glu
            100                 105                 110

Met Leu Met Asp Ile Tyr Lys Asn Gly Asn Lys Ser Asn Met Tyr Asp
        115                 120                 125

Leu Gln Leu Ser Glu Gly Ser Asn Lys Gln Tyr Tyr Thr Asn Asp Ile
    130                 135                 140

Leu Ser His Asn
145

<210>  106

<211>  5
<212>  PRT
<213>  Artificial Sequence

<220>
<223>  IntN AceL-1_gp41-1flanking sequence

<400>  106

Ser Leu Trp Met Gln
1               5


<210>  107
<211>  5
<212>  PRT
<213>  Artificial Sequence

<220>
<223>  IntC AceL-1_gp41-1flanking sequence

<400>  107

Thr Asn Gly Gly Lys
1               5


<210>  108
<211>  129
<212>  DNA
<213>  Artificial Sequence

<220>
<223>  DNA  IntN AceL-1_gp41-1

<400>  108
acaaacggag gaaaatgttt ttttagtgat ggtgagataa atactaggaa tataagcaat        60

aaggaaataa aatcaattaa aataggtaaa atttttacca acattagcaa gggacatact       120

aacatttaa                                                               129


<210>  109
<211>  459
<212>  DNA
<213>  Artificial Sequence

<220>
<223>  DNA IntC AceL-1_gp41-1

<400>  109
atgttagata attatgaaat aatagaagca gattctctat tagaaggaaa atatgataga        60

ccactatatg ataaatttat tgaagcttat gaagtagaca acttagaggt tgatacacca       120

aatggttgga taaagataga aggaattggt aaaactattg aattttatga atgggaaata       180

caaacatctg gtggaaaaca tctaatatgt gcagataaac atctattata taggtgtgat       240

aatatgaatt tttataataa aaaatgtgac ataacagaaa tatactgcca agatttgaat       300

ataggtgatt ttataatgac taaggatggt cctgagatgt tgatggatat ttataaaaat       360

ggtaataaat cgaatatgta tgatttacaa ttatcagaag gctctaataa acaatactac 420

acaaatgata tacttagtca taattcactt tggatgcaa 459

<210> 110
<211> 56
<212> PRT
<213> Artificial Sequence

<220>
<223> IntN AceL-1_gp46-1

<400> 110

Cys Val Asp Glu Ser Thr Leu Ile Asp Val Gln Ile Ile Asp Phe Glu
1               5                   10                  15

Pro Asn Leu Glu Asn Leu Glu Phe Leu Asp Lys Thr Asp Glu Gly Lys
            20                  25                  30

Arg Ile Phe Leu Tyr Ile Lys Lys Ser Asn Lys Ser Leu Tyr Glu Lys
        35                  40                  45

Ile Glu Lys Phe Arg Lys Gly Gln
    50                  55

<210> 111
<211> 122
<212> PRT
<213> Artificial Sequence

<220>
<223> IntC AceL-1_gp46-1

<400> 111

Met Leu Thr Leu Lys Ile Gly Asp Leu Tyr Glu Leu Ser Lys Asn Ile
1               5                   10                  15

Asn Ile Leu Glu Ser Asp Ile Arg Val Ser Thr Pro Gly Gly Leu Lys
            20                  25                  30

Lys Val Phe Ala Val Asp Ile Thr Ala Lys Asn Ser Asp Val Phe Ser
        35                  40                  45

Ile Lys Val Asn Lys His Glu Leu Leu Cys Ser Pro Asp His Leu Ile
    50                  55                  60

Arg Ser Glu Asp Met Trp Val Lys Ser Lys Asp Leu Lys Ile Asn Ser
65                  70                  75                  80

Val Ile Asp Thr Lys Tyr Gly Lys Leu Thr Val Lys Glu Ile Ser Ile

85                  90                  95

Leu Asp Ile Lys Ser Asp Leu Met Asp Leu His Val Asp Gly Ser Glu
            100                 105                 110

Tyr Tyr Thr Asn Asp Ile Ile Ser His Asn
        115                 120

<210>  112
<211>  5
<212>  PRT
<213>  Artificial Sequence

<220>
<223>  IntN AceL-1_gp46-1 flanking sequence

<400>  112

Asn Gly Ser Gly Lys
1               5

<210>  113
<211>  5
<212>  PRT
<213>  Artificial Sequence

<220>
<223>  IntC AceL-1_gp46-1 flanking sequence

<400>  113

Ser Ser Leu Leu Asp
1               5

<210>  114
<211>  186
<212>  DNA
<213>  Artificial Sequence

<220>
<223>  DNA  IntN  AceL-1_gp46-1

<400>  114
aatggttctg gtaagtgtgt tgatgagtca acactaatag atgtacaaat aattgatttt      60

gagcctaatt tagaaaattt agaattttta gacaaaacgg atgagggaaa gaggattttt     120

ctatatataa agaaatctaa taaatccttg tatgaaaaaa ttgaaaaatt tagaaaaggt     180

caataa                                                                186

<210>  115
<211>  381
<212>  DNA
<213>  Artificial Sequence

<220>

<223>  DNA  IntC AceL-1_gp46-1

<400>  115
atgttaacat taaagatagg tgatttatat gaattatcaa aaaatataaa tattttagaa        60

tcagacatcc gtgtatctac cccaggtgga ttgaaaaagg tttttgctgt tgatataaca       120

gcaaaaaata gcgatgtgtt ttctataaaa gttaataaac atgaactact ttgctcacca       180

gatcatctaa taagatcaga agatatgtgg gttaaatcta aagatttaaa aataaattcc       240

gtaatagata caaaatatgg caaacttact gttaaggaga tatcaatttt ggatataaag       300

agtgatttga tggatttaca tgtggatggt agtgaatatt acactaatga tataattagt       360

cacaactcat cactattaga t                                                 381


<210>  116
<211>  15
<212>  PRT
<213>  Artificial Sequence

<220>
<223>  IntN VidaL_T4Lh-1

<400>  116

Cys Val His Pro Asp Thr Lys Val Thr Ile Arg Arg Lys Leu Cys
1               5                   10                  15


<210>  117
<211>  155
<212>  PRT
<213>  Artificial Sequence

<220>
<223>  IntC VidaL_T4Lh-1

<400>  117

Met Lys Glu Leu Leu Asp Leu Tyr Thr Glu Lys Glu Ile Asn Lys Leu
1               5                   10                  15


Leu Glu Arg Tyr Thr Ile Asp Gln Ile Ile Asp Tyr Ser Gln Pro His
            20                  25                  30


Val Val Ser Val Gly Ser Ile Lys Glu Glu Met Asp Ser Gly Asn Phe
        35                  40                  45


Ile Phe Val Asp Ser Pro Asp Gly Tyr Val Ala Val Ser Asp Phe Val
    50                  55                  60


Asp Lys Gly Asn Phe Glu Glu Tyr Arg Phe Thr Tyr Asp Lys Lys Ile
65                  70                  75                  80


Ile Arg Thr Asn Glu Gly His Leu Phe Gln Thr His Leu Gly Trp Glu

85                  90                  95

Thr Ser Lys Asn Leu Tyr Lys Met Tyr Leu Ala Gly His Pro Ile Tyr
            100                 105                 110

Ile Leu His Lys Asn Gly Gly Tyr Lys Lys Ile Asp Ile Glu Lys Thr
        115                 120                 125

Gly Asn Val Ile Pro Ile Val Asp Ile Val Val Glu His Lys Asn His
    130                 135                 140

Arg Tyr Tyr Thr Asp Gly Leu Ser Ser His Asn
145                 150                 155

<210>  118
<211>  5
<212>  PRT
<213>  Artificial Sequence

<220>
<223>  IntN VidaL_T4Lh-1 flanking sequence

<400>  118

Val Phe Leu Ala Ser
1               5

<210>  119
<211>  5
<212>  PRT
<213>  Artificial Sequence

<220>
<223>  IntC VidaL_T4Lh-1 flanking sequence

<400>  119

Thr Asn Val Gly Lys
1               5

<210>  120
<211>  63
<212>  DNA
<213>  Artificial Sequence

<220>
<223>  DNA  IntN  VidaL_T4Lh-1

<400>  120
gtatttttgg ctagttgtgt gcatccagat acaaaagtaa caattcgtag aaaactttgt      60

tag                                                                     63

<210>  121
<211>  480

<212>  DNA
<213>  Artificial Sequence

<220>
<223>  DNA IntC VidaL_T4Lh-1

<400>  121
atgaaagaat tgcttgactt atacacagaa aaagaaataa ataaattatt agaaagatac      60

acaatagacc agattataga ctactcacaa cctcatgtgg tttctgtggg tagtataaaa     120

gaagaaatgg attcaggaaa tttcattttt gttgacagcc cagatggtta cgttgctgtt     180

agtgattttg tagacaaagg aaactttgaa gaatataggt ttacatatga taaaaaaata     240

atccgaacaa acgaaggtca cttattccaa acacatttgg gttgggagac ttctaagaat     300

ttatataaaa tgtacttagc tggtcacccc atttatatat tgcataaaaa tggtggttat     360

aaaaagattg atatagaaaa gaccggaaac gtgattccta tcgttgatat tgtggtggaa     420

cacaaaaacc atagatatta tacggatgga ttgtccagcc ataacacaaa tgtgggcaag     480


<210>  122
<211>  16
<212>  PRT
<213>  Artificial Sequence

<220>
<223>  IntN VidaL_UvsX-2

<400>  122

Cys Leu Pro Lys Glu Ala Val Val Gln Ile Arg Leu Thr Lys Lys Gly
1               5                   10                  15


<210>  123
<211>  123
<212>  PRT
<213>  Artificial Sequence

<220>
<223>  IntC VidaL_UvsX-2

<400>  123

Met Ile Glu Glu Lys Lys Val Thr Val Gln Glu Leu Arg Glu Leu Tyr
1               5                   10                  15


Leu Ser Gly Glu Tyr Thr Ile Glu Ile Asp Thr Pro Asp Gly Tyr Gln
            20                  25                  30


Thr Ile Gly Lys Trp Phe Asp Lys Gly Val Leu Ser Met Val Arg Val
        35                  40                  45


Ala Thr Ala Thr Tyr Glu Thr Val Cys Ala Phe Asn His Met Ile Gln
    50                  55                  60

Leu Ala Asp Asn Thr Trp Val Gln Ala Cys Glu Leu Asp Val Gly Val
65                  70                  75                  80

Asp Ile Gln Thr Ala Ala Gly Ile Gln Pro Val Met Leu Val Glu Asp
                85                  90                  95

Thr Ser Asp Ala Glu Cys Tyr Asp Phe Glu Val Met His Pro Asn His
            100                 105                 110

Arg Tyr Tyr Gly Asp Gly Ile Val Ser His Asn
        115                 120

<210>  124
<211>  5
<212>  PRT
<213>  Artificial Sequence

<220>
<223>  IntN VidaL_UvsX-2 flanking sequence

<400>  124

Ser Gly Lys Ser Tyr
1               5

<210>  125
<211>  5
<212>  PRT
<213>  Artificial Sequence

<220>
<223>  IntC VidaL_UvsX-2 flanking sequence

<400>  125

Ala Gly Glu Ser Gly
1               5

<210>  126
<211>  66
<212>  DNA
<213>  Artificial Sequence

<220>
<223>  DNA  IntN  VidaL_UvsX-2

<400>  126
gctggcgaaa gtggctgttt gccaaaagaa gcagtagtac agattcgatt aacaaaaaaa        60

ggctag                                                                    66

<210>  127
<211>  384
<212>  DNA
<213>  Artificial Sequence

<220>
<223>  DNA  IntC  VidaL_UvsX-2

<400>  127
atgattgaag aaaagaaagt aacagtacaa gagcttagag agctatatct cagcggcgag        60

tatactattg agattgacac accggacgga tatcagacta tcggaaaatg gtttgacaaa       120

ggggtattgt ccatggttag agttgccaca gccacttacg aaacagtgtg tgcatttaat       180

catatgattc aactggctga caatacgtgg gtacaagcct gtgagttaga tgtaggagta       240

gatatacaaa cggcggcagg catccagcct gttatgttag tcgaagatac aagtgatgca       300

gagtgttacg attttgaagt catgcatccg aatcatagat attacggtga cggaattgta       360

agccataact cggggaaaag ttat                                              384


<210>  128
<211>  28
<212>  PRT
<213>  Artificial Sequence

<220>
<223>  IntN VidaL_TerL-6-1

<400>  128

Ser Leu Ala His Glu Thr Ile Val Ser Ile Asn Asp Asn Asn Thr Leu
1               5                   10                  15


Thr Ser Met Cys Ile Gly Asp Leu Tyr Asp Tyr Met
            20                  25


<210>  129
<211>  132
<212>  PRT
<213>  Artificial Sequence

<220>
<223>  IntC VidaL_TerL-6-1

<400>  129

Met Asp Tyr His Ser Asn Gln Val Ser Arg Ile Phe Gly Val Gly Met
1               5                   10                  15


Ser Lys Val His Leu Gly Phe Lys Lys Asn Thr Lys Asn Leu Lys Val
            20                  25                  30


Leu Thr Pro Asn Gly His Glu Glu Phe Tyr Gly Ile Asn Lys Ile Arg
        35                  40                  45


Val Asp Glu Tyr Ile Arg Ile Lys Phe Lys Glu His Lys Glu Ile Arg
    50                  55                  60

Cys Ser Ile Asp His Pro Phe Ile Gln Glu Asn Asp Leu Pro Ile Lys
65                  70                  75                  80

Ala Lys His Ile Asp Lys Ser Lys His Ile Lys Cys Ile Asp Gly Phe
                85                  90                  95

Thr Thr Leu Glu Tyr Ser His Val Val Asn Lys Gln Ile Glu Leu Tyr
            100                 105                 110

Asp Ile Val Asn Ser Gly Ser Glu Tyr Ile Tyr Phe Ser Asn Gly Ile
        115                 120                 125

Leu Ser His Asn
    130

<210>  130
<211>  5
<212>  PRT
<213>  Artificial Sequence

<220>
<223>  IntN VidaL_TerL-6-1 flanking sequence

<400>  130

Ser Val Glu Tyr Glu
1               5

<210>  131
<211>  5
<212>  PRT
<213>  Artificial Sequence

<220>
<223>  IntC VidaL_TerL-6-1 flanking sequence

<400>  131

Cys Lys Phe Met Gly
1               5

<210>  132
<211>  102
<212>  DNA
<213>  Artificial Sequence

<220>
<223>  DNA  IntN  VidaL_TerL-6-1

<400>  132
tccgtggaat atgagagttt ggcacacgaa actatagtaa gtataaatga taataacaca       60

ctaacaagta tgtgcattgg agatttatat gactatatgt aa                          102

<210>  133
<211>  411
<212>  DNA
<213>  Artificial Sequence

<220>
<223>  DNA Intc  VidaL_TerL-6-1

<400>  133
ttggattacc actctaatca agtgtctcga atttttggag ttggtatgag caaagtacat      60

ctagggttta aaaagaacac taaaaattta aaagtgttaa caccaaatgg acacgaagaa     120

ttctacggaa taaacaaaat acgtgtcgat gaatatatac gaataaaatt caaagaacat     180

aaagaaatac gttgctcgat tgaccacccg tttatacaag aaaatgattt accaataaaa     240

gcaaaacata ttgataaaag caaacatata aaatgtattg atggatttac tactttagag     300

tattcgcatg ttgttaataa acaaattgaa ctatatgata ttgtaaactc tggtagtgag     360

tacatatatt tttctaatgg gatattaagt cacaactgta aattcatggg t              411


<210>  134
<211>  30
<212>  PRT
<213>  Artificial Sequence

<220>
<223>  IntN TerL-7-1_VidaL

<400>  134

Cys Leu Trp Gly Ala Ser Thr Val Asn Val Phe Asp Ser Leu Thr Gly
1               5                   10                  15


Lys Asn Ile Asp Ile Lys Leu Glu Asp Leu Tyr Gln Lys Leu
            20                  25                  30


<210>  135
<211>  114
<212>  PRT
<213>  Artificial Sequence

<220>
<223>  IntC TerL-7-1_VidaL

<400>  135

Met Glu Ser Tyr Thr Phe Arg Lys Asn Thr Arg Tyr Lys Ile Met Thr
1               5                   10                  15


Pro Ala Gly Tyr Gln Asn Phe Gly Gly Ile Arg Lys Leu Asn Lys Asn
            20                  25                  30


Val His Tyr Ile Val Glu Leu Ser Asn Lys Lys Ile Leu Lys Cys Ser
        35                  40                  45

Thr Thr His Pro Phe Ile Tyr Asn Asp Arg Glu Ile Phe Ala Asn Lys
    50                  55                  60

Leu Lys Val Gly Ser Leu Leu Asp Ser Thr Ser Lys Lys Lys Ile Ser
65                  70                  75                  80

Val Ile Ser Ile Glu Leu Asp Lys Ser Lys Ile Asp Leu Tyr Asp Ile
                85                  90                  95

Val Glu Val Asn Asn Gly Asn Ile Phe Asn Val Asp Gly Ile Val Ser
            100                 105                 110

His Asn

<210>  136
<211>  5
<212>  PRT
<213>  Artificial Sequence

<220>
<223>  IntN TerL-7-1_VidaL flanking sequence

<400>  136

Ser Gln Glu Cys Asp
1               5

<210>  137
<211>  1
<212>  DNA
<213>  Artificial Sequence

<220>
<223>  IntC TerL-7-1_VidaL flanking sequence

<400>  137
c                                                                        1

<210>  138
<211>  108
<212>  DNA
<213>  Artificial Sequence

<220>
<223>  DNA IntN TerL-7-1_VidaL

<400>  138
tcccaagaat gcgattgttt gtggggcgca tctactgtaa atgtatttga tagtttaact      60

ggaaaaaaca ttgatataaa actcgaagat ttgtatcaaa aactttaa                  108

<210>  139
<211>  345

<212>  DNA
<213>  Artificial Sequence

<220>
<223>  DNA  IntC  TerL-7-1_VidaL

<400>  139
atggaatcat atacttttag aaaaaacaca agatataaaa taatgacacc agcaggatat        60

caaaactttg gtggtattag aaaattgaat aaaaatgtac attatatagt tgaattatcc       120

aataaaaaaa tattaaaatg ttcaactaca catccattta tttataatga tagagagata       180

tttgcaaata aattaaaagt cggtagttta cttgatagta ctagtaaaaa gaaaatttca       240

gtaatatcaa ttgaattaga taaatcaaaa atagatttat atgatatagt agaagtaaat       300

aatggtaata tttttaatgt agatggtatt gtttcacata attgt                       345


<210>  140
<211>  31
<212>  PRT
<213>  Artificial Sequence

<220>
<223>  IntN VidaL_TerL-3

<400>  140

Cys Val Ser Ala Ser Thr Ile Ile Thr Leu Gln Asp Thr His Gly Asn
1               5                   10                  15


Ile Phe Asp Ser Gln Ile Gly Asp Leu Tyr Asn Thr Ile Gly Lys
            20                  25                  30


<210>  141
<211>  114
<212>  PRT
<213>  Artificial Sequence

<220>
<223>  IntC VidaL_TerL-3

<400>  141

Met Ser Lys Ile Phe Lys Glu Asn Thr Asn Gly Tyr Lys Val Leu Thr
1               5                   10                  15


Pro Ala Gly Phe Gln Asp Phe Ala Gly Val Ser Met Met Gly Ile Lys
            20                  25                  30


Pro Leu Leu Arg Leu Glu Phe Glu Arg Gly Ala Tyr Val Glu Cys Thr
        35                  40                  45


Tyr Asp His Lys Phe Tyr Ile Asp Leu Glu Thr Cys Lys Pro Ala Gln
    50                  55                  60

Asp Ile Ala Val Gly Asn Thr Val Val Thr Ser Glu Gly Asp Ile Lys
65                  70                  75                  80

Leu Leu Asn Lys Ile Glu Leu Gly Tyr Ser Glu Pro Val Tyr Asp Leu
                85                  90                  95

Ile Gln Val Glu Gly Gly His Arg Tyr Tyr Thr Asn Lys Ile Leu Ser
            100                 105                 110

Ser Asn

<210>  142
<211>  5
<212>  PRT
<213>  Artificial Sequence

<220>
<223>  IntN VidaL_TerL-3 flanking sequence

<400>  142

Arg Arg Glu Tyr Gly
1               5

<210>  143
<211>  5
<212>  PRT
<213>  Artificial Sequence

<220>
<223>  IntC VidaL_TerL-3 flanking sequence

<400>  143

Cys Glu Phe Leu Val
1               5

<210>  144
<211>  111
<212>  DNA
<213>  Artificial Sequence

<220>
<223>  DNA  IntN  VidaL_TerL-3

<400>  144
cgtcgtgagt acggttgtgt gagcgcatct acaatcatta ctctccaaga cacacacggt      60

aatatatttg actcacaaat aggcgacttg tacaatacga taggtaaata a              111

<210>  145
<211>  357
<212>  DNA
<213>  Artificial Sequence

<220>
<223>  DNA  IntC  VidaL_TerL-3

<400>  145
atgagcaaga tttttaaaga gaatactaat ggatataagg tgttaacacc agcggggttt        60

caagactttg ctggtgttag catgatggga ataaaaccgt tgcttcggct agagttcgag       120

cgaggcgcct acgtcgaatg cacctacgat cataaatttt acatagacct agaaacttgt       180

aagccagccc aagacattgc agtaggaaac actgtggtta cttctgaggg tgatataaaa       240

ttactcaaca aaatagaact gggttattca gaacctgttt atgatcttat acaagttgaa       300

ggcggccacc gatattacac aaacaaaata ctcagctcaa attgcgaatt tttagta          357


<210>  146
<211>  32
<212>  PRT
<213>  Artificial Sequence

<220>
<223>  IntN VidaL_TerL-1

<400>  146

Cys Val Gln Ala Asp Thr Lys Tyr Thr Ile Arg Asn Lys Ile Ser Gly
1               5                   10                  15


Asp Val Leu Asn Val Thr Ala Glu Glu Phe His Lys Met Gln Lys Lys
            20                  25                  30


<210>  147
<211>  121
<212>  PRT
<213>  Artificial Sequence

<220>
<223>  IntC VidaL_TerL-1

<400>  147

Met Lys Leu Ser Asn Phe Thr Asn Arg Lys Phe Ile Glu Thr Ile Asp
1               5                   10                  15


Ala Ser Glu Trp Glu Val Glu Thr Cys Glu Gly Phe Lys Pro Ile Ile
            20                  25                  30


Ser Ser Asn Lys Thr Ile Glu Tyr Val Val Tyr Lys Ile Glu Leu Glu
        35                  40                  45


Asn Gly Leu Ser Ile Lys Cys Ala Asp Thr His Ile Leu Ile Asp Lys
    50                  55                  60


Asn Leu Gln Glu Ile Tyr Ala Lys Asp Ser Phe Asn Lys Ile Ile Phe

65                  70                  75                  80

Thr Lys Phe Gly Asn Ser Lys Val Ile Ser Val Glu Thr Leu Asn Ile
                85                  90                  95

Ser Glu Asn Met Tyr Asp Leu Ser Val Asp Ser Glu Asp His Thr Tyr
            100                 105                 110

Tyr Thr Asp Asp Ile Leu Ser His Asn
        115                 120

<210>  148
<211>  5
<212>  PRT
<213>  Artificial Sequence

<220>
<223>  IntN VidaL_TerL-1 flanking sequence

<400>  148

Arg Gln Gln Gly Lys
1               5

<210>  149
<211>  5
<212>  PRT
<213>  Artificial Sequence

<220>
<223>  IntC VidaL_TerL-1 flanking sequence

<400>  149

Thr Thr Thr Ala Ala
1               5

<210>  150
<211>  114
<212>  DNA
<213>  Artificial Sequence

<220>
<223>  DNA  IntN VidaL_TerL-1

<400>  150
agacaacaag gtaagtgcgt tcaagcagac actaaataca ctataagaaa caaaattagt        60

ggtgatgtgt taaatgttac agcagaagaa ttccacaaaa tgcagaaaaa ataa              114

<210>  151
<211>  378
<212>  DNA
<213>  Artificial Sequence

<220>

<223>  DNA  IntC VidaL_TerL-1

<400>  151
atgaagctat ccaatttcac caatagaaaa tttatagaaa caattgatgc tagtgaatgg      60

gaagtagaaa catgcgaagg tttcaaaccc atcattagtt caaataaaac tattgaatat     120

gtagtctata aaattgaact agaaaatgga ttatctatta aatgtgcaga cactcatatt     180

ttaatagata aaaatttgca agaaatttat gcaaaagata gttttaataa aataatattt     240

acaaagttcg gaaactcaaa agttatttcc gtagaaactt taaatatatc tgaaaatatg     300

tatgatcttt ctgttgattc agaagatcac acatactata cagatgatat cttatcacat     360

aataccacga ccgccgca                                                   378


<210>  152
<211>  32
<212>  PRT
<213>  Artificial Sequence

<220>
<223>  IntN VidaL_gp46-1

<400>  152

Cys Leu Cys Ile Asn Thr Ile Val Lys Val Lys Asn Thr Lys Thr Gly
1               5                   10                  15


Val Ile Tyr Glu Thr Thr Ile Gly Glu Leu Tyr Asn Gly Ala Met Glu
            20                  25                  30


<210>  153
<211>  124
<212>  PRT
<213>  Artificial Sequence

<220>
<223>  IntC VidaL_gp46-1

<400>  153

Met Ser Thr Ile Ser Gln Thr Val Asn Arg Lys Phe Val Asn Ser Phe
1               5                   10                  15


Ser Leu Val Asp Leu Glu Ile Glu Thr Asp Ser Gly Trp Gln Pro Val
            20                  25                  30


Thr Asp Ile His Lys Thr Ile Pro Tyr Thr Val Trp His Ile Glu Thr
        35                  40                  45


Gln Ser Gly Leu Thr Leu Asp Cys Ala Asp Thr His Ile Leu Phe Asp
    50                  55                  60


His Asn Tyr Asn Glu Ile Phe Val Lys Asp Ile Ile Pro Asn Gln Thr

65                  70                  75                  80

Lys Ile Ile Ser Lys His Gly Pro Glu Leu Val Leu Thr Val Ile Glu
                85                  90                  95

Gln Ser Gln Gln Glu Asn Met Phe Asp Leu Thr Val Asp His Pro Asp
            100                 105                 110

His Arg Phe Tyr Ser Asn Asn Ile Leu Ser His Asn
        115                 120

<210>  154
<211>  5
<212>  PRT
<213>  Artificial Sequence

<220>
<223>  IntN VidaL_gp46-1 flanking sequence

<400>  154

Asn Gly Thr Gly Lys
1               5

<210>  155
<211>  5
<212>  PRT
<213>  Artificial Sequence

<220>
<223>  IntC VidaL_gp46-1 flanking sequence

<400>  155

Thr Thr Val Ile Asn
1               5

<210>  156
<211>  114
<212>  DNA
<213>  Artificial Sequence

<220>
<223>  DNA  IntN  VidaL_gp46-1

<400>  156
aacggaacgg gcaagtgcct ttgtataaat actattgtaa aagtaaaaaa caccaaaact      60

ggggtaattt acgaaactac aataggagaa ttatacaatg gcgcgatgga ataa           114

<210>  157
<211>  387
<212>  DNA
<213>  Artificial Sequence

<220>

<223>  DNA  IntC VidaL_gp46-1

<400>  157
atgtctacaa tttctcaaac agtaaataga aaatttgtta atagtttcag cttagttgat      60

cttgaaatcg aaacagattc tggatggcag cctgttactg acatacacaa gactattcca     120

tacacagttt ggcatatcga aacccaaagc gggctgactc ttgactgtgc cgacactcat     180

atcttgtttg atcacaacta caacgagatc tttgtcaaag atataatacc taaccagact     240

aagataatat ctaagcacgg tcctgaatta gtattaacag taatcgaaca gtctcagcaa     300

gaaaatatgt ttgatctaac agttgatcat cctgatcatc gtttttactc aaacaatatc     360

ttatctcaca ataccacagt gataaat                                         387


<210>  158
<211>  43
<212>  PRT
<213>  Artificial Sequence

<220>
<223>  IntN VidaL_gp41-1

<400>  158

Cys Val Ile Ala Glu Thr Glu Val Lys Ile Ile Glu Leu Tyr Asn Ile
1               5                   10                  15


Asp Ser Phe Ile Lys Thr Gly Ile Leu Asn Ser Gln Gly Val Val Ser
            20                  25                  30


Trp Glu Glu Thr Ser Ser His Gly Arg Thr Arg
        35                  40


<210>  159
<211>  154
<212>  PRT
<213>  Artificial Sequence

<220>
<223>  IntC VidaL_gp41-1

<400>  159

Met Asn Leu Leu Asp Lys Arg Val Glu Trp Leu Ser Lys Trp Tyr Pro
1               5                   10                  15


Val Asp Lys Leu Gln Gln Leu Ser Glu Asp Lys Leu Val Leu Leu Tyr
            20                  25                  30


Asn Asn Ser Gln Pro Lys Lys Val Arg Met Gly Ser Leu Glu Gly Val
        35                  40                  45


Pro Thr Ser Ser Tyr Arg Ile Ser Ser Pro Asp Gly Tyr Val Thr Ala

50                  55                  60

His Ala Trp Arg Asn Lys Gly Thr Lys Glu Cys Val Thr Leu Thr Thr
65                  70                  75                  80

Asp Ser Gly Asn Ser Ile Thr Ala Ser Thr Asp His Phe Phe Glu Met
                85                  90                  95

Ser Asp Gly Lys Trp Lys Tyr Ala Gly Cys Leu Phe Pro Gly Gln Cys
            100                 105                 110

Ile Ser Thr Glu Ser Gly Thr Glu Thr Val Thr Ser Val Val Ala Ala
        115                 120                 125

Gly Lys His Thr Val Tyr Asp Phe Tyr Ile Asp His Glu Asn His Arg
    130                 135                 140

Tyr Tyr Thr Asn Gly Ile Ser Ser His Asn
145                 150

<210>  160
<211>  5
<212>  PRT
<213>  Artificial Sequence

<220>
<223>  IntN VidaL_gp41-1 flanking sequence

<400>  160

Ile Phe Ala Gly Gly
1               5

<210>  161
<211>  5
<212>  PRT
<213>  Artificial Sequence

<220>
<223>  IntC VidaL_gp41-1 flanking sequence

<400>  161

Ser Gly Ala Gly Lys
1               5

<210>  162
<211>  147
<212>  DNA
<213>  Artificial Sequence

<220>
<223>  DNA I IntN  VidaL_gp41-1

<400>  162
atatttgccg gcggatgtgt aattgccgaa actgaggtaa aaataattga attatacaac      60

attgacagct ttattaaaac aggaatactt aacagccaag gggtcgtttc gtgggaagaa     120

acttcttccc acggtcgtac tcgatag                                         147


<210>  163
<211>  477
<212>  DNA
<213>  Artificial Sequence

<220>
<223>  DNA IntC VidaL_gp41-1

<400>  163
atgaatttac tagataagag agttgagtgg ttatctaagt ggtacccagt agacaagtta      60

caacaattat cagaagacaa gttggtactc ctatataaca atagtcagcc aaaaaaagtc     120

cgcatgggat cgttggaagg tgttccgacc agcagttatc ggatcagcag ccccgacggg     180

tatgttaccg cacatgcctg gcgaaataaa ggaactaaag agtgtgttac tctaaccaca     240

gactctggaa attctataac tgctagcaca gatcattttt tcgaaatgtc cgacggcaag     300

tggaaatatg caggctgttt gtttccagga cagtgcatta gcacagaatc cggcaccgaa     360

acagtgacca gcgtagtagc agcaggtaag cacacagtat acgacttcta catcgatcat     420

gaaaatcaca gatactatac caatggaata agcagtcata actctggtgc aggcaaa        477


<210>  164
<211>  29
<212>  PRT
<213>  Artificial Sequence

<220>
<223>  IntN GS013_ter-3

<400>  164

Cys Leu Gly Gly Asp Thr Glu Ile Glu Ile Leu Asp Asp Asn Gly Ile
1               5                   10                  15


Val Gln Lys Thr Ser Met Glu Asn Leu Tyr Glu Arg Leu
            20                  25


<210>  165
<211>  103
<212>  PRT
<213>  Artificial Sequence

<220>
<223>  IntC GS013_ter-3

<400>  165

Met Phe Lys Ile Asn Lys Asn Ile Lys Val Lys Thr Pro Asp Gly Phe

1               5                   10                  15

Lys Asp Phe Ser Gly Ile Gln Lys Val Tyr Lys Pro Phe Tyr His Trp
            20                  25                  30

Ile Ile Phe Asp Asp Gly Ser Glu Ile Lys Cys Ser Asp Asn His Ser
        35                  40                  45

Phe Gly Lys Glu Lys Ile Lys Ala Ser Thr Ile Lys Val Asp Asp Ile
    50                  55                  60

Leu Gln Glu Lys Lys Val Leu Tyr Asn Glu Ile Val Glu Glu Gly Ile
65                  70                  75                  80

Tyr Leu Tyr Asp Leu Leu Asp Val Gly Glu Asp Asn Leu Tyr Tyr Ser
                85                  90                  95

Asn Asn Ile Val Ser His Asn
            100


<210>  166
<211>  5
<212>  PRT
<213>  Artificial Sequence

<220>
<223>  IntN GS013_ter-3 flanking sequence

<400>  166

Arg Val Glu Phe Glu
1               5


<210>  167
<211>  5
<212>  PRT
<213>  Artificial Sequence

<220>
<223>  IntC GS013_ter-3 flanking sequence

<400>  167

Cys Glu Phe Leu Gly
1               5


<210>  168
<211>  105
<212>  DNA
<213>  Artificial Sequence

<220>
<223>  DNA  IntN  GS013_ter-3

<400>  168
cgggttgagt ttgaatgttt gggtggtgat acagagattg aaattttgga tgataatgga      60

attgtacaaa aaacttctat ggaaaattta tatgaacgat tgtga                     105


<210>  169
<211>  324
<212>  DNA
<213>  Artificial Sequence

<220>
<223>  DNA IntC GS013_ter-3

<400>  169
atgtttaaga ttaataaaaa tattaaagta aaaacacctg atggatttaa agatttttca      60

ggaatacaaa aagtttataa acctttttac cattggataa tatttgatga cggatcagaa     120

ataaaatgct ccgataatca ttctttcgga aaagaaaaaa ttaaggcatc aacaattaaa     180

gttgatgata ttttacaaga aaagaaagta ttatataatg aaatagtaga agaaggaatt     240

tatctttatg atttacttga tgttggcgaa gacaatcttt actattcaaa caatatagta     300

tcacacaact gcgagttctt gggt                                            324


<210>  170
<211>  29
<212>  PRT
<213>  Artificial Sequence

<220>
<223>  IntN GS013_ter-2

<400>  170

Cys Leu Gly Gly Asp Thr Glu Ile Glu Ile Leu Asp Asp Asn Gly Ile
1               5                   10                  15


Val Gln Lys Thr Ser Met Glu Asn Leu Tyr Glu Arg Leu
            20                  25


<210>  171
<211>  108
<212>  PRT
<213>  Artificial Sequence

<220>
<223>  IntC GS013_ter-2

<400>  171

Met Ser Val Gly Lys Met Phe Lys Ile Asn Lys Asn Ile Lys Val Lys
1               5                   10                  15


Thr Pro Asp Gly Phe Lys Asp Phe Ser Gly Ile Gln Lys Val Tyr Lys
            20                  25                  30

Pro Phe Tyr His Trp Ile Ile Phe Asp Asp Gly Ser Glu Ile Lys Cys
        35                  40                  45

Ser Asp Asn His Ser Phe Gly Lys Glu Lys Ile Lys Ala Ser Thr Ile
    50                  55                  60

Lys Val Asp Asp Ile Leu Gln Glu Lys Lys Val Leu Tyr Asn Glu Ile
65                  70                  75                  80

Val Glu Glu Gly Ile Tyr Leu Tyr Asp Leu Leu Asp Val Gly Glu Asp
                85                  90                  95

Asn Leu Tyr Tyr Ser Asn Asn Ile Val Ser His Asn
            100                 105

<210>  172
<211>  5
<212>  PRT
<213>  Artificial Sequence

<220>
<223>  IntN GS013_ter-2 flanking sequence

<400>  172

Arg Val Glu Phe Glu
1               5

<210>  173
<211>  5
<212>  PRT
<213>  Artificial Sequence

<220>
<223>  IntC GS013_ter-2 flanking sequence

<400>  173

Cys Glu Phe Leu Gly
1               5

<210>  174
<211>  105
<212>  DNA
<213>  Artificial Sequence

<220>
<223>  DNA  IntN  GS013_ter-2

<400>  174
cgtgttgagt ttgaatgttt gggtggtgat acagagattg aaattttgga tgataatgga      60

atagtacaaa aaacttctat ggaaaattta tatgaacgat tgtga                     105

<210>  175
<211>  339
<212>  DNA
<213>  Artificial Sequence

<220>
<223>  DNA I IntC  GS013_ter-2

<400>  175
atgagtgttg gaaaaatgtt taagattaat aaaaatatta aagtaaaaac acctgatgga      60

tttaaagatt tttcaggaat acaaaaagtt tataaacctt tttaccattg gataatattt     120

gatgacggat cagaaataaa atgctccgat aatcattctt tcggaaaaga aaaaattaag     180

gcatcaacaa ttaaagttga tgatatttta caagaaaaga aagtattata taatgaaata     240

gtagaagaag gaatttatct ttatgattta cttgatgttg gcgaagacaa tctttactat     300

tcaaacaata tagtatcaca caactgcgaa ttcttaggt                            339


<210>  176
<211>  39
<212>  PRT
<213>  Artificial Sequence

<220>
<223>  IntN GS013_ter-1

<400>  176

Cys Phe Asn Thr Asn Thr Thr Val Arg Leu Arg Asn Lys Leu Thr Gly
1               5                   10                  15


Glu Ile Ile Glu Val Thr Ile Gly Glu Phe Tyr Glu Lys Ile Lys Lys
            20                  25                  30


Glu Ser Asn Thr Asp Leu Pro
        35


<210>  177
<211>  115
<212>  PRT
<213>  Artificial Sequence

<220>
<223>  IntC GS013_ter-1


<220>
<221>  misc_feature
<222>  (8)..(9)
<223>  Xaa can be any naturally occurring amino acid

<220>
<221>  misc_feature
<222>  (78)..(78)
<223>  Xaa can be any naturally occurring amino acid

<220>
<221>  misc_feature
<222>  (92)..(92)
<223>  Xaa can be any naturally occurring amino acid

<400>  177

Met Ser Lys Phe Ile Glu Glu Xaa Xaa Thr Asp Glu Trp Glu Val Glu
1               5                   10                  15

Thr Pro Ser Gly Trp Gln Ser Phe Ser Gly Val Gly Lys Thr Ile Glu
            20                  25                  30

Tyr Glu Glu Trp Glu Val Val Thr Glu Thr Gly Lys Ser Leu Ile Cys
        35                  40                  45

Ala Asp Lys His Ile Leu Leu Asn Asp Lys Trp Gln Glu Val Tyr Cys
    50                  55                  60

Glu Asp Cys Ser Ile Asp Asp Cys Ile Gln Thr Lys Asn Xaa Ala Glu
65                  70                  75                  80

Lys Ile Leu Gln Leu Lys Lys Thr Ser Arg Ile Xaa Asn Met Tyr Asp
                85                  90                  95

Leu Leu Asp Val Asp Asn Gly Asn Ile Phe Tyr Ser Asn Glu Ile Val
            100                 105                 110

Ser His Asn
        115


<210>  178
<211>  5
<212>  PRT
<213>  Artificial Sequence

<220>
<223>  IntN GS013_ter-1 flanking sequence

<400>  178

Arg Gln Thr Gly Lys
1               5


<210>  179
<211>  5
<212>  PRT
<213>  Artificial Sequence

<220>
<223>  IntC GS013_ter-1 flanking sequence

<400>  179

Ser Thr Thr Val Val
1               5


<210>  180
<211>  135
<212>  DNA
<213>  Artificial Sequence

<220>
<223>  DNA  IntN  GS013_ter-1

<400>  180
cgtcagacgg gtaaatgttt taatacaaat acaacggtaa ggttaaggaa taaacttact      60

ggagaaatta ttgaagtgac tattggagaa ttttatgaaa aaatcaagaa agaaagtaat     120

actgatttgc cttga                                                      135


<210>  181
<211>  360
<212>  DNA
<213>  Artificial Sequence

<220>
<223>  DNA IntC GS013_ter-1

<400>  181
atgtctaaat ttattgaaga artamaaact gatgaatggg aagtagaaac tccttctgga      60

tggcaatctt tttctggggt aggaaaaact atagaatatg aagaatggga ggttgtaacc     120

gaaactggaa aatctcttat atgtgcagat aaacacatct tattaaatga taaatggcaa     180

gaagtttatt gtgargattg ttccattgat gactgtatac aaacaaaaaa tkgcgcagaa     240

aaaatattac aattaaaaaa aacatcaaga attyytaata tgtatgatct tcttgatgtt     300

gataatggta atatatttta cagtaatgaa atagtttcac acaattctac aactgttgtc     360


<210>  182
<211>  31
<212>  PRT
<213>  Artificial Sequence

<220>
<223>  IntN GS020_ter-7

<400>  182

Cys Val Asp Gly Ser Ser Ile Ile Thr Ile Lys Asn Lys Glu Thr Asn
1               5                   10                  15


Leu Ile Glu Lys Ile Thr Ile Glu Glu Leu Tyr Asn Lys Leu Leu
            20                  25                  30


<210>  183
<211>  108
<212>  PRT

<213>  Artificial Sequence

<220>
<223>  IntC GS020_ter-7

<400>  183

Met Lys Thr Asn Thr Lys Tyr Glu Ile Leu Gly Pro Glu Gly Phe Val
1               5                   10                  15

Asp Phe Lys Gly Ile Gln Lys Leu Lys Lys Lys Thr Arg Gln Ile Phe
            20                  25                  30

Phe Glu Cys Gly Leu Thr Leu Arg Ala Ser Tyr Asn His Lys Ile Tyr
        35                  40                  45

Asp Tyr Phe Gly Asp Glu Ile Ile Ile Lys Asp Val Val Ile Gly Ser
    50                  55                  60

Lys Ile Lys Ser His Asn Gly Tyr Leu Ile Val Asn Ser Ile Lys Asp
65                  70                  75                  80

Phe Asp Tyr Glu Ser Asp Val Tyr Asp Val Ile Asp Ser Gly Asp Ser
                85                  90                  95

His Leu Tyr Tyr Thr Asn Asn Ile Val Ser His Asn
            100                 105

<210>  184
<211>  5
<212>  PRT
<213>  Artificial Sequence

<220>
<223>  IntN GS020_ter-7 flanking sequence

<400>  184

Ser Gln Glu Leu Glu
1               5

<210>  185
<211>  5
<212>  PRT
<213>  Artificial Sequence

<220>
<223>  IntC GS020_ter-7 flanking sequence

<400>  185

Cys Asn Phe Leu Gly
1               5

<210>  186
<211>  111
<212>  DNA
<213>  Artificial Sequence

<220>
<223>  DNA  IntN GS020_ter-7

<400>  186
tcgcaggaat tagagtgtgt tgatggttcc tcaattataa ctataaaaaa caaagagaca      60

aatttaatag aaaaaataac aatagaagaa ttatacaata aattgttata g              111


<210>  187
<211>  339
<212>  DNA
<213>  Artificial Sequence

<220>
<223>  DNA  IntC GS020_ter-7

<400>  187
atgaaaacta acacaaaata tgaaatttta ggtcctgaag gattcgtcga tttcaaaggt      60

attcaaaaat taaaaaagaa aactagacaa attttttttg agtgtggact aacattacga      120

gcaagttata accacaagat ttacgattat tttggggatg aaattataat taaagacgta      180

gttattggta gtaaaatcaa atcacataat ggttatttaa ttgttaatag tatcaaggat      240

tttgattatg aaagtgacgt atatgacgtt attgattcag gtgattcaca tttatactac      300

acaaacaaca ttgtttctca taattgtaat tttcttggg                            339


<210>  188
<211>  75
<212>  DNA
<213>  Artificial Sequence

<220>
<223>  IntN WT AceL-TerL-11 original DNA sequence

<400>  188
tgtgtttatg gtgatacaat ggttgaaaca gaagatggta aaataaaaat agaagattta      60

tataaaaggt tggca                                                       75


<210>  189
<211>  315
<212>  DNA
<213>  Artificial Sequence

<220>
<223>  IntC WT AceL-TerL-11 original DNA sequence

<400>  189
atgtttagaa ctaatacaaa taatataaaa atattaagtc caaatggatt ttctaatttt      60

aatggtattc aaaaggttga aagaaacctt tatcaacaca ttatctttga tgatgatact      120

gaaataaaaa cttccattaa tcatcctttt ggtaaagata aaatattagc aagagatgta 180

aaagtaggag attatttaaa tagtaaaaag gtattatata atgagttggt taatgaaaat 240

atatttttat atgatcctat aaatgtagaa aaagaaagtt tatatattac taatggtgtt 300

gtttctcata attgt 315

<210> 190
<211> 75
<212> DNA
<213> Artificial Sequence

<220>
<223> IntN WT AceL-TerL-11 codon optimised DNA sequence

<400> 190
tgcgtgtatg gcgatactat ggtggaaacc gaagatggca aaattaaaat tgaagatctg 60

tataaacgtc tggcc 75

<210> 191
<211> 315
<212> DNA
<213> Artificial Sequence

<220>
<223> IntC WT AceL-TerL-11 codon optimised DNA sequence

<400> 191
ggcatgtttc gtaccaacac caacaacatt aaaattctga gcccgaacgg ctttagcaac 60

tttaacggca ttcagaaagt ggaacgtaac ctgtatcagc atattatttt tgatgatgat 120

accgaaatta aaaccagcat taaccatccg tttggcaaag ataaaattct ggcgcgtgat 180

gtgaaagtgg gcgattatct gaacagcaaa aaagtgctgt ataacgaact ggtgaacgaa 240

aacatttttc tgtatgatcc gattaacgtg gaaaaagaaa gcctgtatat taccaacggc 300

gtggtgagcc ataac 315

<210> 192
<211> 111
<212> DNA
<213> Artificial Sequence

<220>
<223> DNA IntN GS033_TerA-6 codon optimised DNA sequence

<400> 192
agcattagcc aggaatccta tatcaacatt gaggtgaacg ggaaagtgga aaccatcaaa 60

atcggcgacc tgtataaaaa actgtccttc aacgagcgta aattcaacga g 111

<210> 193
<211> 345
<212> DNA
<213> Artificial Sequence

<220>
<223>  DNA  IntC  GS033_TerA-6 codon optimised DNA  sequence

<400>  193
atgaaactgc cggagagcgt ggtgaaaaac aacatcaacc tgaaaatcga aaccccgtat        60

ggctttgaga acttctatgg tgtgaacaaa atcaaaaaag acaaatatat ccacctggag       120

tttaccaacg gcgaaaaact gaaatgctcc ctggatcatc ctctgtctac cattgacggc       180

atcgttaaag cgaaagatct ggacaaatat accgaggtct atacgaaatt tggtggctgc       240

tttctgaaaa aatccaaagt gatcaacgag tccatcgagc tgtatgatat cgtgaactct       300

gggctgaaac acctgtatta ttccaacaat attatcagtc acaac                       345


<210>  194
<211>  104
<212>  PRT
<213>  Artificial Sequence

<220>
<223>  IntC Wt AceL-TerL3

<400>  194

Met Phe Ile Thr Asn Thr Asp Asn Ile Lys Ile Leu Ser Pro Ser Gly
1               5                   10                  15


Phe Ser Asn Phe Asn Gly Ile Gln Lys Val Glu Arg Asn Leu Tyr Gln
            20                  25                  30


His Ile Ile Phe Asp Asp Glu Ser Glu Ile Lys Thr Ser Ile Asn His
        35                  40                  45


Pro Phe Gly Lys Asn Lys Ile Leu Ala Arg Asn Val Lys Val Gly Asp
    50                  55                  60


Tyr Leu Ser Ser Lys Lys Val Leu Tyr Asn Glu Leu Val Asn Glu Lys
65                  70                  75                  80


Ile Phe Leu Tyr Asp Pro Ile Asn Val Glu Lys Glu Asn Leu Tyr Ile
                85                  90                  95


Thr Asn Gly Val Val Ser His Asn
            100


<210>  195
<211>  104
<212>  PRT
<213>  Artificial Sequence

<220>
<223>  IntC WTAceL Terl4

<400>  195

Met Phe Arg Thr Asn Thr Asp Asn Ile Lys Ile Leu Ser Pro Ser Gly
1               5                   10                  15

Phe Ser Ile Phe Asn Gly Ile Gln Lys Val Glu Arg Asp Leu Tyr Gln
            20                  25                  30

His Ile Ile Phe Asp Asp Lys Ser Glu Ile Lys Thr Ser Ile Asn His
        35                  40                  45

Pro Phe Gly Lys Asp Lys Ile Leu Ala Arg Asn Ile Lys Val Gly Asp
    50                  55                  60

Tyr Leu Asn Ser Lys Lys Val Leu Tyr Asn Glu Leu Val Ala Glu Lys
65                  70                  75                  80

Ile Thr Leu Tyr Asp Pro Ile Asn Val Glu Lys Glu Asn Leu Tyr Ile
                85                  90                  95

Thr Asn Gly Val Ile Ser His Asn
            100


<210>  196
<211>  104
<212>  PRT
<213>  Artificial Sequence

<220>
<223>  IntC WT AceL-TerL5

<400>  196

Met Phe Arg Thr Asn Thr Asp Asn Ile Lys Ile Leu Ser Pro Ser Gly
1               5                   10                  15

Phe Ser Asn Phe Asn Gly Ile Gln Lys Val Glu Arg Asp Leu Tyr Gln
            20                  25                  30

His Ile Ile Phe Asp Asp Lys Ser Glu Ile Lys Thr Ser Ile Asn His
        35                  40                  45

Pro Phe Gly Lys Asp Lys Ile Leu Ala Arg Asn Ile Lys Val Gly Asp
    50                  55                  60

Tyr Leu Asn Ser Lys Lys Val Leu Tyr Asn Glu Leu Val Asn Glu Lys
65                  70                  75                  80

Ile Thr Leu Tyr Asp Pro Ile Asn Val Glu Lys Glu Asn Leu Tyr Ile
                85                  90                  95

Thr Asn Gly Val Ile Ser His Asn
            100

## Description

### BACKGROUND OF THE INVENTION

Inteins are internal protein sequences that excise themselves out of a precursor protein in an autocatalytic reaction called protein splicing. In protein *trans*-splicing the intein domain is split and located on two separate polypeptides. Protein *trans*-splicing catalysed by split inteins is a powerful technique to assemble a polypeptide backbone from two separate parts. During the reaction the N-and C-terminal intein fragments (also termed Int^{N} and Int^{C}) first associate and fold into the active intein domain and then link the flanking sequences, also termed the N- and C-terminal exteins (Ex^{N} and Ex^{C}), with a peptide bond while at the same time precisely excising the intein sequence. Apart of their homologous N- and C-terminal exteins, inteins will generally also excise themselves out of heterologous sequence flanks. Moreover an intein is a self-contained entity, that is, it does not require any additional cofactors or energy sources to perform the protein splicing reaction.

Thus, the split intein based *trans*-splicing reaction has found various applications in basic protein research and biotechnology, e.g. for segmental isotope labeling of proteins, preparation of cyclic polypeptides, transgene expression, as well as more recently for chemical modification of proteins and protein semi-synthesis (R. Borra, J. A. Camarero, Biopolymers 2013; T. C. Evans, Jr., M. Q. Xu, S. Pradhan, Annu. Rev. Plant Biol. 2005, 56, 375; C. J. Noren, J. Wang, F. B. Perler, Angew. Chem. 2000, 112, 458; Angew. Chem. Int. Ed. Engl. 2000, 39, 450; M. Vila-Perello, T. W. Muir, Cell 2010, 143, 191-200; G. Volkmann, H. Iwai, Mol. Biosyst. 2010, 6, 2110; G. Volkmann, H. D. Mootz, Cell. Mol. Life. Sci. 2013, 70, 118) .

However, split inteins are rare, and especially for chemical modification of proteins and protein semi-synthesis special properties are required. Specifically, one of the intein fragments should be as short as possible to facilitate its efficient and inexpensive assembly by solid-phase peptide synthesis. All naturally occurring split inteins reported so far show the break-point at the position of the homing endonuclease domain in the related contiguous maxi-inteins. This split site gives rise to an Int^{N} of about 100 amino acids (aa) and an Int^{C} of about 35 aa ( I. Giriat, T. W. Muir, J. Am. Chem. Soc. 2003, 125, 7180-7181; H. Wu, Z. Hu, X. Q. Liu, Proc. Natl. Acad. Sci. USA 1998, 95, 9226). Split inteins with shorter Int^{N} or Int^{C} fragments have been created artificially from naturally contiguous inteins (J. H. Appleby, K. Zhou, G. Volkmann, X. Q. Liu, J. Biol. Chem. 2009, 284, 6194; A. S. Aranko, S. Zuger, E. Buchinger, H. Iwai, PloS One 2009, 4, e5185; Y. T. Lee, T. H. Su, W. C. Lo, P. C. Lyu, S. C. Sue, PloS One 2012, 7, e43820; C. Ludwig, M. Pfeiff, U. Linne, H. D. Mootz, Angew. Chem. 2006, 118, 5343; Angew. Chem. Int. Ed. Engl. 2006, 45, 5218; W. Sun, J. Yang, X. Q. Liu, J. Biol. Chem. 2004, 279, 35281; G. Volkmann, X. Q. Liu, PloS One 2009, 4, e8381), but these generally show lower splicing yields and rates and tend to associate and fold less efficiently. Moreover, another drawback of known split inteins is a limited compatibility with diverse target proteins due to the solubility and expression issues of the recombinant split intein fusion constructs.

Hence, there is a need for alternative split inteins that overcome the known problems.

### SUMMARY OF THE INVENTION

The present invention is based on the unexpected finding that isolated polypeptides comprising one intein or at least one intein fragment of said intein, wherein the N-terminal intein fragment of said intein is split after 14-60 amino acids from the intein's N-terminal end can be provided, which are naturally split inteins. Thus, these inteins provide the shortest naturally occurring N-terminal intein fragments discovered so far. Moreover, they exhibit excellent splicing yields and rates.

Thus, in other words herein is provide an isolated polypeptide comprising at least one intein or at least one intein fragment of said intein, wherein said intein is a naturally split intein with a N-terminal intein fragment split after 14-60 amino acids from the intein' s N-terminal position.

Due to the very short N-terminal these inteins, or their Int^{N} fragments, respectively, can be easily generated via solid peptide synthesis, which is faster, more reliable and robust than protein generation via recombinant protein expression techniques.

Hence, these novel split inteins are ideally suited for all kinds of efficient protein modifications.

Moreover, it was surprisingly possible to further modify some of these natural split inteins to even increase splicing yields and rates and thus render the novel split inteins suited for an even wider range of applications and assay conditions.

In a further aspect, the present invention relates to an isolated polypeptide as described above wherein said at least one intein fragment, is selected from the group comprising:
a) a N-terminal intein fragment having 100% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, or,
b) a N-terminal intein fragment having at least 70%, or 80% or 85% or 90% or 95% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, and/or
c) a C-terminal intein fragment having 100% sequence identity with SEQ ID NO: 6, 27, 39, 45, 51, 57, 63, 69, 75, 81, 87, 93, 99, 105, 111, 117, 123, 129, 135, 141, 147, 153, 159, 165, 171, 177, 183, 194, 195 or 196 or,
d) a C-terminal intein fragment having at least 70%, or 80% or 85% or 90% or 95% sequence identity with SEQ ID NO: 6, 27, 39, 45, 51, 57, 63, 69, 75, 81, 87, 93, 99, 105, 111, 117, 123, 129, 135, 141, 147, 153, 159, 165, 171, 177, 183, 194, 195 or 196, or
e) wherein the at least one intein has at least 70%, or 80% or 85% or 90% or 95% sequence identity with SEQ ID NO:1 or 26.

In a further aspect, the present invention relates to an isolated polypeptide as described above, wherein said polypeptide comprises at least one N-terminal intein fragment and at least one C-terminal intein fragment, wherein
1) if said at least one N-terminal intein fragment is selected from SEQ ID NO: 5 then said at least one C-terminal intein fragment is selected from SEQ ID NO: 6, or
2) if said at least one N-terminal intein fragment is selected from SEQ ID NO: 5, 28-33 then said at least one C-terminal intein fragment is selected from SEQ ID NO: 27, or
3) if said at least one N-terminal intein fragment is selected from SEQ ID NO: 38 then said at least one C-terminal intein fragment is selected from SEQ ID NO: 39, or
4) if said at least one N-terminal intein fragment is selected from SEQ ID NO: 44 then said at least one C-terminal intein fragment is selected from SEQ ID NO: 45, or
5) if said at least one N-terminal intein fragment is selected from SEQ ID NO: 50 then said at least one C-terminal intein fragment is selected from SEQ ID NO: 51, or
6) if said at least one N-terminal intein fragment is selected from SEQ ID NO: 56 then said at least one C-terminal intein fragment is selected from SEQ ID NO: 57, or
7) if said at least one N-terminal intein fragment is selected from SEQ ID NO: 62 then said at least one C-terminal intein fragment is selected from SEQ ID NO: 63, or
8) if said at least one N-terminal intein fragment is selected from SEQ ID NO: 68 then said at least one C-terminal intein fragment is selected from SEQ ID NO: 69, or
9) if said at least one N-terminal intein fragment is selected from SEQ ID NO: 74 then said at least one C-terminal intein fragment is selected from SEQ ID NO: 75, or
10) if said at least one N-terminal intein fragment is selected from SEQ ID NO: 80 then said at least one C-terminal intein fragment is selected from SEQ ID NO: 81, or
11) if said at least one N-terminal intein fragment is selected from SEQ ID NO: 86 then said at least one C-terminal intein fragment is selected from SEQ ID NO: 87, or
12) if said at least one N-terminal intein fragment is selected from SEQ ID NO: 92 then said at least one C-terminal intein fragment is selected from SEQ ID NO: 93, or
13) if said at least one N-terminal intein fragment is selected from SEQ ID NO: 98 then said at least one C-terminal intein fragment is selected from SEQ ID NO: 99, or
14) if said at least one N-terminal intein fragment is selected from SEQ ID NO: 104 then said at least one C-terminal intein fragment is selected from SEQ ID NO: 105, or
15) if said at least one N-terminal intein fragment is selected from SEQ ID NO: 110 then said at least one C-terminal intein fragment is selected from SEQ ID NO: 111, or
16) if said at least one N-terminal intein fragment is selected from SEQ ID NO: 116 then said at least one C-terminal intein fragment is selected from SEQ ID NO: 117, or
17) if said at least one N-terminal intein fragment is selected from SEQ ID NO: 122 then said at least one C-terminal intein fragment is selected from SEQ ID NO: 123, or
18) if said at least one N-terminal intein fragment is selected from SEQ ID NO: 128 then said at least one C-terminal intein fragment is selected from SEQ ID NO: 129, or
19) if said at least one N-terminal intein fragment is selected from SEQ ID NO: 134 then said at least one C-terminal intein fragment is selected from SEQ ID NO: 135, or
20) if said at least one N-terminal intein fragment is selected from SEQ ID NO: 140 then said at least one C-terminal intein fragment is selected from SEQ ID NO: 141, or
21) if said at least one N-terminal intein fragment is selected from SEQ ID NO: 146 then said at least one C-terminal intein fragment is selected from SEQ ID NO: 147, or
22) if said at least one N-terminal intein fragment is selected from SEQ ID NO: 152 then said at least one C-terminal intein fragment is selected from SEQ ID NO: 153, or
23) if said at least one N-terminal intein fragment is selected from SEQ ID NO: 158 then said at least one C-terminal intein fragment is selected from SEQ ID NO: 159, or
24) if said at least one N-terminal intein fragment is selected from SEQ ID NO: 164 then said at least one C-terminal intein fragment is selected from SEQ ID NO: 165, or
25) if said at least one N-terminal intein fragment is selected from SEQ ID NO: 170 then said at least one C-terminal intein fragment is selected from SEQ ID NO: 171, or
26) if said at least one N-terminal intein fragment is selected from SEQ ID NO: 176 then said at least one C-terminal intein fragment is selected from SEQ ID NO: 177, or
27) if said at least one N-terminal intein fragment is selected from SEQ ID NO: 182 then said at least one C-terminal intein fragment is selected from SEQ ID NO: 183
28) if said at least one N-terminal intein fragment is selected from SEQ ID NO: 2 then said at least one C-terminal intein fragment is selected from SEQ ID NO: 194, or
29) if said at least one N-terminal intein fragment is selected from SEQ ID NO: 3 then said at least one C-terminal intein fragment is selected from SEQ ID NO: 195, or
30) if said at least one N-terminal intein fragment is selected from SEQ ID NO: 4 then said at least one C-terminal intein fragment is selected from SEQ ID NO: 196.

In a further aspect, the present invention relates to an isolated polypeptide as described above wherein said polypeptide at the N-terminal end and/or at the C-terminal end of said at least one intein fragment further comprises a sequence selected from:
1) in the case of SEQ ID NO: 5 and/or 6 SEQ ID NO: 25 and/or 26, respectively,
2) in the case of SEQ ID NO: 5, 28-33 and/or 27 SEQ ID NO: 34 and/or 35, respectively,
3) in the case of SEQ ID NO:38 and/or 39 SEQ ID NO: 40 and/or 41, respectively,
4) in the case of SEQ ID NO:44 and/or 45 SEQ ID NO: 46 and/or 47, respectively,
5) in the case of SEQ ID NO:50 and/or 51 SEQ ID NO: 52 and/or 53, respectively,
6) in the case of SEQ ID NO:56 and/or 57 SEQ ID NO: 58 and/or 59, respectively,
7) in the case of SEQ ID NO:62 and/or 63 SEQ ID NO: 64 and/or 65, respectively,
8) in the case of SEQ ID NO:68 and/or 69 SEQ ID NO: 70 and/or 71, respectively,
9) in the case of SEQ ID NO:74 and/or 75 SEQ ID NO: 76 and/or 77, respectively,
10) in the case of SEQ ID NO:80 and/or 81 SEQ ID NO: 82 and/or 83, respectively,
11) in the case of SEQ ID NO:86 and/or 87 SEQ ID NO: 88 and/or 89, respectively,
12) in the case of SEQ ID NO:92 and/or 93 SEQ ID NO: 94 and/or 95, respectively,
13) in the case of SEQ ID NO:98 and/or 99 SEQ ID NO: 100 and/or 101, respectively,
14) in the case of SEQ ID NO:1 04 and/or 105 SEQ ID NO: 106 and/or 107, respectively,
15) in the case of SEQ ID NO:110 and/or 111 SEQ ID NO: 112 and/or 113, respectively,
16) in the case of SEQ ID NO:116 and/or 117 SEQ ID NO: 118 and/or 119, respectively,
17) in the case of SEQ ID NO:122 and/or 123 SEQ ID NO: 124 and/or 125, respectively,
18) in the case of SEQ ID NO:128 and/or 129 SEQ ID NO: 130 and/or 131, respectively,
19) in the case of SEQ ID NO:134 and/or 135 SEQ ID NO: 136 and/or 137, respectively,
20) in the case of SEQ ID NO:140 and/or 141 SEQ ID NO: 142 and/or 143, respectively,
21) in the case of SEQ ID NO:146 and/or 147 SEQ ID NO: 148 and/or 149, respectively,
22) in the case of SEQ ID NO:152 and/or 153 SEQ ID NO: 154 and/or 155, respectively,
23) in the case of SEQ ID NO:158 and/or 159 SEQ ID NO: 160 and/or 161, respectively,
24) in the case of SEQ ID NO:164 and/or 165 SEQ ID NO: 166 and/or 167, respectively,
25) in the case of SEQ ID NO:170 and/or 171 SEQ ID NO: 172 and/or 173, respectively,
26) in the case of SEQ ID NO:176 and/or 177 SEQ ID NO: 178 and/or 179, respectively,
27) in the case of SEQ ID NO:182 and/or 183 SEQ ID NO: 184 and/or 185, respectively.

In a further aspect, the present invention relates to an isolated polypeptide as described above, wherein the N-terminal intein fragment has 100% sequence identity with a sequence selected from sequences comprising SEQ ID NO: 5, 11 or 12 or has at least 90% or 95% sequence identity with a sequence selected from sequences comprising SEQ ID NO: 5, 11 or 12 and/or,
wherein the C-terminal intein fragment has 100% sequence identity with a sequence selected from sequences comprising SEQ ID NO: 6 or 13-18 or has at least 90% or 95% sequence identity with a sequence selected from sequences comprising SEQ ID NO: 6 or 13-18.

In a further aspect, the present invention relates to an isolated polypeptide as described above, wherein the N-terminal intein fragment is selected from sequences comprising SEQ ID NO: 5, 11 or 12 and/or,
wherein the C-terminal intein fragment is selected from sequences comprising SEQ ID NO: 6 or 13-18.

In a further aspect, the present invention relates to an isolated polypeptide as described above, wherein the polypeptide comprises the N-terminal intein fragment with SEQ ID NO: 5 or 12 and the C-terminal intein fragment with SEQ ID NO:13.

In a further aspect, the present invention relates to an isolated polypeptide as described above, wherein the N-terminal intein fragment has 100% sequence identity with a sequence selected from sequences comprising SEQ ID NOs: 5, 28-33 or has at least 90% or 95% sequence identity with a sequence selected from sequences comprising SEQ ID NO:s: 28-33 and/or, wherein the C-terminal intein fragment has 100% sequence identity with SEQ ID NO: 27 or has at least 90% or 95% sequence identity with with SEQ ID NO: 27.

In a further aspect, the present invention relates to an isolated polypeptide as described above, wherein the polypeptide further comprises at least one C-terminal extein and/or at least one N-terminal extein sequence.

In a further aspect, the present invention relates to two isolated polypeptides as described above, wherein one of the isolated polypeptides comprises least one heterologous N-terminal extein fused to a N-terminal intein fragment having at least 70%, or 80% or 85% or 90% or 95% or 99% sequence identity with SEQ ID NO:5 28, 38, 44, 50, 56, 62, 68, 74, 80, 86, 92, 98, 104, 110, 116, 122, 128, 134, 140, 146, 152, 158, 164, 170, 176 or 182 and,
wherein the second one of the isolated polypeptides comprises at least one C-terminal extein sequence fused to a C-terminal intein fragment having at least 70%, or 80% or 85% or 90% or 95% or 99% sequence identity with SEQ ID NO:6 27, 39, 45, 51, 57, 63, 69, 75, 81, 87, 93, 99, 105, 111, 117, 123, 129, 135, 141, 147, 153, 159, 165, 171 , 177 or 183.

In a further aspect, the present invention relates to an isolated polypeptide as described above, wherein the polypeptide further comprises at least one component selected from a solubility factor, a marker, a linker, an epitope, an affinity tag, a fluorophore or a fluorescent protein, a toxic compound or protein and a small-molecule or a small-molecule binding protein.

In a further aspect, the present invention relates to an isolated nucleic acid molecule comprising a nucleotide sequence encoding for a polypeptide of claims 1 to 11 or a homolog, variant or complement thereof.

In a further aspect, the present invention relates to a method using an isolated polypeptide or a nucleic acid molecule as described above, wherein the method is selected from the group consisting of modification of a protein, protein lipidation, protein immobilization, protein backbone semi-synthesis, regioselective protein side chain modification and artificial control of protein splicing by light.

In a further aspect, the present invention relates to the use of a polypeptide or a nucleic acid molecule according as described above, wherein the use is selected from the group consisting of modification of a protein, protein lipidation, protein immobilization, protein backbone semisynthesis and use as molecular switch.

In a further aspect, the present invention relates to a kit comprising at least one polypeptide or a nucleic acid molecule as described above.

### BRIEF DESCRIPTION OF THE DRAWINGS

The invention will be better understood with reference to the detailed description when considered in conjunction with the non-limiting examples and the accompanying drawings.

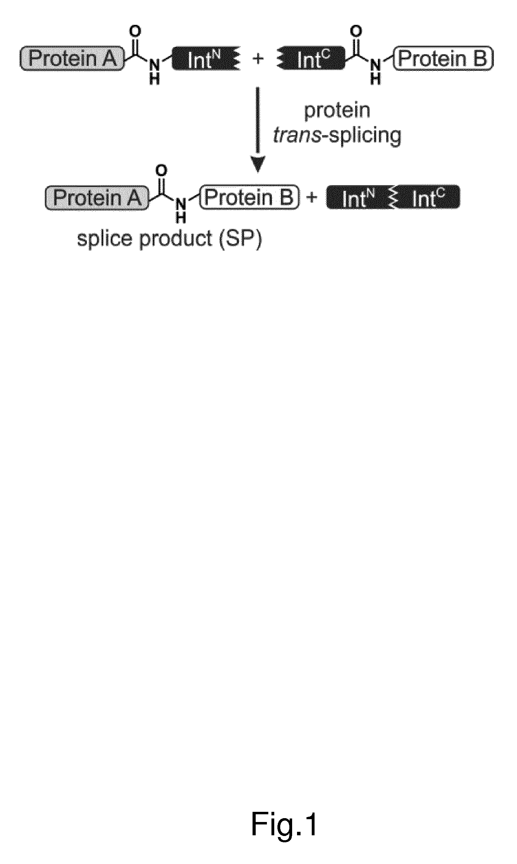

In **Figure 1** the protein trans-splicing mediated by inteins is schematically depicted. In detail, the Int^{N} and Int^{C} fragments ligate their flanking sequences with a native peptide bond. These can either be their native exteins or unrelated peptides or proteins.

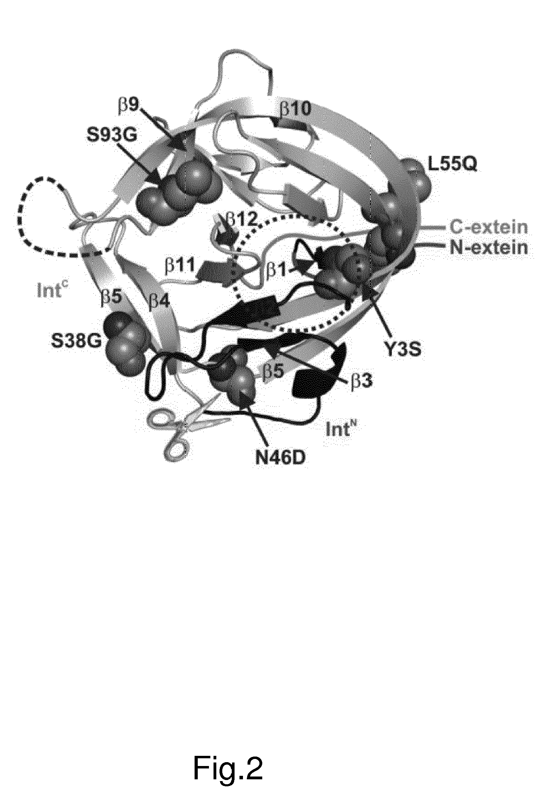

**Figure 2** shows a model of the of AceL-TerL (SEQ ID NO:1) sequence. In detail, the probable location of the split site is indicated by scissors and selected mutations are shown. Moreover, the active site is indicated by the dotted circle and the unstructured loop representing the position of the removed homing endonuclease domain is represented by the dashed line. It can be seen that the AceL-TerL inteins have a novel split site corresponding to a probable surface loop region of the intein with no defined secondary structure following β-strand 3 and α-helix 1.

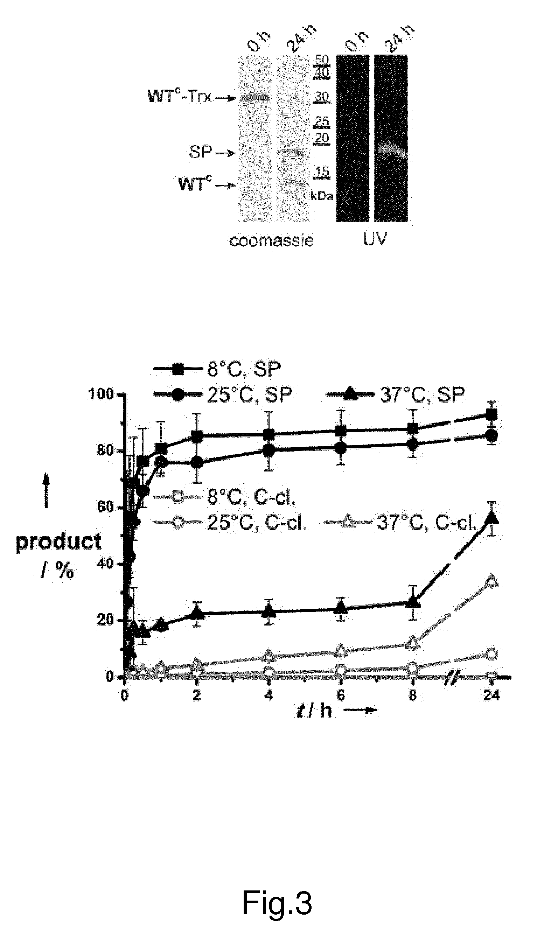

**Figure 3** shows results of the characterization of the AceL-TerL intein (SEQ ID NO:1). Top: WT^{C} (SEQ ID NO:6)-Trx-His6 (15 µM) was incubated with pepWT^{N} (SEQ ID NO:5, 45 µM) at 25°C for 24 h and the reaction mixture was analyzed by SDS-PAGE using UV illumination or Coomassie Brilliant Blue staining. Calculated molecular masses are: WT^{C}-Trx = 26.4 kDa; SP = 15.2 kDa; Int^{N} = 2.9 kDa; Int^{C} = 12.2 kDa; C-terminal cleavage product (Trx) = 14.1 kDa. Bottom: Time-courses of SP and C-terminal cleavage product (C-cl.) formation at the indicated temperatures.

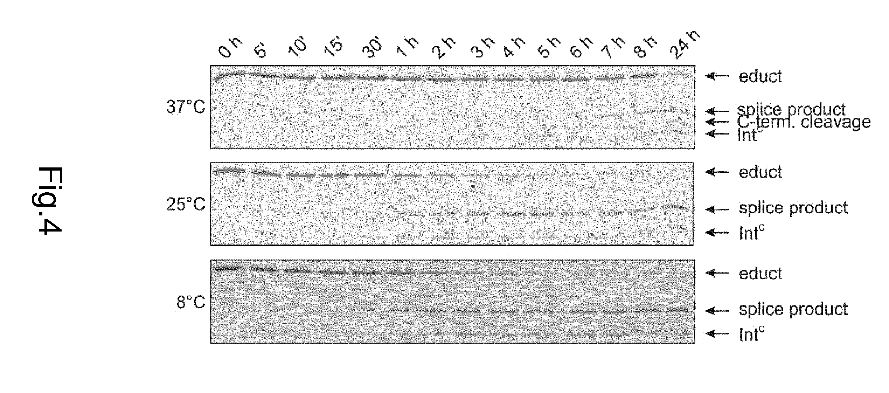

**Figure 4** demonstrates the temperature dependence of the AceL-TerL intein (SEQ ID NO:1). In detail, the intein construct WT^{C}-Trx-His6 (15 µM; = educt) was incubated with pepWT^{N} (45 µM) at 37°C, 25°C, and 8°C. Aliquots were removed at indicated time points and analysed by SDS-PAGE using Coomassie Brilliant Blue staining. Positions of expected protein products are indicated. The Int^{C} fragment can appear as a double band due to succinimide hydrolysis over time.

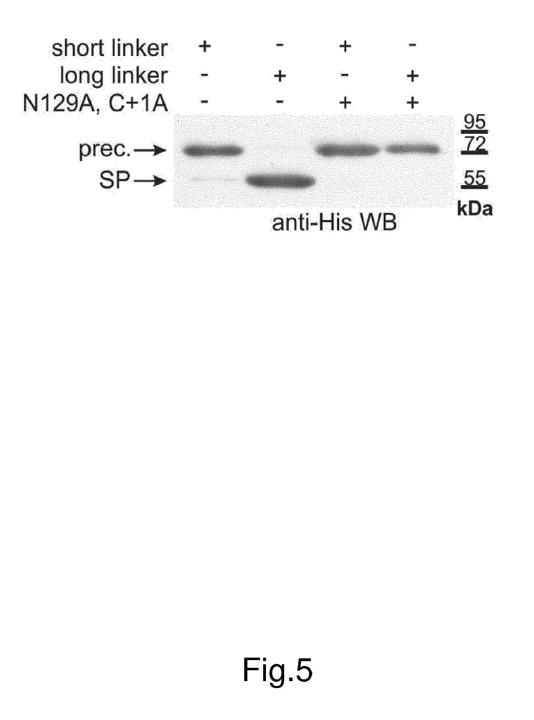

**Figure 5** shows cis-splicing of the AceL-TerL intein (SEQ ID NO:1). In detail, the Int^{N} and Int^{C} fragments of the AceL-TerL intein were artificially fused and inserted into a recombinant construct with MBP and FKBP as extein sequences (MBP-AceL-TerLcis-FKBP-His6). Two different linkers between the intein fragments were evaluated, a short linker of only two residues (MG) and a long linker of seven amino acids (MGSGGSG) (SEQ ID NO:7 and 8, respectively). Control constructs with mutations of two catalytically essential amino acid residues at the C-terminal splice junction (N129A, C+1A, SEQ ID NO:9 and 10, respectively) were also prepared for control experiments. All constructs were expressed in *E. coli* for 3 h at 28°C following induction with IPTG. Samples were removed after 3 h and total cell lysates were used for Western Blot analysis. Calculated molecular masses are 73.5 kDa for the precursor protein (MBP-AceL-TerLcis-FKBP-His6) and 58.4 kDa for the splice product (MBP-FKBP-His6).

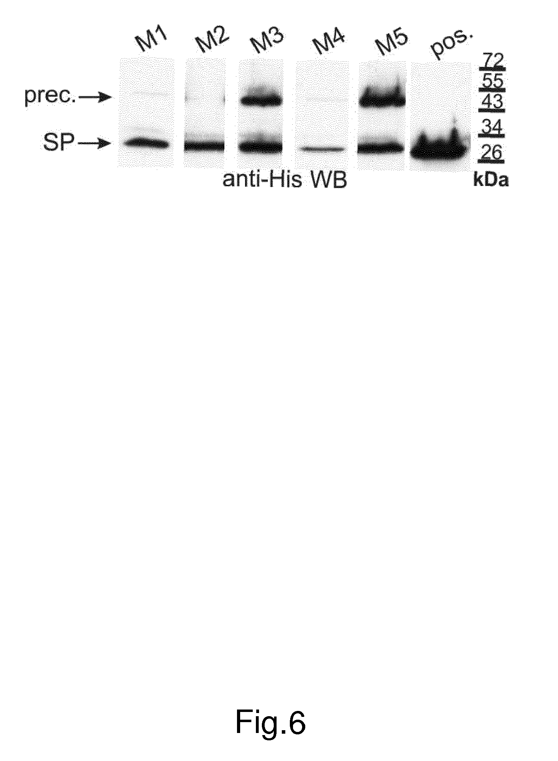

**Figure 6** shows cis-splicing of the selected AceL-TerL mutants in the KanR protein. Selected colonies that conferred kanamycin resistance in the selection scheme were cultivated in liquid medium supplemented with 50 µg/mL kanamycin and 100 µg/mL ampicillin at 37°C, and total cell lysates were used for Western Blot analysis (anti-His). Calculated molecular masses are 47.5 kDa for the precursor protein (His6-KanR (1-114)-AceL-TerL*cis* library-KanR(115-268)) and 32.4 kDa for the splice product (His6-KanR(1-114)- SGEFECEFL-KanR(115-268)). The positive control (pos.) shows the His6-Kanr protein without an intein insertion.

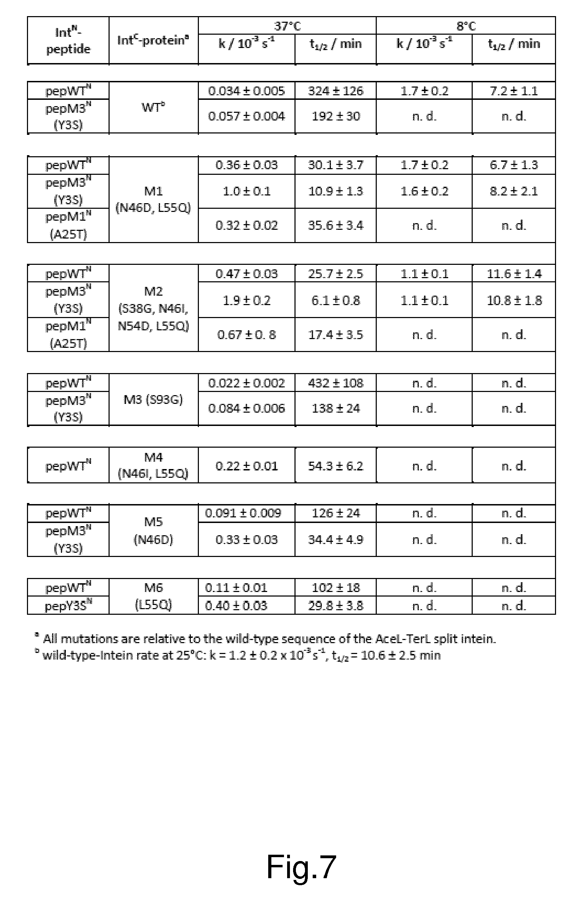

Figure 7 details kinetic parameters of AceL-TerL mutant inteins with the Int^{C}-Trx-H6 constructs.

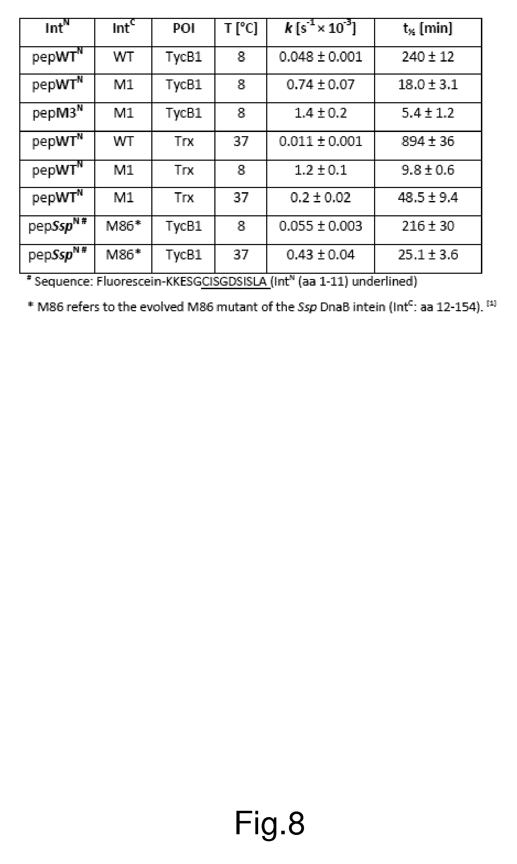

**Figure 8** details kinetic parameters of AceL-TerL mutant inteins with the MBP-Int^{C}-POI-His6 fusion proteins. Also depicted are the results obtained with the highly evolved M86 mutant of the artificially split Ssp DnaB intein, which represents the current benchmark intein for split intein-mediated N-terminal chemical modification of proteins.

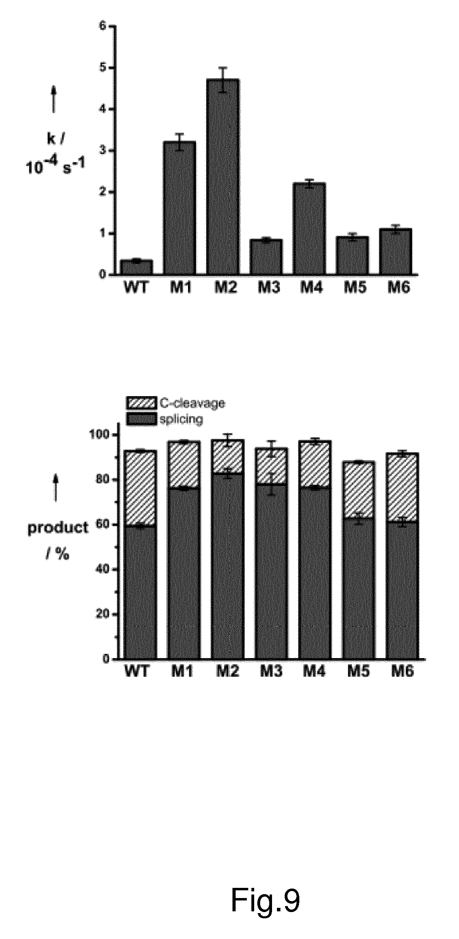

**Figure 9** shows results of the characterization of the improved AceL-TerL intein mutants. In detail, time-courses of splice reactions were monitored at 37°C and rate constants were determined by fitting product formation to pseudo-first order kinetics.
   Top: Rate constants and product yields of the AceL-TerL intein (SEQ ID NO:1)
   Bottom: Rate constants and product yields of the mutants M1 - M6.

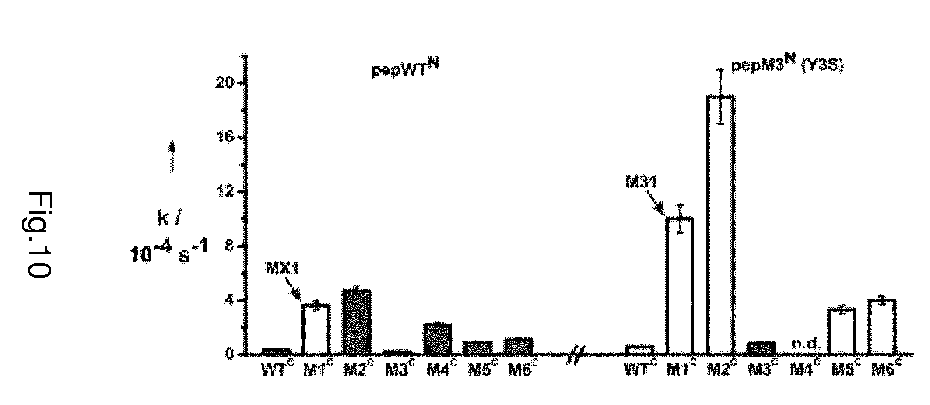

**Figure 10** shows more results of the characterization of the improved AceL-TerL intein mutants. In detail, rate constants of combinations of the indicated Int^{N} and Int^{C} constructs are depicted.

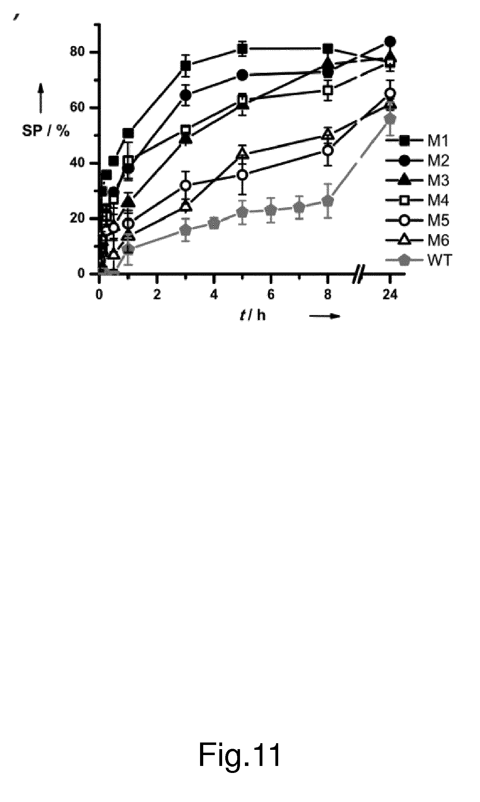

**Figure 11** shows further results of the characterization of the improved AceL-TerL intein mutants. In these experiments the AceL-TerL mutants were prepared as split inteins with the indicated Int^{C} fragment in the fusion constructs Int^{C}-Trx-His6 and incubated with synthetic peptides containing the Int^{N} fragment. Formation of splice and cleavage products was determined from densitometric analyses of Coomassie-stained SDS-PAGE gels. Time-courses of the splice product formation of the six mutants (native combinations of Int^{N} and Int^{C} fragments from wild-type and mutants M1-M6, SEQ ID NO:11-18) at 37°C are shown.

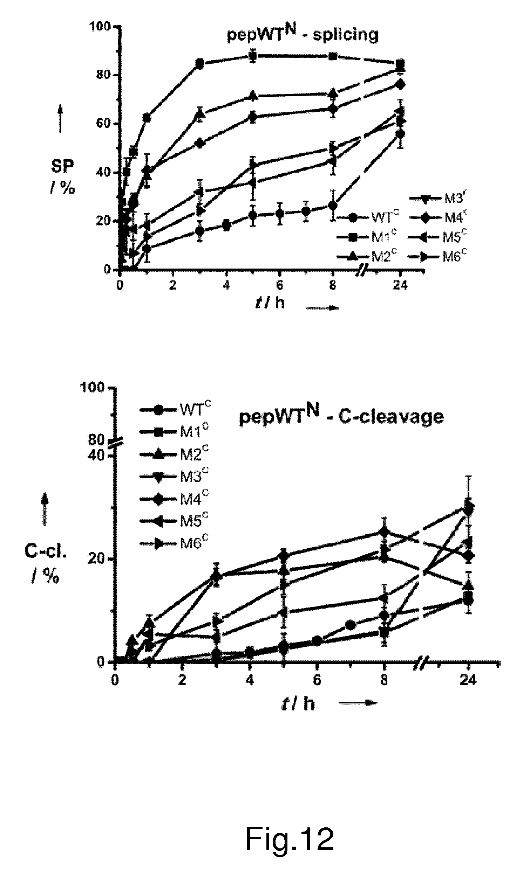

**Figure 12** shows further results of the characterization of the improved AceL-TerL intein mutants. In these experiments the AceL-TerL mutants were prepared as split inteins with the indicated Int^{C} fragment in the fusion constructs Int^{C}-Trx-His6 and incubated with synthetic peptides containing the Int^{N} fragment. Formation of splice and cleavage products was determined from densitometric analyses of Coomassie-stained SDS-PAGE gels. In detail, splice product formation (top) and C-terminal cleavage product formation (bottom) of reactions combining each of the indicated Int^{C} fragments with pepWT^{N} are shown.

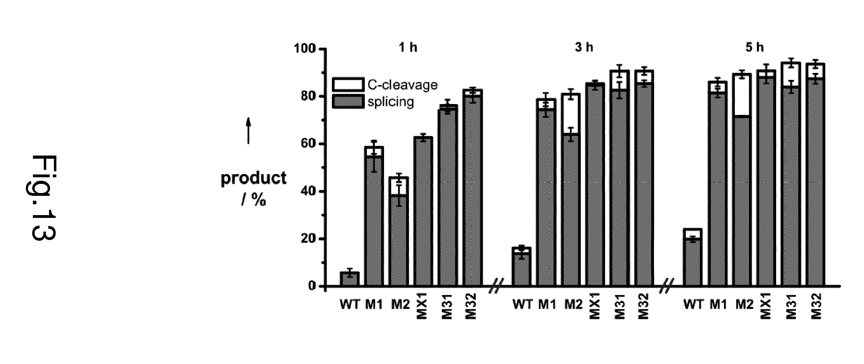

**Figure 13** shows further results of the characterization of the improved AceL-TerL intein mutants. In these experiments the AceL-TerL mutants were prepared as split inteins with the indicated Int^{C} fragment in the fusion constructs Int^{C}-Trx-His6 and incubated with synthetic peptides containing the Int^{N} fragment. Formation of splice and cleavage products was determined from densitometric analyses of Coomassie-stained SDS-PAGE gels. In detail, different splicing and C-terminal cleavage yields are shown. This figure illustrates, for example, that the M1 mutant has a more favourable ratio between splicing and cleavage than the M2 mutant.

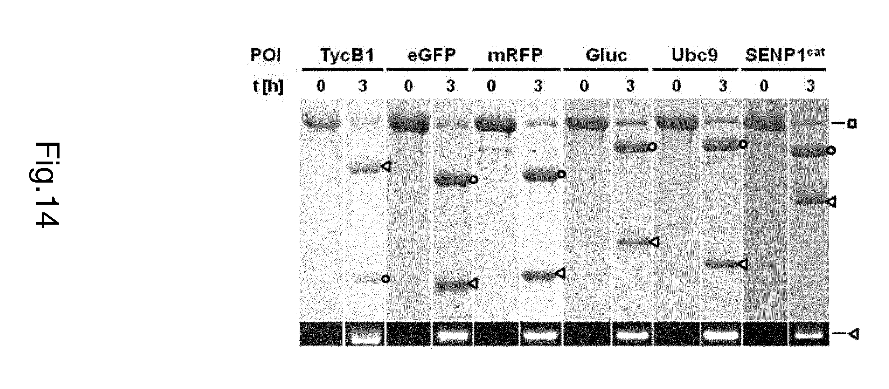

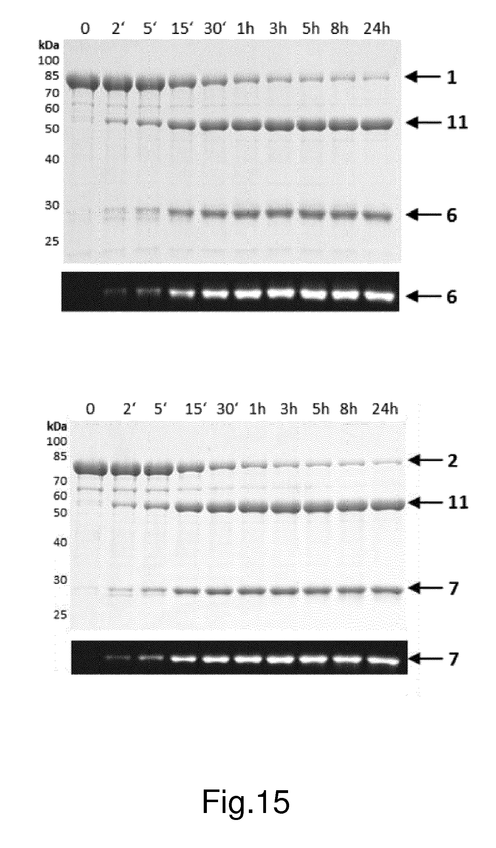

**Figure 14** depicts results demonstrating that, e.g. AceL-TerL MX1 can be generally used for protein labelling. The indicated proteins of interest (POI) were expressed and purified as fusion constructs in the format MBP-M1^{C}-POI-H6 (indicated as squares; MBP = maltose-binding protein) and incubated with pepWT^{N} at 8°C. Reactions were analyzed by SDS-PAGE using UV illumination (bottom) and Coomassie staining (top). The fluorescently labelled splice products are marked by a triangle and the MBP-M1^{C} by-products are marked by circles. Note that for each protein the lanes were normalized to the migration of the precursor protein (Abbreviations: green fluorescent protein = EGFP, red fluorescent protein = mRFP, Gaussia princeps luciferase = Gluc, murine E2 conjugating enzyme = Ubc9, human protease from the SUMO pathway = SENP1). **Figures 15** - **17**
The results presented in Figures 15 - 17 also demonstrate that, e.g. AceL-TerL MX1 can be generally used for protein labelling. The AceL TerL mutant MX1 (consisting of WT-Int^{N} (SEQ ID NO:5) and M1-Int^{C} (SEQ ID NO:13) fragments) was applied in all cases. Proteins of interest (POI) were expressed and purified in the format MBP-M1^{C}-POI-His6. Precursor proteins **1-5** (15 µM) were incubated with 45 µM pepWT^{N} at 8 °C, and samples were removed at the indicated time points for SDS-PAGE analysis.
   (EN = ExteinN sequence KKEFE).
   Splicing with constructs containing the POIs

   Figure 15: eGFP and mRFP

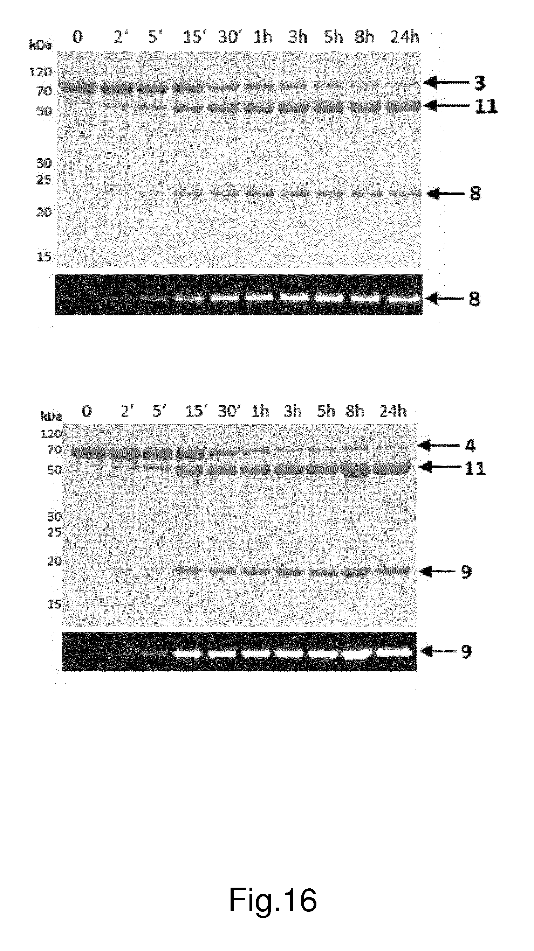

   Figure 16: Gluc and Ubc9

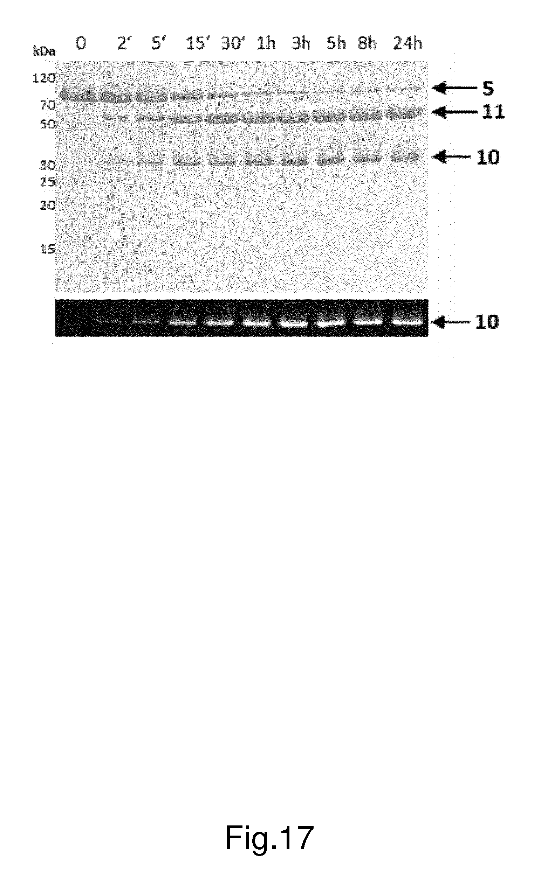

   Figure 17: SENP1

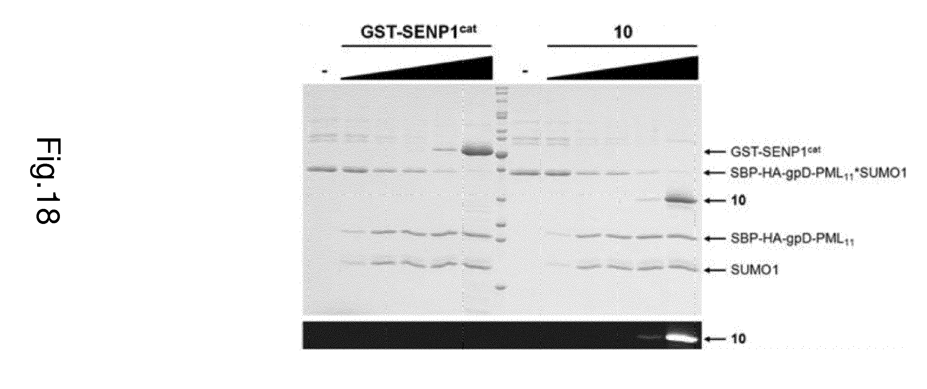

**Figure 18** shows a SENP1 cleavage assay. In detail, the substrate protein SBP-HA-gpD-PML11*SUMO1 (10 µM) was incubated for 10 min at 37 °C with increasing concentrations (1 nM, 10 nM, 100 nM, 1 µM, 10 µM) of GST-SENP1cat (positive control) or N-terminally fluorescein-labeled FI-SENP1cat (protein 10) in 20 mM HEPES, 150 mM NaCl, 1 mM DTT (pH 8). Reactions were quenched by addition of reducing SDS sample buffer, and loaded to a 15% SDS gel. Thus, this result demonstrates that the enzyme SENP1 fluorescently labeled via novel intein AceL-TerL MX1 is fully catalytically active.

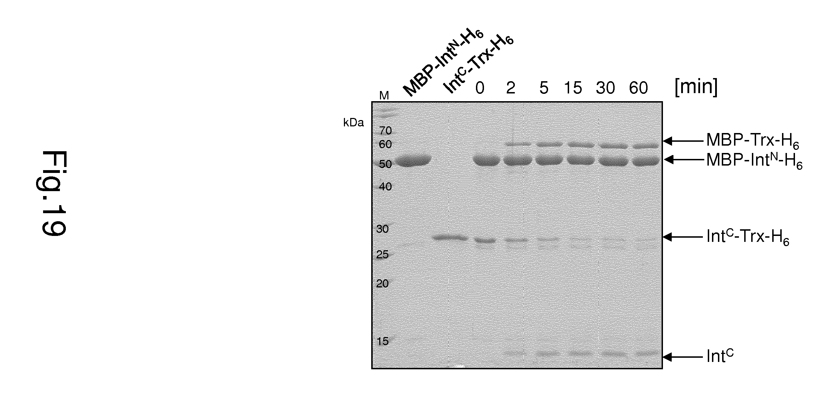

**Figure 19** demonstrates the activity of the GS033_TerA-6 intein (SEQ ID NO: 26). In detail, the intein construct GS033-TerA-6-Int^{C}-Trx-His6 (i.e. comprising the Int^{C} fragment with SEQ ID NO: 27) was incubated with MBP-GS033_TerA-6-Int^{N}-GG-His6 (i.e. the Int^{N} fragment with SEQ ID NO: 28). Aliquots were removed at indicated time points and analysed by SDS-PAGE using Coomassie Brilliant Blue staining. Positions of expected protein products are indicated.

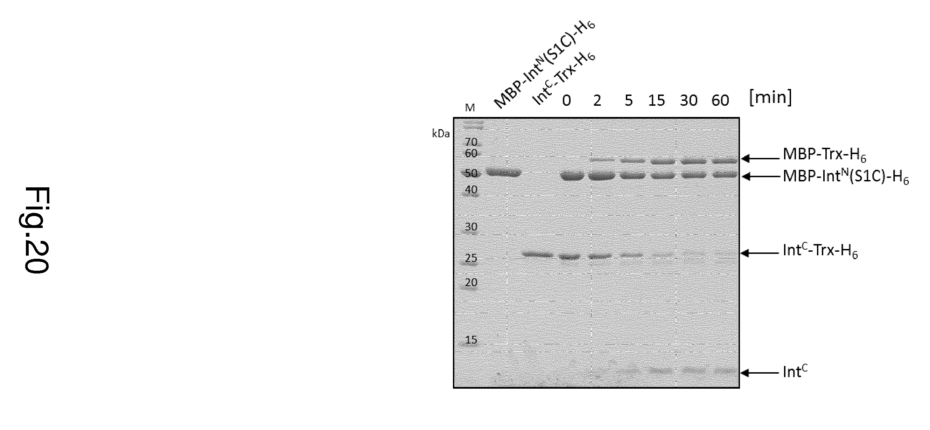

**Figure 20** demonstrates the activity of the GS033_TerA-6 intein with a Ser1Cys mutation in the Int^{N} fragment. In detail, the intein construct GS033_TerA-6-Int^{C}-Trx-HiS₆ (i.e. comprising the Int^{C} fragment with SEQ ID NO: 27) was incubated with MBP-GS033_TerA-6-Int^{N}(S1C)- GG-His6 (i.e., the Int^{N} fragment with SEQ ID NO: 29) . Aliquots were removed at indicated time points and analysed by SDS-PAGE using Coomassie Brilliant Blue staining. Positions of expected protein products are indicated.

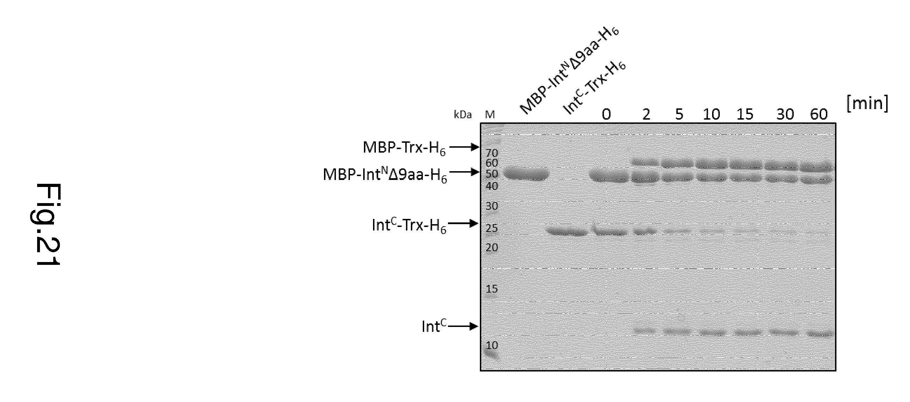

**Figure 21** demonstrates the activity of the GS033_TerA-6 intein with a truncation of 9 amino acids in the Int^{N} fragment. In detail, the intein construct GS033-TerA-6-Int^{C}-Trx-His₆ (i.e. comprising the Int^{C} fragment with SEQ ID NO: 27) was incubated with MBP-GS033_TerA-6-Int^{N}(□9aa)-GG-His6 (i.e. comprising the Int^{N} fragment with SEQ ID NO: 30). Aliquots were removed at indicated time points and analysed by SDS-PAGE using Coomassie Brilliant Blue staining. This experiment was repeated using a synthetic peptide comprising the Int^{N} fragment with SEQ ID NO: 31 in a FI-KKEFE-Int^{N} moiety(data not shown).

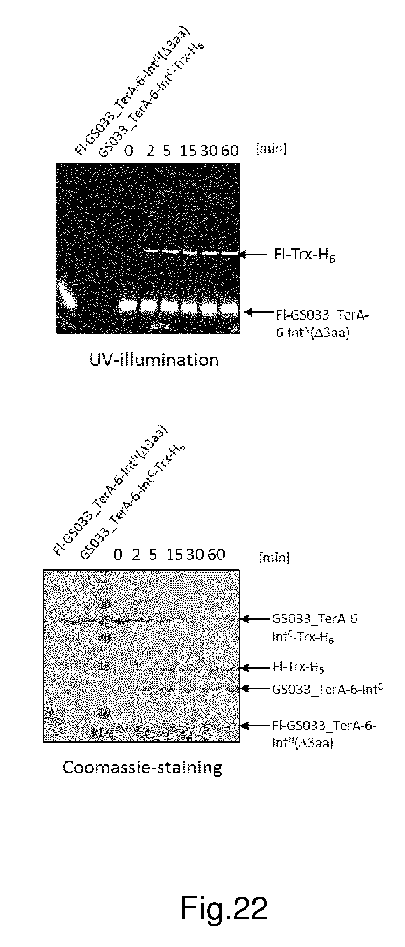

**Figure 22** demonstrates the activity of the GS033_TerA-6 intein with a truncation of 3 amino acids in the Int^{N} fragment and using a synthetic peptide containing the Int^{N} fragment. In detail, the intein construct GS033-TerA-6-Int^{C}-Trx-His6 (i.e. comprising the Int^{C} fragment with SEQ ID NO: 27) was incubated with FI-GS033_TerA-6-Int^{N}(□3aa) His6 (i.e. comprising the Int^{N} fragment with SEQ ID NO: 32 in a FI-KKEFE-Int^{N}(delta3aa)-A moiety). Aliquots were removed at indicated time points and analysed by SDS-PAGE using UV illumination (top) and Coomassie Brilliant Blue staining (bottom). Positions of expected protein products are indicated.

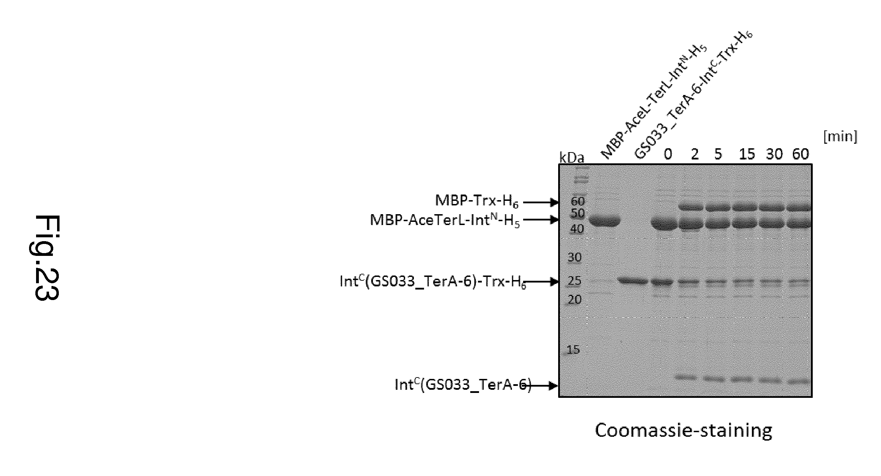

**Figure 23** demonstrates the ability of the Int^{C} fragment of GS033_TerA-6 intein to trans-splice with the Int^{N} fragment of the AceL-TerL intein (cross-splicing) (i.e. the Int^{N} fragment with SEQ ID NO: 5). In detail, the intein construct GS033-TerA-6-Int^{C}-Trx-His6 (i.e. comprising the Int^{C} fragment with SEQ ID NO: 27) was incubated with MBP-AceL-TerL-Int^{N}-MGGY-H₅ (ie. comprising the Int^{N} fragment with SEQ ID NO: 5). Aliquots were removed at indicated time points and analysed by SDS-PAGE using Coomassie Brilliant Blue staining. Positions of expected protein products are indicated.

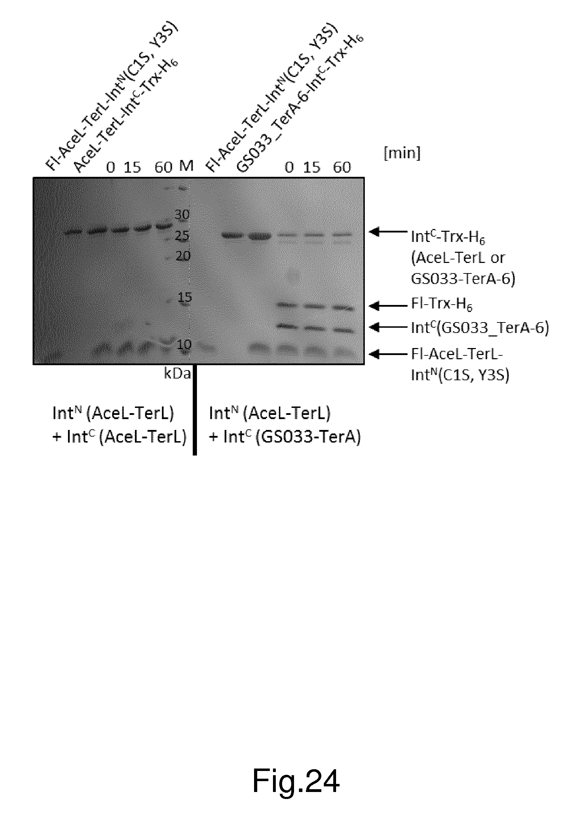

**Figure 24** demonstrates the ability of the Int^{C} fragment of GS033_TerA-6 intein to trans-splice with the Int^{N} fragment of the AceL-TerL intein (cross-splicing) containing an C1S amino acid substitution of the catalytic first amino acid of the intein and a Y3S mutation. In detail, the intein construct GS033_TerA-6-Int^{C}-Trx-His6 (i.e. comprising the Int^{C} fragment with SEQ ID NO: 27) was incubated with FI-KKEFE-AceL-TerL-Int^{N}(C1S, Y3S) (i.e. comprising the Int^{N} fragment with SEQ ID NO: 33) a construct having altered flanking residues when compared to the wild type seqeunce. Aliquots were removed at indicated time points and analysed by SDS-PAGE using UV illumination (data not shown) and Coomassie Brilliant Blue staining. Positions of expected protein products are indicated.

### DETAILED DESCRIPTION

As stated above, the present invention is based on the unexpected finding of novel naturally split inteins that split after 14-60 amino acids from the intein's N-terminal position.

Thus, these inteins provide the shortest naturally occurring N-terminal intein fragments discovered so far. Moreover, they exhibit excellent splicing yields and rates.

In a first aspect the present invention thus relates to an isolated polypeptide comprising at least one intein or at least one intein fragment of said intein, wherein said intein is a naturally split intein with a N-terminal intein fragment split after 14-60 amino acids from the intein' s N-terminal position.

As used herein, the term "isolated polypeptide" refers to a polypeptide, peptide or protein segment or fragment, which has been separated from sequences, which flank it in a naturally occurring state, e.g., a polypeptide fragment, which has been removed from the sequences which are normally adjacent to the fragment, e.g., the sequences adjacent to the fragment in a protein, in which it naturally occurs. The term also applies to a polypeptide, which has been substantially purified from other components, which naturally accompany the polypeptide, e.g., proteins, RNA or DNA which naturally accompany it in the cell. The term therefore includes, for example, a recombinant polypeptide, which is encoded by a nucleic acid incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences. It also includes a recombinant polypeptide, which is part of a hybrid polypeptide comprising additional amino acids.

Moreover, the isolated polypeptide described herein may comprise in addition to all features described below regulatory sequences, i.e. segments that on nucleic acid level are capable of increasing or decreasing the expression of specific genes within an organism.

The term "intein" as used herein refers to a segment of a protein capable of catalysing a protein splicing reaction that excises the intein sequence from a precursor protein and joins the flanking sequences (N- and C-exteins) with a peptide bond. Hundreds of intein and intein-like sequences have been found in a wide variety of organisms and proteins. They are typically 150-550 amino acids in size and may also contain a homing endonuclease domain.

The term "split intein" as used herein refers to any intein, in which one or more peptide bond breaks exists between the N-terminal and C-terminal amino acid sequences such that the N-terminal and C-terminal sequences become separate fragments that can non-covalently reassociate, or reconstitute, into an intein that is functional for trans-splicing reactions.

The term "intein fragment" as used herein refers to a separate molecule resulting from peptide bond breaks between the N-terminal and C-terminal amino acid sequences in split inteins.

As the N-terminal intein fragments of these inteins are strikingly short, the isolated polypeptides are ideally suited for use in a range of protein modifications, since the protein of interest-Int^{N} (POI- Int^{N}) peptide complex can be easily obtained using solid-phase peptide synthesis. Moreover, since these inteins are natural split inteins generated by evolution, they exhibit high splicing yields and rates, without exhibiting the problems encountered with split inteins artificially engineered to have short Int^{N} fragments.

In a preferred embodiment of this aspect of the present invention the N-terminal intein fragment is split after 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 amino acids from the intein' s N-terminal position. In an especially preferred embodiment the N-terminal intein fragment is split after 24, 25 or 36 amino acids from the intein' s N-terminal position.

In various embodiments of this aspect of the present invention the intein is a naturally split intein with a N-terminal intein fragment split after 24-37 amino acids from the intein' s N-terminal position. Thus, the N-terminal intein fragment of such an intein and/or the protein interest-Int^{N} peptide complex is even shorter and hence better suited for the chemical synthesis, e.g. for solid peptide synthesis, which is faster, easier to perform and much more reliable than protein generation via recombinant protein expression.

In a further aspect the present invention relates to an isolated polypeptide as described above, wherein said at least one intein fragment, is selected from the group comprising:
a) a N-terminal intein fragment having 100% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, or,
b) a N-terminal intein fragment having at least 70%, or 80% or 85% or 90% or 95% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, and/or
c) a C-terminal intein fragment having 100% sequence identity with SEQ ID NO: 6, 27, 39, 45, 51, 57, 63, 69, 75, 81, 87, 93, 99, 105, 1 11, 117, 123, 129, 135, 141, 147, 153, 159, 165, 171, 177, 183, 194, 195 or 196 or,
d) a C-terminal intein fragment having at least 70%, or 80% or 85% or 90% or 95% sequence identity with SEQ ID NO: 6, 27, 39, 45, 51, 57, 63, 69, 75, 81, 87, 93, 99, 105, 111, 117, 123, 129, 135, 141, 147, 153, 159, 165, 171, 177, 183, 194, 195 or 196, or
e) wherein the at least one intein has at least 70%, or 80% or 85% or 90% or 95% sequence identity with SEQ ID NO:1 or 26.

In a preferred embodiment of the invention the split intein is formed by one peptide bond break, i.e. there is one C-terminal intein fragment and one N-terminal intein fragment.

As used herein, the term "N-terminal split intein" or "N-terminal intein fragment" or "N-terminal intein sequence" (abbreviated "Int^{N}")" refers to any intein sequence that comprises an N-terminal amino acid sequence that is functional for trans-splicing reactions. It thus also comprises a sequence that is spliced out when trans-splicing occurs. It can comprise a sequence that is a modification of the N-terminal portion of a naturally occurring intein sequence. For example, it can comprise additional amino acid residues and/or mutated residues so long as the inclusion of such additional and/or mutated residues does not render the Int^{N} non-functional in trans-splicing. Preferably, the inclusion of the additional and/or mutated residues improves or enhances the trans-splicing activity of the Int^{N}.

As used herein, the term "C-terminal split intein" or "C-terminal intein fragment" or "C-terminal intein sequence" (abbreviated "Int^{C}")" refers to any intein sequence that comprises a C-terminal amino acid sequence that is functional for trans-splicing reactions. An Int^{C} thus also comprises a sequence that is spliced out when trans-splicing occurs. An Int^{C} can comprise a sequence that is a modification of the C-terminal portion of a naturally occurring intein sequence. For example, it can comprise additional amino acid residues and/or mutated residues so long as the inclusion of such additional and/or mutated residues does not render the Int^{C} non-functional in trans-splicing. Preferably, the inclusion of the additional and/or mutated residues improves or enhances the trans-splicing activity of the Int^{C}.

The term "sequence identity" as used herein refers to peptides that share identical amino acids at corresponding positions or nucleic acids sharing identical nucleotides at corresponding positions.

In order to take account for the fact that peptides may exists, which do not fall under the term "sequence identity" as they may not have similar amino acids at corresponding positions, but have the same function, because they contain, e.g., conservative substitutions, the amino acid sequences herein are referred to in the context of percent identity.

The determination of percent identity described herein between two amino acid or nucleotide sequences can be accomplished using a mathematical algorithm. For example, a mathematical algorithm useful for comparing two sequences is the algorithm of Karlin and Altschul (1990, Proc. Natl. Acad. Sci. USA 87:2264-2268), modified as in Karlin and Altschul (1993, Proc. Natl. Acad. Sci. USA 90:5873-5877). This algorithm is incorporated into the NBLAST and XBLAST programs and can be accessed, for example, at the National Center for Biotechnology Information (NCBI) world wide web site having the universal resource locator "www.ncbi.nlm.nih.gov/BLAST". Blast nucleotide searches can be performed with BLASTN program, whereas BLAST protein searches can be performed with BLASTX program or the NCBI "blastp" program.

The term "mutant" as used herein refers to polypeptide the sequence of which has one or more amino acids added, deleted, substituted or otherwise chemically modified in comparison to a polypeptide according to one of the claimed sequences, provided always that the mutant retains substantially the same properties as the polypeptide according to one of the claimed sequences.

In one embodiment of this aspect of the present invention the isolated polypeptide comprises at least a N-terminal intein fragment having 100% sequence identity with SEQ ID NOs: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, or,
a N-terminal intein fragment having at least 70%, or 80% or 85% or 90% or 95% sequence identity with SEQ ID NOs: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182

In a different embodiment of this aspect of the present invention the isolated polypeptide comprises at leasta C-terminal intein fragment having 100% sequence identity with SEQ ID NOs: 6, 27, 39, 45, 51, 57, 63, 69, 75, 81, 87, 93, 99, 105, 11 1, 117, 123, 129, 135, 141, 147, 153, 159, 165, 171, 177, 183, 194, 195 or 196 or,
a C-terminal intein fragment having at least 70%, or 80% or 85% or 90% or 95% sequence identity with SEQ ID NOs: 6, 27, 39, 45, 51, 57, 63, 69, 75, 81, 87, 93, 99, 105, 111, 117, 123, 129, 135, 141, 147, 153, 159, 165, 171, 177, 183 194, 195 or 196.

These isolated polypeptides can advantageously be used for example for labelling of a protein. Due to the small size of the N-terminal intein fragment the protein of interest-Int^{N} (POI- Int^{N}) peptide complex can be obtained by using solid-phase peptide synthesis. The label e.g. EGFP attached to the Int^{C} fragment (Int^{C}-EGFP) could be generated by recombinant protein expression. Upon combining the two chimeric protein complexes, i.e. POI-Int^{N} and Int^{C}-EGFP, the trans splicing reaction could take place generating POI-EGFP.

Thus, the two separate intein fragments are useful by themselves. For example, it is possible, to pre-assemble - possibly in form of a kit - the Int^{C}-EGFP fusion protein or merely the Int^{C} fragment, e.g., for easy protein labelling. The ready Int^{C}-EGFP fusion proteins could be then used as soon as a protein of interest is decided upon for easy and robust protein labelling. Of course, the reverse scenario is also possible, where the protein of interest is known and pre-generated fused to the Int^{N} fragment. As soon as it is decided upon which labels should be used the Int^{C}-label fusion proteins could be prepared and protein labelling could be carried out.

Moreover, the EGFP of the fusion protein in this example can of course be readily replaced by any protein of interest.

Additionally, it is preferred in the above scenario that the Int^{C}-protein fusion protein is generated via recombinant expression.

In a different embodiment of this aspect of the invention one of the intein fragments could be attached to a short PEG linker with a thiol group and then bound to a maleimido-coated glass surface. Upon addition of the protein of interest fused to the complementary intein fragment and trans-splicing the protein of interest would remain bound to the glass surface. Thus, it is possible to preassemble such a glass surface, e.g. with the Int^{N} fragment, in order to later on immobilize any protein of interest fused to the complementary Int^{C} fragment. Moreover, Int^{N} fragment preassembled in such a fashion could act as capture probe array.

In various embodiments of this aspect of the present invention an isolated polypeptide comprises at least one N-terminal intein fragment and at least one C-terminal intein fragment, wherein:
1) if said at least one N-terminal intein fragment is selected from SEQ ID NO: 5 then said at least one C-terminal intein fragment is selected from SEQ ID N 6, or
2) if said at least one N-terminal intein fragment is selected from SEQ ID NO: 5, 28-33 then said at least one C-terminal intein fragment is selected from SEQ ID NO: 27, or
3) if said at least one N-terminal intein fragment is selected from SEQ ID NO: 38 then said at least one C-terminal intein fragment is selected from SEQ ID NO: 39, or
4) if said at least one N-terminal intein fragment is selected from SEQ ID NO: 44 then said at least one C-terminal intein fragment is selected from SEQ ID NO: 45, or
5) if said at least one N-terminal intein fragment is selected from SEQ ID NO: 50 then said at least one C-terminal intein fragment is selected from SEQ ID NO: 51, or
6) if said at least one N-terminal intein fragment is selected from SEQ ID NO: 56 then said at least one C-terminal intein fragment is selected from SEQ ID NO: 57, or
7) if said at least one N-terminal intein fragment is selected from SEQ ID NO: 62 then said at least one C-terminal intein fragment is selected from SEQ ID NO: 63, or
8) if said at least one N-terminal intein fragment is selected from SEQ ID NO: 68 then said at least one C-terminal intein fragment is selected from SEQ ID NO: 69, or
9) if said at least one N-terminal intein fragment is selected from SEQ ID NO: 74 then said at least one C-terminal intein fragment is selected from SEQ ID NO: 75, or
10) if said at least one N-terminal intein fragment is selected from SEQ ID NO: 80 then said at least one C-terminal intein fragment is selected from SEQ ID NO: 81, or
11) if said at least one N-terminal intein fragment is selected from SEQ ID NO: 86 then said at least one C-terminal intein fragment is selected from SEQ ID NO: 87, or
12) if said at least one N-terminal intein fragment is selected from SEQ ID NO: 92 then said at least one C-terminal intein fragment is selected from SEQ ID NO: 93, or
13) if said at least one N-terminal intein fragment is selected from SEQ ID NO: 98 then said at least one C-terminal intein fragment is selected from SEQ ID NO: 99, or
14) if said at least one N-terminal intein fragment is selected from SEQ ID NO: 104 then said at least one C-terminal intein fragment is selected from SEQ ID NO: 105, or
15) if said at least one N-terminal intein fragment is selected from SEQ ID NO: 110 then said at least one C-terminal intein fragment is selected from SEQ ID NO: 111, or
16) if said at least one N-terminal intein fragment is selected from SEQ ID NO: 116 then said at least one C-terminal intein fragment is selected from SEQ ID NO: 117, or
17) if said at least one N-terminal intein fragment is selected from SEQ ID NO: 122 then said at least one C-terminal intein fragment is selected from SEQ ID NO: 123, or
18) if said at least one N-terminal intein fragment is selected from SEQ ID NO: 128 then said at least one C-terminal intein fragment is selected from SEQ ID NO: 129, or
19) if said at least one N-terminal intein fragment is selected from SEQ ID NO: 134 then said at least one C-terminal intein fragment is selected from SEQ ID NO: 135, or
20) if said at least one N-terminal intein fragment is selected from SEQ ID NO: 140 then said at least one C-terminal intein fragment is selected from SEQ ID NO: 141, or
21) if said at least one N-terminal intein fragment is selected from SEQ ID NO: 146 then said at least one C-terminal intein fragment is selected from SEQ ID NO: 147, or
22) if said at least one N-terminal intein fragment is selected from SEQ ID NO: 152 then said at least one C-terminal intein fragment is selected from SEQ ID NO: 153, or
23) if said at least one N-terminal intein fragment is selected from SEQ ID NO: 158 then said at least one C-terminal intein fragment is selected from SEQ ID NO: 159, or
24) if said at least one N-terminal intein fragment is selected from SEQ ID NO: 164 then said at least one C-terminal intein fragment is selected from SEQ ID NO: 165, or
25) if said at least one N-terminal intein fragment is selected from SEQ ID NO: 170 then said at least one C-terminal intein fragment is selected from SEQ ID NO: 171, or
26) if said at least one N-terminal intein fragment is selected from SEQ ID NO: 176 then said at least one C-terminal intein fragment is selected from SEQ ID NO: 177, or
27) if said at least one N-terminal intein fragment is selected from SEQ ID NO: 182 then said at least one C-terminal intein fragment is selected from SEQ ID NO: 183, or
28) if said at least one N-terminal intein fragment is selected from SEQ ID NO: 2 then said at least one C-terminal intein fragment is selected from SEQ ID NO: 194, or
29) if said at least one N-terminal intein fragment is selected from SEQ ID NO: 3 then said at least one C-terminal intein fragment is selected from SEQ ID NO: 195, or
30) if said at least one N-terminal intein fragment is selected from SEQ ID NO: 4 then said at least one C-terminal intein fragment is selected from SEQ ID NO: 196.

Advantageously the resulting polypeptide has split intein activity exhibiting excellent splicing yields and rates.

Furthermore, of course all application envisaged for one of the intein fragments also apply for both together.

In preferred embodiments of this aspect of the present invention the isolated polypeptide comprises exactly one N-terminal intein fragment and exactly one C-terminal intein fragment selected as described above.

In yet another aspect the present invention relates to an isolated polypeptide as described above, wherein said polypeptide at the N-terminal end and/or at the C-terminal end of said at least one intein fragment further comprises a sequence selected from:
1) in the case of SEQ ID NO:5 and/or 6 SEQ ID NO: 25 and/or 26, respectively
2) in the case of SEQ ID NO:5, 28-33 and/or 27 SEQ ID NO: 34 and/or 35, respectively
3) in the case of SEQ ID NO:38 and/or 39 SEQ ID NO: 40 and/or 41, respectively
4) in the case of SEQ ID NO:44 and/or 45 SEQ ID NO: 46 and/or 47, respectively
5) in the case of SEQ ID NO:50 and/or 51 SEQ ID NO: 52 and/or 53, respectively
6) in the case of SEQ ID NO:56 and/or 57 SEQ ID NO: 58 and/or 59, respectively
7) in the case of SEQ ID NO:62 and/or 63 SEQ ID NO: 64 and/or 65, respectively
8) in the case of SEQ ID NO:68 and/or 69 SEQ ID NO: 70 and/or 71, respectively
9) in the case of SEQ ID NO:74 and/or 75 SEQ ID NO: 76 and/or 77, respectively
10) in the case of SEQ ID NO:80 and/or 81 SEQ ID NO: 82 and/or 83, respectively
11) in the case of SEQ ID NO:86 and/or 87 SEQ ID NO: 88 and/or 89, respectively
12) in the case of SEQ ID NO:92 and/or 93 SEQ ID NO: 94 and/or 95, respectively
13) in the case of SEQ ID NO:98 and/or 99 SEQ ID NO: 100 and/or 101, respectively
14) in the case of SEQ ID NO:104 and/or 105 SEQ ID NO: 106 and/or 107, respectively
15) in the case of SEQ ID NO:110 and/or 111 SEQ ID NO: 112 and/or 113, respectively
16) in the case of SEQ ID NO:116 and/or 117 SEQ ID NO: 118 and/or 119, respectively
17) in the case of SEQ ID NO:122 and/or 123 SEQ ID NO: 124 and/or 125, respectively
18) in the case of SEQ ID NO:128 and/or 129 SEQ ID NO: 130 and/or 131, respectively
19) in the case of SEQ ID NO:134 and/or 135 SEQ ID NO: 136 and/or 137, respectively
20) in the case of SEQ ID NO:140 and/or 141 SEQ ID NO: 142 and/or 143, respectively
21) in the case of SEQ ID NO:146 and/or 147 SEQ ID NO: 148 and/or 149, respectively
22) in the case of SEQ ID NO:152 and/or 153 SEQ ID NO: 154 and/or 155, respectively
23) in the case of SEQ ID NO:158 and/or 159 SEQ ID NO: 160 and/or 161, respectively
24) in the case of SEQ ID NO:164 and/or 165 SEQ ID NO: 166 and/or 167, respectively
25) in the case of SEQ ID NO:170 and/or 171 SEQ ID NO: 172 and/or 173, respectively
26) in the case of SEQ ID NO:176 and/or 177 SEQ ID NO: 178 and/or 179, respectively
27) in the case of SEQ ID NO:182 and/or 183 SEQ ID NO: 184 and/or 185, respectively.

Such an isolated polypeptide has the advantage that the autocatalytic reaction - the protein splicing - proceeds with higher efficiency, if 1-5 of the wild type extein residues (also termed flanking sequences, i.e. sequences flanking the intein) are present.

In this context, it is emphasized that in general the sequences in this application are shown without the +1 residue following the Int^{C}, as this residue is strictly not part of the intein. However, it should be noted that this residue is usually involved in the intein's activity and forms part of the intein active site.

Additionally, in case of an isolated polypeptide comprising one N-terminal intein fragment and one C-terminal intein fragment, wherein the N-terminal intein fragment having 100% sequence identity with SEQ ID NO: 5 or having at least 70%, or 80% or 85% or 90% or 95% sequence identity with SEQ ID NO:5 and the C-terminal intein fragment having 100% sequence identity with SEQ ID NO: 6 having at least 70%, or 80% or 85% or 90% or 95% sequence identity with SEQ ID NO: 6 the activity maximum at low temperatures such as 8°C is ideal to preserve potentially fragile proteins of interest for example in *in vitro* protein labelling experiments.

Moreover, it was surprisingly possible to further modify the AceL-TerL intein (SEQ ID NO: 1) to increase splicing yields and rates even at 37°C.

Thus, in a further aspect the present invention relates to an isolated polypeptide wherein the N-terminal intein fragment has 100% sequence identity with a sequence selected from sequences comprising SEQ ID NO: 5, 11 and 12, or has at least 90% or 95% sequence identity with a sequence selected from sequences comprising SEQ ID NO:5, 11 or 12 and/or, wherein the C-terminal intein fragment has 100% sequence identity with a sequence selected from sequences comprising SEQ ID NO:6 or 13-18 or has at least 90% or 95% sequence identity with a sequence selected from sequences comprising SEQ ID NO:6 or 13-18.

Such isolated polypeptides provide novel split inteins ideally suited for protein modification and semi-synthesis due to their superior splicing yields and rates.

In various embodiments of this aspect of the invention the isolated polypeptide comprises at least one N-terminal intein fragment and at least one C-terminal intein fragment wherein the N-terminal intein fragment is selected from sequences comprising SEQ ID NO: 5, 11 or 12 and the C-terminal intein fragment is selected from sequences comprising SEQ ID NO:6 or 13-18.

Besides their superior splicing yields and rates the novel split inteins are capable of efficient splicing at 37°C. This characteristic is advantageous as it renders the inteins more thermostable, better suited for expression of fusion proteins in organisms such as *E. coli* and in general broadens their practical utility for a range of applications.

In detail, a clear improvement over the wild-type AceL-TerL intein trans-splicing reactions at 37°C could be observed for all the mutants. Rates were increased by about 2- to 14-fold (Figure 9 and Figure 11) and yields were increased to 65-85% after 24h compared to about 60% for the wild-type intein, with concomitant decrease of the C-cleavage product (Figure 9).

Especially preferred combinations of N-terminal intein fragments selected from WT^{N}, M1^{N}, M3^{N} and C-terminal intein fragments selected from sequences WT^{C}, M1^{C}, M2^{C}, M3^{C}, M4^{C}, M5^{C}, M6^{C} are listed in Table 1.

As used herein the term "wild-type" refers to the phenotype of the typical form of a species as it occurs in nature. In relation to the provided intein sequences it should be noted that the term can also refer to artificially joined sequences, which themselves possess the wild-type succession of amino acids or nucleotides, respectively.

The term "splicing yield" as used herein refers to the amount of splice product produced. Ideally the intein mediated trans-splicing reaction would only result in splice product comprising the extein sequences attached to the intein fragments prior to the reaction. However, under unfavourable conditions or when using an intein, which is not suitable, the formation of cleavage side-product may occur. This lowers the overall splicing yield.

The term "splicing rate" as used herein refers to the change in concentration of the products per unit time. It is expressed using a rate constant k. The rate constant, k, which is temperature dependent, is a proportionality constant for a given reaction.

In still a further aspect the present invention relates to an isolated polypeptide wherein the polypeptide comprises the N-terminal intein fragment with SEQ ID NO: 5 or 12 and the C-terminal intein fragment with SEQ ID NO:13.

Such an isolated polypeptide provides a novel split intein with even better splicing yields and rates.

In detail, these combinations spliced significantly faster with a beneficial ratio between splicing and cleavage yields (Figure 10).

In yet a further aspect the present invention relates to an isolated polypeptide as described above wherein the N-terminal intein fragment has 100% sequence identity with a sequence selected from sequences comprising SEQ ID NOs: 5, 28-33 or has at least 90% or 95% sequence identity with a sequence selected from sequences comprising SEQ ID NOs: 28-33 and/or, wherein the C-terminal intein fragment has 100% sequence identity with SEQ ID NO: 27 or has at least 90% or 95% sequence identity with with SEQ ID NO: 27.

As shown in figures 19-22 these polypeptides exhibit high splicing yields and rates, even if the Int^{N} and/or Int^{C} sequences were slightly modified.

Moreover, it was unexpectedly found that the Int^{C} with SEQ ID NO: 27 is not only capable of splicing with the wild-type Int^{N} sequence with SEQ ID NO: 28, but is also capable of splicing with the Int^{N} with SEQ ID NO: 5. In addition, this cross splicing proceeds with both the original Cys1 of SEQ ID NO: 5 and with a Ser1 at this position (cf., Figures 23 and 24). The C1S substitution could be advantageous, for example in order to minimize the oxidation risk of the peptide.

In addition, the fact that the Int^{C} with SEQ ID NO: 27 is capable of splicing with the Int^{N} with SEQ IS NO 5, i.e. cross-splices, is advantageous as it substantially extends the possibilities for use of both intein fragments. In other words, it could for example be envisaged to pre-assemble - possibly in form of a kit - an Int^{C}-EGFP fusion protein comprising SEQ ID NO: 27 or merely the Int^{C} fragment, e.g., for easy protein labelling. The ready Int^{C}-EGFP fusion proteins could then be used with either the Int^{N} of SEQ ID NO: 5, SEQ ID NO: 28-33, whichever one is available, cheapest to generate or for other reasons most suitable for easy and robust protein labelling.

In still a further aspect, the present invention relates to an isolated polypeptide further comprising at least one C-terminal extein or at least one N-terminal extein sequence.

The term "extein" or "extein sequence" as used herein refers to the peptide sequences that link to form a new polypeptide after the intein has excised itself during splicing. In other words, the N-terminal and C-terminal exteins (also termed Ex^{N} and Ex^{C} flank the Int^{N} and Int^{C} fragments, respectively prior to the trans-splicing reaction.

In various embodiments of this aspect of the present invention the isolated polypeptide comprises at least one C-terminal extein and least one N-terminal extein sequence.

In various embodiments of this aspect of the present invention the isolated polypeptide comprises exactly one C-terminal extein and exactly one N-terminal extein sequence.

In yet still other embodiments of this aspect of the present invention at least one of the peptide sequences of the isolated polypeptide selected from N-terminal intein, C-terminal intein, C-terminal extein and/or N-terminal extein is a recombinant protein.

Through using extein sequences that are heterologous to the chosen intein sequences, the full power of split inteins can be exploited, as the extein sequences will be joined by a peptide bond following the trans-splicing reaction with virtually no trace of the previously existing intein sequences.

Thus, in a further aspect the invention explicitly relates to two isolated polypeptides as described above wherein one of the isolated polypeptides comprises least one heterologous N-terminal extein fused to a N-terminal intein fragment having at least 70%, or 80% or 85% or 90% or 95% or 99% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182 and
wherein the second one of the isolated polypeptides comprises at least one C-terminal extein sequence fused to a C-terminal intein fragment having at least 70%, or 80% or 85% or 90% or 95% or 99% sequence identity with SEQ ID NO:6, 27, 39, 45, 51, 57, 63, 69, 75, 81, 87, 93, 99, 105, 111, 117, 123, 129, 135, 141, 147, 153, 159, 165, 171, 177, 183, 194, 195, 196.

While the at least one polypeptide as described above of course does not exclude the presence or use of a second polypeptide as described above advantageously the complementary Int^{N} and Int^{C} fragments with their respective extein sequences are used together, as thereby the full potential of split inteins is realized, e.g. in applications such as modification of a protein, protein lipidation, protein immobilization, protein backbone semisynthetic, artificial control of protein splicing by light and use as a molecular switch. This is especially the case, if the extein sequences are heterologous to the intein fragments and possibly to each other. As mentioned above in such a case the extein sequences, which can be synthesized separately, will be joined by a peptide bond following the trans-splicing reaction with virtually no trace of the previously existing intein sequences.

In various embodiments of all aspect of the present invention the isolated polypeptide is a contiguous *cis* splicing polypeptide.

The term "cis splicing polypeptide" as used herein refers to a polypeptide, which has a continuous polypeptide sequence.

In a preferred embodiment the contiguous *cis* splicing polypeptide has the amino acid sequence of SEQ ID NO:7 or 8.

In a further aspect the present invention relates to an isolated polypeptide further comprising at least one component selected from a solubility factor, a marker, a linker, an epitope, an affinity tag, a fluorophore or a fluorescent protein, a toxic compound or protein and a small-molecule or a small-molecule binding protein .

As used herein the term "marker" refers to a component allowing detecting directly or indirectly the molecules having said marker. Thus, in some embodiments the marker is a label such as a fluorophore.

As used herein the term "linker" refers to a chemical moiety that connects parts of the isolated polypeptide or the nucleic acids described herein. Thus, a linker may be especially employed in the case of *cis* splicing polypeptides.

As used herein the term "epitope" refers to an immunogenic amino acid sequence.

As used herein the term "affinity tag" refers to a polypeptide sequence, which has affinity for a specific capture reagent and which can be separated from a pool of proteins and thus purified on the basis of its affinity for the capture reagent.

As used herein the term "fluorophore" means a compound or group that fluoresces when exposed to and excited by a light source, i.e. it re-emits light.

As used herein the term "fluorescent protein" refers to a protein that is fluorescent, e.g., it may exhibit low, medium or intense fluorescence upon exposure to electromagnetic radiation.

As used herein the term "small molecule" refers to any molecule, or chemical entity, with a molecular weight of less than about 1,000 Daltons.

These additional features are advantageous in order to increase the range of applications for which the isolated polypeptide can be used.

Preferred solubility factors are maltose binding protein (MPB), SUMO (small-ubiquitin like modifier), StreptagII, a His-tag, SBP (streptavidin binding peptide) and GST (glutathione-S-transferase.

Especially preferred is MBP as an N-terminal tag.

This has the advantage that protein expression and solubility are significantly increased.

Preferred markers are green and red fluorescent proteins (EGFP and mRFP), Gaussia princeps luciferase Gluc.

In various embodiments of this aspect of the present invention the isolated polypeptide comprises a linker fragment inserted between the at least two intein fragments.

In still other various embodiments of this aspect of the present invention the linker fragment is selected from the amino acid sequence MG or the amino acid sequence MGSGGSG.

This has the advantage that protein expression of the contiguous *cis* splicing polypeptide is improved.

In various embodiments of all aspect of the present invention the isolated polypeptide has the highest splicing yield and splicing rate at 8°C or 37°C.

In a further aspect the present invention relates an isolated nucleic acid molecule comprising a nucleotide sequence encoding for at least one polypeptide described above or a homolog, mutant or complement thereof.

The term "variant" as used herein refers to a nucleic acid molecule which is substantially similar in structure and biological activity to a nucleic acid molecule according to one of the claimed sequences.

The term "mutant" refers to a nucleic acid molecule the sequence of which has one or more nucleotides added, deleted, substituted or otherwise chemically modified in comparison to a nucleic acid molecule according to one of the claimed sequences, provided always that the mutant retains substantially the same properties as the nucleic acid molecule according to one of the claimed sequences.

As used herein, the term "complement" refers to the complementary nucleic acid of the used/known/discussed nucleic acid. This is an important concept since in molecular biology, complementarity is a property of double-stranded nucleic acids such as DNA and RNA as well as DNA:RNA duplexes. Each strand is complementary to the other in that the base pairs between them are non-covalently connected via two or three hydrogen bonds. Since there is in principle - exceptions apply for thymine/uracil and the tRNA wobble conformation - only one complementary base for any of the bases found in nucleic acids, one can reconstruct a complementary strand for any single strand.

However, for double stranded DNA the term "complement" can also refer to the complementary DNA (cDNA). cDNA can be synthesized from a mature mRNA template in a reaction catalyzed by the enzyme reverse transcriptase.

In various embodiments of this aspect of the present invention the isolated nucleic acid molecule has or comprises SEQ ID NO:19-23.

In further embodiments of this aspect of the invention the isolated nucleic acid molecule is comprised in a vector, preferably a plasmid.

The term "vector", as used herein, refers to a molecular vehicle used to transfer foreign genetic material into another cell. The vector itself is generally a DNA sequence that consists of an insert (sequence of interest) and a larger sequence. The purpose of a vector to transfer genetic information to another cell is typically to isolate, multiply, or express the insert in the target cell.

The term "plasmid", as used herein, refers to plasmid vectors, i.e. circular DNA sequences that are capable of autonomous replication within a suitable host due to an origin of replication ("ORI"). Furthermore, a plasmid may comprise a selectable marker to indicate the success of the transformation or other procedures meant to introduce foreign DNA into a cell anda multiple cloning site which includes multiple restriction enzyme consensus sites to enable the insertion of an insert. Plasmid vectors called cloning or donor vectors are used to ease the cloning and to amplify a sequence of interest. Plasmid vectors called expression or acceptor vectors are specifically for the expression of a gene of interest in a defined target cell. Those plasmid vectors generally show an expression cassette, consisting of a promoter, the transgene and a terminator sequence. Expression plasmids can be shuttle plasmids containing elements that enable the propagation and selection in different host cells.

In further embodiments of this aspect of the at least one isolated nucleic acid molecule is comprised in a host cell.

The term "host cell", as used herein refers to a transgenic cell, which is used as expression host. Said cell, or its progenitor, has thus been transfected with a suitable vector comprising the cDNA of the protein to be expressed.

In a further aspect the present invention relates to a protein expression system comprising an isolated polypeptide as described above or an isolated nucleic acid molecule as described above expressed from a plasmid in a host cell, e.g. *E.coli.*

In yet another aspect the present invention relates to a method using an isolated polypeptide as described above or an isolated nucleic acid molecule as described above, wherein the method is selected from the group consisting of modification of a protein, protein lipidation, protein immobilization, protein backbone semi-synthesis, regioselective protein side chain modification and artificial control of protein splicing by light, by a small molecule or a temperature change.

As used herein the term "protein lipidation" refers to the covalent modification of peptides, polypeptides and proteins with a variety of lipids, including fatty acids, isoprenoids, and cholesterol. Lipid modifications play important roles in the localization and function of proteins.

The term "semi-synthesis" as used herein refers to partial chemical synthesis, i.e. a type of chemical synthesis that uses compounds isolated from natural sources (e.g. plant material or bacterial or cell cultures) as starting materials. This is opposed to a total synthesis where large molecules are synthesized from a stepwise combination of small and cheap (usually petrochemical) building blocks.

The term "backbone semi-synthesis" as used herein refers to generation of a protein consisting of a polypeptide segment derived from recombinant protein expression and a segment obtain by organic peptide synthesis.

In a preferred embodiment of this aspect of the present invention the isolated polypeptide is modified in such a way it can be specifically induced to cleave, resulting in a separation of the intein and the N-terminal and/or C-terminal extein sequences. Such a modification can, for example, be achieved via point mutations in at least one of the extein sequences or by omitting one of the extein sequences.

Such a system could, e.g. be used for the purification of proteins with the subsequent cleavage of the employed affinity tag.

Protein trans-splicing (PTS) using inteins is especially well suited for these applications since the protein of interest (POI) will be assembled from two parts (fused as extein sequences to the intein fragments) and each of these fusion constructs can be prepared individually before the PTS reaction. Due to the small size the split intein fragments, one of the intein fusion proteins POI-Int^{N} or Int^{C}-POI can be obtained by using solid-phase peptide synthesis. Alternatively, both fusion proteins can be recombinant but treated individually with bioconjugation chemicals to regioselectively introduce a synthetic label.

Moreover, a method of protein modification employing an isolated polypeptide as described above can be carried out it in complex systems like a living cell or a cell extract.

In various embodiments of this aspect of the present invention the modification is a protein-terminal modification.

In various embodiments of this aspect of the present invention the N-terminal modification is protein labelling.

In a further aspect the present invention relates to a method for the ligation of at least one first peptide to at least one second peptide using an isolated polypeptide as described above or an isolated nucleic acid molecule as described above.

In this context it should be noted that the isolated polypeptide as described above or the isolated nucleic acid molecule as described above, respectively, can be used to generate one of the functional groups for the ligation reaction e.g. for native chemical ligation (NCL) or expressed protein ligation (EPL) reactions or can be employed to create the peptide bonds itself via intein mediated protein trans-splicing.

Thus, in various embodiments of this aspect of the present invention the method comprises covalently linking the N-terminus of the first protein to the C-terminus of the second protein, i.e. it is an intein mediated protein trans-splicing reaction.

In this context the isolated polypeptide described above is advantageous, because it allows for ligation at low concentrations and in the presence of other components.

In yet another aspect the present invention relates to the use of a polypeptide or a nucleic acid molecule as described above, wherein the use is selected from the group consisting of modification of a protein, protein lipidation, protein immobilization, protein backbone semisynthesis, regioselective protein side chain modification, and use as molecular switch.

In various aspects of this embodiment of the present invention the modification of a protein is selected from site-selective introduction of synthetic moieties into proteins and N-terminal modification.

In various aspects of this embodiment of the invention the described polypeptide or nucleic acids is used protein engineering or protein semi-synthesis.

Moreover, inteins have also been recognized as molecular switches that mediate protein splicing as an output signal only when a certain input signal is given. The latter represents the condition under which the intein is active. By fusing additional polypeptides that mediate a protein-protein interaction to the "free" termini of the split intein fragments the systems can be designed to either work as a biosensor or as an experimental tool to control biological activities that rely on the primary structure of the spliced polypeptide or protein.

In another aspect the present invention relates to a kit comprising a polypeptide or a nucleic acid molecule as described above.

In a preferred embodiment the kit comprises at least one polypeptide as described above comprising an Int^{N} fragment as described above fused to a marker.

In various embodiments of this embodiment of the intention the kit further comprises at least one component selected from the group consisting of at least one vector, at least one resin, DTT, at least one plasmid, at least one expression host, at least one loading buffer, at least one antibody.

As used herein the term "expression host" refers to prokaryotic and eukaryotic expression system hosts, including but not limited to bacteria.

Such a kit is advantageous, *inter alia,* for ligation and labelling of recombinant proteins, as no proteases, which potentially splice the target protein, have to be used.

In one embodiment of this aspect of the present invention the kit comprises a vector encoding the Int^{c} fragment, into which the protein of interest can be easily cloned. Thus, the Int^{C} -protein of interest fusion protein can be easily expressed.

In addition, in one embodiment of this aspect of the invention the kit comprises the Int^{N} fragment as synthetic peptide fused to a chemical modification desired for the protein of interest, e.g. a fluorophore, biotin etc. In a preferred embodiment the kit comprises the Int^{N} fragment as synthetic peptide fused to a variety of chemical modifications.

In an alternative embodiment the Int^{C} and Int^{N} fragments along with their respective components are comprised in separate kits.

In various embodiments of all aspect of the present invention the splice site of the isolated polypeptide is at amino acids 24-25 from the intein's N-terminal position.

This is advantageous as due to the small size of the N-terminal intein fragment the POI- Int^{N} complex can be obtained by using solid-phase peptide synthesis.

In various embodiments of all aspect of the present invention the N-terminal intein fragment of the isolated polypeptide comprises 24-25 amino acids and the C-terminal intein fragment of the isolated polypeptide comprises 104-105 amino acids.

This property has the advantage that small proteins and especially small intein fragments are more suitable for a range of applications such as chemical or biological, i.e. recombinant protein synthesis.

In various embodiments of all aspect of the present invention the isolated polypeptide is or is comprised in a recombinant protein and/or an antibody and/or a protein hormone.

Other embodiments are within the following non-limiting examples.

### EXAMPLES

### Example 1: Isolation and Characterization of AceL-TerL with SEQ ID NO: 1

Several closely related phage genes with inteins were identified in metagenomic data from the Antarctic permanently stratified saline Ace Lake (F. M. Lauro, M. Z. DeMaere, S. Yau, M. V. Brown, C. Ng, D. Wilkins, M. J. Raftery, J. A. Gibson, C. Andrews-Pfannkoch, M. Lewis, J. M. Hoffman, T. Thomas, R. Cavicchioli, ISME J. 2011, 5, 879). The genes were of a T4 bacteriophage-type DNA packaging large subunit terminase, and were subsequently termed AceL-TerL inteins (from Ace lake terminase large subunit).

It was shown that these inteins have a novel split site corresponding to a probable surface loop region of the intein with no defined secondary structure following β-strand 3 and α-helix 1 of the typical intein structure (Figure 2). In contrast, previously reported split sites close to the N-terminal splice junction were all created artificially.

The intein with SEQ ID NO: 1, simply termed AceL-TerL intein hereafter, was chosen for functional characterization. To this end, the Int^{N} of 25 aa was prepared by solid-phase peptide synthesis with three native N-extein residues, two lysine residues, and a 5(6)-carboxyfluoresceine moiety (FI-) to give pepWT^{N} (FI-KKEFE-IntN). The Int^{C} (aa 26-129) was recombinantly expressed in E. coli as a fusion with hexahistidine-tagged thioredoxin as a model protein (construct WT^{C}-Trx-H6) and purified using Ni-NTA chromatography. Upon mixing of the two components spontaneous protein trans-splicing was observed (Figure 3). The formation of the splice product (SP) FI-KKEFE-Trx-H6 and of the excised intein fragments as by-products indicated that this intein was fully active.

As demonstrated in (Figure 7) a clear temperature dependence was observed from splicing assays at 8°C, 25°C, and 37°C, with the highest rate at 8°C (k = 1.7 ± 0.2 x 10⁻³ s⁻¹; t½ = 7.2 ± 1.1 min) and a ∼50-fold slower rate at 37°C. Importantly, no C-terminal cleavage side-product (Trx-H6) could be detected at 8°C (Figure 3 and Figure 4). Such cleavage products are often observed for engineered inteins and may significantly limit the practical utility of the intein system. Together, these results indicated the excellent potential of this naturally split intein for protein trans-splicing applications, also aided by its total length of only 129 aa, being one of the shortest known inteins. The rate and yield of about 90% of the AceL-TerL intein at 8°C is remarkable.

### Example 2: Modification of wild-type AceL-TerL

Although low temperatures like 8°C appear ideal to preserve potentially fragile proteins of interest in *in vitro* experiments, expression of the intein fusion proteins in *E. coli* has to be performed at higher temperatures. Furthermore, inteins with higher thermostability should be beneficial for high activity in diverse sequence contexts, potentially also at lower temperatures, and for cellular applications.

Thus, in order to generate modified AceL-TerL inteins with a higher activity at 37°C, the AceL-TerL intein was converted into a contiguous, cis-splicing intein by fragment fusion (Figure 5) and inserted on the DNA level into the KanR gene. Active intein alleles capable of splicing out of the translated gene product can be selected because they render the host *E*. *coli* cells resistant to the antibiotic kanamycin. The non-mutated intein gave rise to colony growth at 25°C under selective conditions, but not at 37°C. This finding correlated with protein splicing activity determined by Western blot analyses (data not shown). These results provided the basis for selection by temperature.

Subsequently, a library encoding mutant inteins was created using error prone PCR (epPCR) and used to transform *E. coli* cells. Randomly picked kanamycin-resistant colonies that were selected on plates at 37°C were then re-streaked on plates with kanamycin concentrations of up to 150 µg/ml. Plasmids isolated from resistant clones were analyzed by DNA-sequencing. Five different mutant inteins, termed M1 to M5 (Table 1), were selected and confirmed by Western blotting to have acquired splicing activity at this elevated temperature (Figure 6). The mutant inteins contained one to four amino acid substitutions, in both the Int^{N} and IntC parts (Table 1). To discern the effect of individual mutations, an additional construct with the single L55Q mutation, termed M6 (Table 1), was created by site-directed mutagenesis.

**Table 1: Mutations in the modified AceL-TertL intein fragments**

| **Mutant Name** | **Int^{N}** | **Int^{C}** |
|---|---|---|
| M1 | A25T | N46D, L55Q |
| M2 | - | S38G, N46I, N54D, L55Q |
| M3 | Y3S | S93G |
| M4 | - | N46I, L55Q |
| M5 | - | N46D |
| M6 | - | L55Q |
| MX1 | - | N46D, L55Q |
| M31 | Y3S | N46D, L55Q |

### Example 3: Characterization of the modified AceL-TerL intein fragments

The effect of the mutations M1 to M6 was investigated. The Int^{C} fragments of the M1 to M6 mutants, termed M1^{C} to M6^{C}, were expressed and purified as Int^{C}-Trx-H6 fusion proteins, and the Int^{N} parts of the M1 and M3 mutants, termed M1^{N} and M3^{N}, were included in two synthetic peptides of the format FI-KKEFE-IntN (pepM1^{N} and pepM3^{N}, respectively). A clear improvement over the wild-type intein protein trans-splicing reactions at 37°C could be observed for all the mutants. Rates were increased by about 2- to 14-fold (Figure 9 and Figure 11) and yields were increased to 65-85% after 24h compared to about 60% for the wild-type intein, with concomitant decrease of the C-cleavage product (Figure 9).

Moreover, the mutations seemed to have additive effects, as exemplified by the observation that the combined N46D and L55Q mutations (pepWT^{N} + M1^{C}) resulted in higher rates than the individual mutations (pepWT^{N} + M5^{C} and pepWT^{N} + M6^{C}, respectively) (Figure 10 and Figure 12).

### Example 4: Further optimization of the modified AceL-TerL intein fragments

The Int^{N}-mutation of the M3 mutant (Y3S) were combined with the Int^{C}-mutations of the M1 (N46D, L55Q) or M2 (S38G, N46I, N54D, L55Q) mutants (pepM3N + M1C and pepM3N + M2C). These combinations spliced ∼29-fold and ∼56-fold faster, respectively, than the wild-type at the selection temperature of 37°C (Figure 10).

As the combinations of pepM3^{N} + M1^{C} and pepWT^{N} + M1^{C} surprisingly demonstrated a superior ratio between splicing and cleavage yields these were chosen for subsequent experiments. They were termed MX1 mutant (pepWT^{N} + M1^{C}) and M31 mutant (pepM3^{N} + M1^{C}) (Table 1 and Figure 10).

### Example 5 N-terminal chemical modification of proteins

The maltose binding protein (MBP) was included as an N-terminal tag to give the construct MBP-Int^{C}-TycB1 with the AceL-TerL MX1 and M31. MBP improved semi-synthetic protein trans-splicing of the wild-type AceL-TerL intein and mutants (data not shown). In particular, the MX1 mutant was efficiently expressed, well soluble, and spliced at 8°C to give yields of 80-95% with a 13-fold higher rate than the M86 DnaB mutant intein and a 15-fold higher rate than the unevolved wild-type AceL-TerL intein (Figure 8). Similar results were obtained in a detailed kinetic study using Trx as the protein of interest (Figure 8).

### Example 6: Chemical modification using AceL-TerL mutants

In order to demonstrate the applicability of the AceL-TerL intein mutants for the chemical modification of a diverse range of proteins of interest, the MX1 mutant was fused with green and red fluorescent proteins EGFP and mRFP, Gaussia princeps luciferase Gluc, as well as the murine E2 conjugating enzyme Ubc9 and the human protease SENP1 from the SUMO pathway.

As shown in Figures 14-18, all tested proteins were efficiently modified with the synthetic fluorophore at the N-terminus, with ∼ 80% yields of the desired conjugates after 3 h at 8°C. For a biochemical characterization of the proteins, the fluorescently labeled enzymes TycB1 and SENP1 were prepared and purified on a preparative scale and could be shown to be fully catalytically active (Figures 15-18). These labeled proteins will prove useful in future biophysical studies.

In summary, a novel type of naturally split intein with an unusually short N-terminal fragment of only 25 amino acid was unexpectedly identified from metagenomic data. Significantly improved mutants of this intein could surprisingly be generated. These intein fragments and the corresponding inteins respectively, are likely to serve as powerful and generally applicable tools for the N-terminal chemical modification of proteins using semisynthetic protein trans-splicing. Advantages of this approach over chemical ligation reactions include the low required reactant concentrations, the absence of non-proteinogenic functional groups to facilitate the reaction, and the orthogonality to the cellular chemical environment. The high activity of the new split inteins at low temperatures like 8°C is of particular advantage for in vitro labeling experiments with fragile proteins.

**Table 2. Sequences overview:**

| **SEQ ID NO:** | **Name** | **Sequence** |
|---|---|---|
| 1 | aa WTAceL-TerL-11 | CVYGDTMVETEDGKIKIEDLYKRLAMFRTNTNNIKILSPNGFSNFN GIQKVERNLYQHIIFDDDTEIKTSINHPFGKDKILARDVKVGDYLNS KKVLYNELVNENIFLYDPINVEKESLYITNGVVSHN |
| 2 | aa WTAceL-TerL-3 Int^{N} | CVDGNTIVETEDGKIKIPDLYKKL |
| 3 | aa WTAceL-TerL-4 Int^{N} | CVDGNTIVETEDGKIKIPDLYKKM |
| 4 | aa WTAceL-TerL-5 Int^{N} | CVDGNTIVETEDGKIKIPDLYKKL |
| 5 | Aa WTAceL-TerL-11 Int^{N} | CVYGDTMVETEDGKIKIEDLYKRLA |
| 6 | aa WTACeL-TerL-11 Int^{C} | MFRTNTNNIKILSPNGFSNFNGIQKVERNLYQHIIFDDDTEIKTSINH PFGKDKILARDVKVGDYLNSKKVLYNELVNENIFLYDPINVEKESL YITNGVVSHN |
| 7 | aa (MBP-AceL-TerL^{cis}-FKBP-His6) pIT063 | MEIEEGKLVIWINGDKGYNGLAEVGKKFEKDTGIKVTVEHPDKLEE KFPQVAATGDGPDIIFWAHDRFGGYAQSGLLAEITPDKAFQDKLYP FTWDAVRYNGKLIAYPIAVEALSLIYNKDLLPNPPKTWEEIPALDKE LKAKGKSALMFNLQEPYFTWPLIAADGGYAFKYENGKYDIKDVG VDNAGAKAGLTFLVDLIKNKHMNADTDYSIAEAAFNKGETAMTIN GPWAWSNIDTSKVNYGVTVLPTFKGQPSKPFVGVLSAGINAASPN KELAKEFLENYLLTDEGLEAVNKDKPLGAVALKSYEEELAKDPRIA ATMENAQKGEIMPNIPQMSAFWYAVRTAVINAASGRQTVDEALK DAQTNSSSNNNNNNNNNNNLGIEGRISEFEFECVYGDTMVETEDGKI KIEDLYKRLAMGMFRTNTNNIKILSPNGFSNFNGIQKVERNLYQHII FDDDTEIKTSINHPFGKDKILARDVKVGDYLNSKKVLYNELVNENI FLYDPINVEKESLYITNGVVSHNCEFLSRNNGNGNGTRGVQVETISP GDGRTFPKRGQTCVVHYTGMLEDGKKFDSSRDRNKPFKFMLGKQ EVIRGWEEGVAQMSVGQRAKLTISPDYAYGATGHPGIIPPHATLVF DVELLKLETSYGSRSHHHHHH |
| 8 | aa (MBP-AceL-TerL^{cis}-FKBP-His6) pIT064: | MEIEEGKLVIWINGDKGYNGLAEVGKKFEKDTGIKVTVEHPDKLEE KFPQVAATGDGPDIIFWAHDRFGGYAQSGLLAEITPDKAFQDKLYP FTWDAVRYNGKLIAYPIAVEALSLIYNKDLLPNPPKTWEEIPALDKE LKAKGKSALMFNLQEPYFTWPLIAADGGYAFKYENGKYDIKDVG VDNAGAKAGLTFLVDLIKNKHMNADTDYSIAEAAFNKGETAMTIN GPWAWSNIDTSKVNYGVTVLPTFKGQPSKPFVGVLSAGINAASPN KELAKEFLENYLLTDEGLEAVNKDKPLGAVALKSYEEELAKDPRIA ATMENAQKGEIMPNIPQMSAFWYAVRTAVINAASGRQTVDEALK DAQTNSSSNNNNNNNNNNNLGIEGRISEFEFECVYGDTMVETEDGKI KIEDLYKRLAMGSGGSGMFRTNTNNIKILSPNGFSNFNGIQKVERN LYQHIIFDDDTEIKTSINHPFGKDKILARDVKVGDYLNSKKVLYNEL VNENIFLYDPINVEKESLYITNGVVSHNCEFLSRNNGNGNGTRGVQ VETISPGDGRTFPKRGQTCVVHYTGMLEDGKKFDSSRDRNKPFKF MLGKQEVIRGWEEGVAQMSVGQRAKLTISPDYAYGATGHPGIIPP HATLVFDVELLKLETSYGSRSHHHHHH |
| 9 | pIT065:inaktiv(N129A,C+1A ), | MEIEEGKLVIWINGDKGYNGLAEVGKKFEKDTGIKVTVEHPDKLEE KFPQVAATGDGPDIIFWAHDRFGGYAQSGLLAEITPDKAFQDKLYP FTWDAVRYNGKLIAYPIAVEALSLIYNKDLLPNPPKTWEEIPALDKE LKAKGKSALMFNLQEPYFTWPLIAADGGYAFKYENGKYDIKDVG VDNAGAKAGLTFLVDLIKNKHMNADTDYSIAEAAFNKGETAMTIN GPWAWSNIDTSKVNYGVTVLPTFKGQPSKPFVGVLSAGINAASPN KELAKEFLENYLLTDEGLEAVNKDKPLGAVALKSYEEELAKDPRIA ATMENAQKGEIMPNIPQMSAFWYAVRTAVINAASGRQTVDEALK DAQTNSSSNNNNNNNNNNNLGIEGRISEFEFECVYGDTMVETEDGKI KIEDLYKRLAMGMFRTNTNNIKILSPNGFSNFNGIQKVERNLYQHII FDDDTEIKTSINHPFGKDKILARDVKVGDYLNSKKVLYNELVNENI FLYDPINVEKESLYITNGVVSHAAEFLSRNNGNGNGTRGVQVETISP GDGRTFPKRGQTCVVHYTGMLEDGKKFDSSRDRNKPFKFMLGKQ EVIRGWEEGVAQMSVGQRAKLTISPDYAYGATGHPGIIPPHATLVF DVELLKLETSYGSRSHHHHHH |
| 10 | pIT066:inaktiv(N129A,C+1A ), | MEIEEGKLVIWINGDKGYNGLAEVGKKFEKDTGIKVTVEHPDKLEE KFPQVAATGDGPDIIFWAHDRFGGYAQSGLLAEITPDKAFQDKLYP FTWDAVRYNGKLIAYPIAVEALSLIYNKDLLPNPPKTWEEIPALDKE LKAKGKSALMFNLQEPYFTWPLIAADGGYAFKYENGKYDIKDVG VDNAGAKAGLTFLVDLIKNKHMNADTDYSIAEAAFNKGETAMTIN GPWAWSNIDTSKVNYGVTVLPTFKGQPSKPFVGVLSAGINAASPN KELAKEFLENYLLTDEGLEAVNKDKPLGAVALKSYEEELAKDPRIA ATMENAQKGEIMPNIPQMSAFWYAVRTAVINAASGRQTVDEALK DAQTNSSSNNNNNNNNNNLGIEGRISEFEFECVYGDTMVETEDGKI |
| | | KIEDLYKRLAMGSGGSMFRTNTNNIKILSPNGFSNFNGIQKVERN LYQHIIFDDDTEIKTSINHPFGKDKILARDVKVGDYLNSKKVLYNEL VNENIFLYDPINVEKESLYITNGVVSHAAEFLSRNNGNGNGTRGVQ VETISPGDGRTFPKRGQTCVVHYTGMLEDGKKFDSSRDRNKPFKF MLGKQEVIRGWEEGVAQMSVGQRAKLTISPDYAYGATGHPGIIPP HATLVFDVELLKLETSYGSRSHHHHHH |
| 11 | aaM1^{N} | CVYGDTMVETEDGKIKIEDLYKRLT |
| 12 | aa M3^{N} | CVSGDTMVETEDGKIKIEDLYKRLA |
| 13 | aa M1^{C} | MFRTNTNNIKILSPNGFSNFDGIQKVERNQYQHIIFDDDTEIKTSINH PFGKDKILARDVKVGDYLNSKKVLYNELVNENIFLYDPINVEKESL YITNGVVSHN |
| 14 | aa M2^{C} | MFRTNTNNIKILGPNGFSNFIGIQKVERDQYQHIIFDDDTEIKTSINHP FGKDKILARDVKVGDYLNSKKVLYNELVNENIFLYDPINVEKESLY ITNGVVSHN |
| 15 | aa M3^{C} | MFRTNTNNIKILSPNGFSNFNGIQKVERNLYQHIIFDDDTEIKTSINH PFGKDKILARDVKVGDYLNGKKVLYNELVNENIFLYDPINVEKESL YITNGVVSHN |
| 16 | aa M4^{C} | MFRTNTNNIKILSPNGFSNFIGIQKVERNQYQHIIFDDDTEIKTSINHP FGKDKILARDVKVGDYLNSKKVLYNELVNENIFLYDPINVEKESLY ITNGVVSHN |
| 17 | aa M5^{C} | MFRTNTNNIKILSPNGFSNFDGIQKVERNLYQHIIFDDDTEIKTSINH PFGKDKILARDVKVGDYLNSKKVLYNELVNENIFLYDPINVEKESL YITNGVVSHN |
| 18 | aa M6^{C} | MFRTNTNNIKILSPNGFSNFNGIQKVERNQYQHIIFDDDTEIKTSINH PFGKDKILARDVKVGDYLNSKKVLYNELVNENIFLYDPINVEKESL YITNGVVSHN |
| 19 | DNA M1^{C} | ATGTTTCGTACCAACACCAACAACATTAAAATTCTGAGCCCGAA CGGCTTTAGTAACTTTGACGGCATTCAGAAAGTGGAACGTAACC AGTATCAGCATATTATTTTTGATGATGATACCGAAATTAAAACC AGCATTAACCATCCGTTTGGCAAAGATAAAATTCTGGCGCGTGA TGTGAAAGTGGGCGATTATCTGAACAGCAAAAAAGTGCTGTATA ACGAACTGGTGAACGAAAACATTTTTCTGTATGATCCGATTAAC GTGGAAAAAGAAAGCCTGTATATTACCAACGGCGTGGTGAGCC ATAAC |
| 20 | DNA M2^{C} | ATGTTTCGTACCAACACCAACAACATTAAAATTCTGGGCCCGAA CGGCTTTAGCAACTTTATCGGCATTCAGAAAGTGGAACGTGACC AGTATCAGCATATTATTTTTGATGATGATACCGAAATTAAAACC AGCATTAACCATCCGTTTGGCAAAGATAAAATTCTGGCGCGTGA TGTGAAAGTGGGCGATTATCTGAACAGCAAAAAAGTGCTGTATA ACGAACTGGTGAACGAAAACATTTTTCTGTATGATCCGATTAAC GTGGAAAAAGAAAGCCTGTATATTACCAACGGCGTGGTGAGCC ATAAC |
| 21 | DNA M3^{C} | ATGTTTCGTACCAACACCAACAACATTAAAATTCTGAGCCCGAA CGGCTTTAGCAACTTTAACGGCATTCAGAAAGTGGAACGTAACC TGTATCAGCATATTATTTTTGATGATGATACCGAAATTAAAACC AGCATTAACCATCCGTTTGGCAAAGATAAAATTCTGGCGCGTGA TGTGAAAGTGGGCGATTATCTGAACGGCAAAAAAGTGCTGTATA ACGAACTGGTGAACGAAAACATTTTTCTGTATGATCCGATTAAC GTGGAAAAAGAAAGCCTGTATATTACCAACGGCGTGGTGAGCC ATAAC |
| 22 | DNA M4^{C} | ATGTTTCGTACCAACACCAACAACATTAAAATTCTGAGCCCGAA CGGCTTTAGCAACTTTATCGGCATTCAGAAAGTGGAACGTAACC AGTATCAGCATATTATTTTTGATGATGATACCGAAATTAAAACC AGCATTAACCATCCGTTTGGCAAAGATAAAATTCTGGCGCGTGA TGTGAAAGTGGGCGATTATCTGAACAGCAAAAAAGTGCTGTATA ACGAACTGGTGAACGAAAACATTTTTCTGTATGATCCGATTAAC GTGGAAAAGGAAAGCCTGTATATTACCAACGGCGTGGTGAGCC ATAAC |
| 23 | DNA M5^{C} | ATGTTTCGTACCAACACCAACAACATTAAAATTCTGAGCCCGAA CGGCTTTAGCAACTTTGACGGCATTCAGAAAGTGGAACGTAACC TGTATCAGCATATTATTTTTGATGATGATACCGAAATTAAAACC AGCATTAACCATCCGTTTGGCAAAGATAAAATTCTGGCGCGTGA TGTGAAAGTGGGCGATTATCTGAACAGCAAAAAAGTGCTGTATA ACGAACTGGTGAACGAAAACATTTTTCTGTATGATCCGATTAAC GTGGAAAAAGAAAGCCTGTATATTACCAACGGCGTGGTGAGCC ATAAC |
| 24 | Int^{N} WTAceL-TerL-11 flanking sequence | qtefe |
| 25 | Int^{C} WTAceL-TerL-11 flanking sequence | ceflg |
| 26 | GS033_TerA-6intein | SISQESYINIEVNGKVETIKIGDLYKKLSFNERKFNEMKLPESVVKN NINLKIETPYGFENFYGVNKIKKDKYIHLEFTNGEKLKCSLDHPLSTI DGIVKAKDLDKYTEVYTKFGGCFLKKSKVINESIELYDIVNSGLKH LYYSNNIISHN |
| 27 | Wild type Int^{C} of GS033_TerA-6 | MKLPESVVKNNINLKIETPYGFENFYGVNKIKKDKYIHLEFTNGEK LKCSLDHPLSTIDGIVKAKDLDKYTEVYTKFGGCFLKKSKVINESIE LYDIVNSGLKHLYYSNNIISHN |
| 28 | Wild type Int^{N} of GS033_TerA-6 | SISQESYINIEVNGKVETIKIGDLYKKLSFNERKFNE |
| 29 | Int^{N} of GS033_TerA-6 with S1C substitution | CISQESYINIEVNGKVETIKIGDLYKKLSFNERKFNE |
| 30 | Int^{N} of GS033_TerA-6 (minus 9aa) | SISQESYINIEVNGKVETIKIGDLYKKL |
| 31 | Int^{N} of GS033_TerA-6 (minus 9aa synthetic) | SISQESYINIEVNGKVETIKIGDLYKKL |
| 32 | Int^{N} of GS033_TerA-6 (minus 3aa synthetic) | SISQESYINIEVNGKVETIKIGDLYKKLSFNERK |
| 33 | Int^{N}AceL-TerL-11 with C1S and Y3S substitutions (synthetic) | SVSGDTMVETEDGKIKIPDLYKRLA |
| 34 | Int^{N} GS033_TerA-6 flanking sequence | kvefe |
| 35 | Int^{C} GS033_TerA-6 flanking sequence | ceflg |
| 36 | DNA Int^{N} GS033_TerA-6 | AAGGTTGAGTTTGAGTCAATTTCTCAAGAATCTTACATAAATAT CGAAGTTAATGGTAAGGTCGAAACAATTAAAATTGGCGATTTAT ATAAAAAACTTTCATTTAACGAAAGAAAATTTAATGAGTGA |
| 37 | DNA Int^{C} GS033_TerA-6 | ATGAAATTACCAGAATCTGTAGTAAAAACAATATCAACTTAAA AATAGAAACTCCATATGGATTTGAGAATTTTTATGGAGTAAATA AAATAAAGAAGGATAAGTATATACATTTAGAATTTACCAATGGT GAAAAACTAAAGTGCTCTTTAGATCATCCATTATCAACAATTGA TGGAATTGTAAAAGCAAAAGATTTAGACAAATATACAGAAGTA TATACAAAATTTGGTGGATGCTTTCTAAAAAAATCAAAAGTTAT TAATGAATCAATAGAATTATATGATATTGTAAACTCGGGACTAA AGCATTTATATTATTCAAATAATATAATATCTCACAACTGCGAA TTCTTAGGG |
| 38 | Int^{N} TerA-1_CP21-BP | CCLENTRVQVRNKYTNKIETLTIKELYARLQELKKS |
| 39 | Int^{C} TerA-1_CP21-BP | MSEIQDINPYEILTPQGFKPFVDIIKSIQTTGITITLEDSREISVTLDHK FKHLNDYKEAKYFKVGDKLQCSKIIKIENIEGEFYEPLEVQDHEYIA NDFINHN |
| 40 | Int^{N} TerA-1_CP21-BP flankinrt sequence | frgfs |
| 41 | Int^{C} TerA-1_CP21-BP flanking sequence | cniiv |
| 42 | DNA Int^{N} TerA-1_CP21-BP | TTTAGGGGATTCAGTTGCTGTCTCGAAAATACTCGAGTGCAGGT AAGAAATAAATATACTAATAAAATAGAAACGCTTACCATAAAG GAATTGTATGCTAGGTTACAAGAACTCAAAAAATCTTAA |
| 43 | DNA Int^{C} TerA-1_CP21-BP | GTGAGTGAAATCCAAGATATAAATCCATATGAAATATTAACACC ACAAGGATTTAAACCTTTTGTTGATATCATTAAATCAATTCAAA CAACTGGCATAACAATAACTTTAGAGGATTCAAGAGAGATATCA GTTACATTAGATCACAAATTTAAACACTTAAATGATTATAAAGA AGCCAAATATTTTAAAGTAGGTGATAAATTACAGTGTTCAAAAA TTATTAAAATTGAAAATATTGAAGGTGAATTTTATGAACCTTTA GAAGTTCAAGATCACGAGTATATAGCCAACGACTTTATAAATCA TAATTGTAATATAATCGTT |
| 44 | Int^{N} CP81-BP_TerA | CVAGDTKITVRNKKTGVIEDITMEELYNRIG |
| 45 | Int^{C} CP81-BP_TerA | MYEVLTPNGFSDFDDISREKKDVYKVITEDDFIKVTKGHKFETPNG FKQLKHLKINDLIKYKNKFSKIVLIDYVGVEYVYDLINVHKNNEYY TNNFVSHN |
| 46 | Int^{N}CP81-BP_TerA flanking sequence | ifide |
| 47 | Int^{c} CP81-BP_TerA flanking sequence | cafid |
| 48 | DNA Int^{N} CP81-BP_TerA | ATTTTTATTGATGAATGTGTAGCTGGTGACACAAAAATTACAGT |
| | | TAGAAATAAGAAAACAGGTGTCATTGAAGATATAACAATGGAA GAGTTATATAACAGAATAGGATAA |
| 49 | DNA Int^{C}CP81-BP_TerA | ATGTATGAAGTACTAACACCAAATGGATTTAGTGATTTTGATGA TATATCAAGAGAAAAAAAAGATGTATATAAAGTAATAACAGAA GATGATTTTATAAAAGTAACAAAAGGTCATAAATTTGAAACACC TAATGGTTTTAAACAATTAAAACATCTTAAAATTAATGATTTAA TAAAATATAAAAATAAATTTTCAAAAATTGTTTTAATAGATTAT GTTGGAGTAGAATATGTATATGATTTAATTAATGTACATAAAAA TAACGAGTATTATACAAATAATTTTGTTTCACACAATTGTGCGTT TATAGAT |
| 50 | Int^{N} AceL-1_ClpC-1 | CFSKKTSIKLRNKKTGDLEEIDISDLIYELHIS |
| 51 | Int^{C} AceL-1_ClpC-1 | MIKLYNKKQNKKFTKSYDLGDYQILTDSGYIGLVSLHETIPYEVWK LKLSNGYELECADDHIIFDNEMNEIFVKNLELGDRVKVDDGYAVVI ELVNTGLLESMYDFELVEDSNRRYYTNGILSHN |
| 52 | Int^{N} AceL-1_ClpC-1 flanking sequence | sgvgk |
| 53 | Int^{C} AceL-1_ClpC-lflanking sequence | telak |
| 54 | DNA Int^{N} AceL-1-ClpC-1 | AGCGGGGTTGGTAAATGTTTTTCTAAAAAAACATCAATAAAATT AAGGAATAAAAAAACTGGTGATTTAGAAGAAATTGATATTTCTG ATCTAATATATGAACTACACATTAGCTAA |
| 55 | DNA Int^{C} AceL-1_ClpC-1 | ATGATAAAATTATATAATAAAAAACAAAATAAAAAATTCACCA AATCTTATGATTTGGGTGATTACCAAATACTAACTGATAGTGGA TATATTGGTTTGGTCTCATTACATGAGACAATACCATATGAAGTT TGGAAATTGAAATTATCTAATGGATATGAATTAGAGTGTGCTGA TGATCATATTATTTTTGATAATGAAATGAATGAGATATTTGTAA AGAATCTAGAATTAGGAGACAGAGTAAAAGTAGATGATGGATA TGCTGTTGTTATAGAATTAGTAAATACTGGTCTATTAGAAAGTA TGTATGATTTTGAGTTAGTAGAAGATTCAAATAGAAGGTATTAT ACAAATGGTATTTTATCACACAACACAGAACTGGCTAAA |
| 56 | Int^{N} AceL-1_ClpC-3 | CVSPNTKIKIRNSSTGEISEVTIAEFNKMI |
| 57 | Int^{C} AceL-1_ClpC-2 | MKKIVKSVSVEGFEVLSDNGWVPIKNVHTTVPYELYNLRTANGLR LECADNHIVFTSKLKEVYVKDLNVDDKIMTEDGVSLVSSIEKTKAK VTMYDLEVDSEDHRYYTDGILSHN |
| 58 | Int^{N} AceL-1_ClpC-2 flanking sequence | agvgk |
| 59 | Int^{C}AceL-1_ClpC-2 flanking sequence | tslie |
| 60 | DNA Int^{N} AceL-1_ClpC-2 | GCAGGAGTAGGTAAATGCGTTAGTCCTAATACGAAGATTAAGAT TAGGAACAGTAGCACTGGAGAAATTTCAGAAGTTACGATAGCG GAATTCAATAAGATGATTTAA |
| 61 | DNA Int^{C} AceL-1_ClpC-2 | ATGAAAAAATTGTTAAGAGTGTAAGTGTAGAAGGATTTGAGG TACTCTCTGATAATGGATGGGTACCAATTAAAAATGTACATACC ACTGTACCCTACGAACTCTATAACCTCCGTACAGCCAACGGTTT GCGGTTAGAATGTGCAGACAATCATATCGTGTTTACTTCTAAGC TAAAAGAGGTATATGTTAAAGACTTAAATGTTGACGATAAGATT ATGACTGAGGATGGAGTATCTTTAGTATCATCAATTGAAAAGAC TAAAGCTAAAGTAACGATGTATGATCTTGAAGTAGATAGTGAAG ATCATCGTTACTATACTGACGGTATTCTTTCACATAACACTTCTC TAATAGAA |
| 62 | Int^{N} AceL-1_RadAl-1 | CVHPNTLVKIKIDSTGEERTITVKDLHELIKSVK |
| 63 | Int^{C} AceL-1_RadA1-1 | MKRKFIESISADNISIMTDTGWEKVKGSHVTIEYKVFNLVTDRLSLQ CADDHIVFKEDFSEVFVKDLEVGDLIQTVNGLESVTEVYETDDLVN MHDLEIDSKNHRYYTDGILSHN |
| 64 | Int^{N} AceL-1_RadA1-1 flanking sequence | pgvgk |
| 65 | Int^{C} AceL-1_RadA1-1 flanking sequence | ttlll |
| 66 | DNA Int^{N} AceL-1_RadA1-1 | CCAGGAGTTGGTAAATGCGTCCATCCAAATACATTGGTAAAAT CAAAATTGATTCTACTGGTGAGGAGCGTACTATTACAGTCAAAG ACCTCCACGAACTAATTAAATCTGTAAAATGA |
| 67 | DNA Int^{C} AceL-LRadA1-1 | ATGAAACGTAAATTTATAGAAAGTATTTCTGCAGACAATATCAG CATCATGACAGATACTGGTTGGGAAAAAGTTAAAGGTAGTCAC GTTACAATTGAGTATAAAGTATTCAACCTTGTCACTGACAGGTT ATCACTACAATGTGCAGATGATCATATCGTTTTTAAAGAGGACT TCTCAGAGGTCTTTGTAAAGGACCTTGAGGTTGGTGATTTAATA CAAACAGTAAACGGTTTAGAATCAGTTACTGAAGTATATGAAAC AGACGACTTGGTAAATATGCACGATTTAGAAATTGATTCTAAAA |
| | | ACCATAGGTATTATACTGATGGAATTCTTTCACATAATACTACAT TATTATTG |
| 68 | Int^{N} AceL-1_TerL-10 | CVSGDTKVTLKDNDTGKIINVNIEEMVSVSSLDV |
| 69 | Int^{C} AceL-1_TerL-10 | MEVGKMSKSYKVLSPSGFVDFAGIQKITRSKYRHFIFDDGTEIKCSL NHRFGEEEIVASTLHHGTELQGKKILYAEDVEDDIDLYDLLNVANG NLYYTNGLVSHN |
| 70 | Int^{N} AceL-1_TerL-10 flanking sequence | ntefe |
| 71 | Int^{C} AceL-1_TerL-10 flanking sequence | ceflg |
| 72 | DNA Int^{N} AceL-1-TerL-10 | AACACGGAGTTTGAGTGTGTTTCTGGTGATACAAAGGTTACTCT CAAAGACAATGATACAGGAAAGATTATTAATGTAAATATTGAA GAAATGGTGAGTGTGAGTTCTTTGGATGTATAA |
| 73 | DNA Int^{C} AceL-1_TerL-10 | ATGGAAGTTGGAAAGATGTCTAAAAGTTATAAAGTGTTATCACC ATCAGGGTTTGTGGATTTTGCTGGTATTCAAAAAATAACACGCA GCAAATATCGACATTTTATTTTTGATGATGGCACAGAAATCAAA TGTTCGTTAAATCATAGATTTGGTGAAGAGGAAATAGTAGCCTC AACACTCCATCACGGCACAGAGCTTCAGGGTAAAAAAATACTGT ATGCAGAAGATGTTGAGGATGATATTGATTTATATGATTTGTTA AATGTTGCCAATGGAAATCTTTACTACACCAACGGATTAGTATC ACACAATTGTGAGTTCCTTGGC |
| 74 | Int^{N} AceL-1_TerL-3 | CFFFNTIISVETNNQQYETRIGILYYSMVSKERNLTILEKIKIKLYDLL FILEKH |
| 75 | int^{C} AceL-1_TerL-2 | MLRIFKRCLIYLIKKMIEFIELYEYKKISLDECDINKKILNSISLMDLK VETDTGYETSSNIHITQPFKHYNIETVDGYEIICADNHILFDEEFNEV FTKDLKIGDLIKTKNGNSVIKSIYIDTHKSSMFDLTIDHPNHRFYTNG ILSHN |
| 76 | Int^{N}AceL-1_TerL-2 flanking sequence | rqvgk |
| 77 | Int^{C}AceL-1_TerL-2flanking sequence | tisss |
| 78 | DNA Int^{N} AceL-1_TerL-2 | AGGCAAGTTGGAAAATGTTTTTTTTCAATACAATTATATCCGTT GAAACCAATAATCAGCAATATGAAACTAGAATAGGAATTCTTTA TTATTCAATGGTTTCAAAGGAAAGAAATTTAACTATTTTAGAGA AAATTAAAATAAAATTATATGATTTATTATTCATTTTAGAAAAA CATTAA |
| 79 | DNA Int^{C} AceL-1_TerL-2 | ATGCTTAGAATTTTTAAAAGGTGTTTAATTTATTTAATTAAAAAA ATGATTGAATTTATTGAATTATATGAATATAAAAAAATCTCATT AGATGAGTGTGATATAAATAAAAAAATATTAAACTCAATATCTC TTATGGATTTAAAGGTAGAGACTGATACAGGATATGAAACATCA TCTAATATACACATAACACAACCATTTAAACACTATAATATTGA AACGGTTGATGGTTATGAAATAATATGTGCTGATAATCATATAT TATTTGATGAGGAATTCAATGAAGTATTTACAAAGGATTTAAAA ATAGGAGATTTAATTAAAACAAAAAATGGCAACAGTGTTATCA AGAGTATTTATATAGACACACATAAGTCATCCATGTTTGACCTA ACAATAGACCATCCAAACCACAGGTTCTATACAAATGGTATACT TTCACATAATACGATATCATCTTCT |
| 80 | Int^{N} AceL-1_TerL-3 | CVDGNTIVETEDGKIKIEDLYKKT |
| 81 | Int^{C} AceL-1_TerL-3 | MFITNTDNIKILSPSGFSNFNGIQKVERNLYQHIIFDDESEIKTSINHPF GKNKILARNVKVGDYLSSKKVLYNELVNEKIFLYDPINVEKENLYI TNGVVSHN |
| 82 | Int^{N} AceL-1_TerL-3 flanking sequence | qqefe |
| 83 | Int^{C} AceL-1_TerL-3 flanking sequence | ceflg |
| 84 | DNA Int^{N} AceL-1_TerL-3 | CAACAAGAGTTTGAATGTGTTGACGGTAATACGATAGTCGAAAC GGAAGATGGTAAAATAAAAATAGAAGATTTATATAAAAAATTG TGA |
| 85 | DNA Int^{C} AceL-1_TerL-3 | ATGTTTATAACTAATACAGATAATATAAAAATATTAAGTCCAAG TGGATTTTCTAATTTTAATGGTATTCAAAAGGTTGAAAGAAACC TTTATCAACACATTATCTTTGATGATGAATCTGAAATAAAAACTT CTATTAACCACCCTTTTGGTAAAAATAAAATATTAGCAAGAAAT GTAAAAGTAGGAGATTATTTAAGTAGTAAAAAAGTATTATATAA TGAGTTGGTTAATGAAAAAATATTTTTATATGACCCTATAAATG TAGAAAAAGAAAACTTATATATTACTAACGGTGTTGTTTCTCAT AATTGTGAGTTTTTAGGT |
| 86 | Int^{N} AceL-1_TerL-4 | CVDGNTIVETEDGKIKIEDLYKKM |
| 87 | Int^{C} AceL-1_TerL-4 | MFRTNTDNIKILSPSGFSIFNGIQKVERDLYQHIIFDDKSEIKTSINHPF GKDKILARNIKVGDYLNSKKVLYNELVAEKITLYDPINVEKENLYIT NGVISHN |
| 88 | Int^{N} AceL-1_TerL4 flanking sequence | qqefe |
| 89 | Int^{C} AceL-1_TerL-4 flanking sequence | ceflg |
| 90 | DNA Int^{N} AceL-1_TerL-4 | CAACAAGAGTTTGAGTGTGTGGATGGAAATACGATAGTCGAAA CGGAAGATGGCAAAATAAAAATAGAAGATTTATATAAAAAAAT GTGA |
| 91 | DNA Int^{C} AceL-1_TerL-4 | ATGTTTAGAACAAATACAGATAATATAAAAATTTTAAGTCCAAG TGGGTTTTCTATTTTTAATGGCATTCAAAAGGTTGAAAGAGACC TCTATCAACATATTATCTTTGATGATAAATCTGAAATAAAGACTT CTATCAACCACCCCTTTGGTAAAGATAAAATATTAGCGAGAAAT ATAAAGGTTGGTGATTATTTAAATAGTAAGAAAGTTTTATATAA TGAGTTGGTCGCCGAAAAGATTACTTTATATGATCCTATAAATG TAGAAAAAGAAAATTTATATATCACTAACGGTGTTATTTCTCAT AATTGTGAGTTTTTAGGT |
| 92 | Int^{N} AceL-1_TerL-5 | CVDGNTIVETEDGKIKIEDLYKKT |
| 93 | Int^{C} AceL-1_TerL-5 | MFRTNTDNIKILSPSGFSNFNGIQKVERDLYQHIIFDDKSEIKTSINHP FGKDKILARNIKVGDYLNSKKVLYNELVNEKITLYDPINVEKENLYI TNGVISHN |
| 94 | Int^{N} AceL-1_TerL5 flanking sequence | qqefe |
| 95 | Int^{C} AceL-1_TerL-5 flanking sequence | ceflg |
| 96 | DNA Int^{N} AceL-1_TerL-5 | CAACAAGAGTTTGAATGTGTTGACGGTAATACGATAGTTGAAAC GGAAGATGGTAAAATAAAAATAGAAGATTTATATAAAAAATTA TAG |
| 97 | DNA Int^{C} AceL-1_TerL-5 | ATGTTTAGAACCAATACAGATAATATAAAAATATTAAGTCCAAG TGGATTTTCTAATTTTAACGGCATTCAAAAGGTTGAAAGAGACC TCTATCAACATATTATCTTTGATGATAAGTCTGAAATAAAAACTT CTATTAACCACCCTTTTGGTAAAGATAAAATATTAGCGAGAAAT ATAAAAGTAGGAGATTATTTAAATAGTAAGAAGGTTTTATATAA TGAGTTGGTTAATGAAAAAATTACTTTATATGACCCTATAAATG TAGAAAAAGAAAACTTATATATTACTAACGGTGTTATTTCTCAT AATTGTGAGTTTTTAGGT |
| 98 | Int^{N} AceL-1_UvsW-3-1 | CRTYDSTMDIDVGNSDFAEYLLNNSKK |
| 99 | Int^{C} AceL-1_UvsW-3-1 | MKFNIPIGELAESIAKYKGVLLNDNCEINIKDLDCKVNTPSGTATINI IIKKEKLEGIKLLLANGVEIKCANKHILRYNNADVFADSLAIGDSVE TINGNVKVSSINNIDDTTFYDIGIDAPYLYYDADGVLHHN |
| 100 | Int^{N} AceL-1_UvsW-3-1 flanking sequence | tgagk |
| 101 | Int^{C} AceL-1_UvsW-3-1 flanking sequence | titta |
| 102 | DNA Int^{N} AceL-1_UvsW-3-1 | ACTGGTGCAGGCAAATGTCGAACTTATGATTCTACAATGGATAT AGATGTAGGTAATTCTGATTTTGCTGAATATTTGCTAAATAATA GTAAGAAATAG |
| 103 | DNA Int^{C} AceL-1_UvsW-3-1 | ATGAAATTTAACATACCAATAGGGGAACTAGCAGAGTCGATCG CGAAGTACAAAGGAGTACTATTAAACGATAACTGCGAAATTAA TATTAAAGATCTTGATTGTAAAGTTAATACACCATCAGGAACTG CTACTATTAATATTATAATTAAAAAAGAAAAGTTAGAAGGCATA AAACTATTACTTGCAAATGGTGTAGAAATAAAGTGTGCTAATAA GCATATATTAAGATATAATAATGCAGACGTATTTGCAGATTCAT TAGCAATTGGCGACTCGGTAGAAACTATTAACGGAATGTTAAG GTTAGTAGTATTAACAATATTGACGATACTACATTTTACGATATC GGAATAGATGCACCGTACTTATATTATGATGCAGACGGAGTATT ACATCATAATACAATTACCACAGCA |
| 104 | Int^{N} AceL-1_gp41-1 | CFFSDGEINTRNISNKEIKSIKIGKIFTNISKGHTNI |
| 105 | Int^{C} AceL-1_gp41-1 | MLDNYEIIEADSLLEGKYDRPLYDKFIEAYEVDNLEVDTPNGWIKIE GIGKTIEFYEWEIQTSGGKHLICADKHLLYRCDNMNFYNKKCDITEI YCQDLNIGDFIMTKDGPEMLMDIYKNGNKSNMYDLQLSEGSNKQ YYTNDILSHN |
| 106 | Int^{N} AceL-1_gp41-1flanking sequence | slwmq |
| 107 | Int^{C} AceL-1_gp41-1flanking sequence | tnggk |
| 108 | DNA Int^{N} AceL-1_gp41-1 | ACAAACGGAGGAAAATGTTTTTTAGTGATGGTGAGATAAATAC |
| | | TAGGAATATAAGCAATAAGGAAATAAAATCAATTAAAATAGGT AAAATTTTACCAACATTAGCAAGGACATACTAACATTTAA |
| 109 | DNA Int^{C} AceL-1_gp41-1 | ATGTTAGATAATTATGAAATAATAGAAGCAGATTCTCTATTAGA AGGAAAATATGATAGACCACTATATGATAAATTTATTGAAGCTT ATGAAGTAGACAACTTAGAGGTTGATACACCAAATGGTTGGATA AAGATAGAAGGAATTGGTAAAACTATTGAATTTTATGAATGGGA AATACAAACATCTGGTGGAAAACATCTAATATGTGCAGATAAAC ATCTATTATATAGGTGTGATAATATGAATTTTTATAATAAAAAA TGTGACATAACAGAAATATACTGCCAAGATTTGAATATAGGTGA TTTTATAATGACTAAGGATGGTCCTGAGATGTTGATGGATATTT ATAAAAATGGTAATAAATCGAATATGTATGATTTACAATTATCA GAAGGCTCTAATAAACAATACTACACAAATGATATACTTAGTCA TAATTCACTTTGGATGCAA |
| 110 | Int^{N} AceL-1_gp46-1 | CVDESTLIDVQIIDFEPNLENLEFLDKTDEGKRIFLYIKKSNKSLYEKI EKFRKGQ |
| 111 | Int^{C} AceL-1_gp46-1 | MLTLKIGDLYELSKNINILESDIRVSTPGGLKKVFAVDITAKNSDVFS IKVNKHELLCSPDHLIRSEDMWVKSKDLKINSVIDTKYGKLTVKEIS ILDIKSDLMDLHVDGSEYYTNDIISHN |
| 112 | Int^{N} AceL-1_gp46-1 flanking sequence | ngsgk |
| 113 | Int^{C} AceL-1_gp46-1 flanking sequence | sslld |
| 114 | DNA Int^{N} AceL -1_gp46-1 | AATGGTTCTGGTAAGTGTGTTGATGAGTCAACACTAATAGATGT ACAAATAATTGATTTTGAGCCTAATTTAGAAAATTTAGAATTTTT AGACAAAACGGATGAGGGAAAGAGGATTTTTCTATATATAAAG AAATCTAATAAATCCTTGTATGAAAAAATTGAAAAATTTAGAAA AGGTCAATAA |
| 115 | DNA Int^{C} AceL-1_gp46-1 | ATGTTAACATTAAAGATAGGTGATTTATATGAATTATCAAAAAA TATAAATATTTTAGAATCAGACATCCGTGTATCTACCCCAGGTG GATTGAAAAAGGTTTTTGCTGTTGATATAACAGCAAAAAATAGC GATGTGTTTTCTATAAAAGTTAATAAACATGAACTACTTTGCTCA CCAGATCATCTAATAAGATCAGAAGATATGTGGGTTAAATCTAA AGATTTAAAAATAAATTCCGTAATAGATACAAAATATGGCAAAC TTACTGTTAAGGAGATATCAATTTTGGATATAAAGAGTGATTTG ATGGATTTACATGTGGATGGTAGTGAATATTACACTAATGATAT AATTAGTCACAACTCATCACTATTAGAT |
| 116 | Int^{N} VidaL_T4Lh-1 | CVHPDTKVTIRRKLC |
| 117 | Int^{C} VidaL_T4Lh-1 | MKELLDLYTEKEINKLLERYTIDQIIDYSQPHVVSVGSIKEEMDSGN FIFVDSPDGYVAVSDFVDKGNFEEYRFTYDKKIIRTNEGHLFQTHLG WETSKNLYKMYLAGHPIYILHKNGGYKKIDIEKTGNVIPIVDIVVEH KNHRYYTDGLSSHN |
| 118 | Int^{N} VidaL_T4Lh-1 flanking sequence | vflas |
| 119 | Int^{C} VidaL_T4Lh-1 flaking sequence | tnvgk |
| 120 | DNA Int^{N} VidaL_T4Lh-1 | GTATTTTTGGCTAGTTGTGTGCATCCAGATACAAAAGTAACAAT TCGTAGAAAACTTTGTTAG |
| 121 | DNA Int^{C} VidaL_T4Lh-1 | ATGAAAGAATTGCTTGACTTATACACAGAAAAAGAAATAAATA AATTATTAGAAAGATACACAATAGACCAGATTATAGACTACTCA CAACCTCATGTGGTTTCTGTGGGTAGTATAAAAGAAGAAATGGA TTCAGGAAATTTCATTTTTGTTGACAGCCCAGATGGTTACGTTGC TGTTAGTGATTTTGTAGACAAAGGAAACTTTGAAGAATATAGGT TTACATATGATAAAAAAATAATCCGAACAAACGAAGGTCACTTA TTCCAAACACATTTGGGTTGGGAGACTTCTAAGAATTTATATAA AATGTACTTAGCTGGTCACCCCATTTATATATTGCATAAAAATG GTGGTTATAAAAAGATTGATATAGAAAAGACCGGAAACGTGAT TCCTATCGTTGATATTGTGGTGGAACACAAAAACCATAGATATT ATACGGATGGATTGTCCAGCCATAACACAAATGTGGGCAAG |
| 122 | Int^{C} VidaL_UvsX-2 | CLPKEAVVQIRLTKKG |
| 123 | In^{C} VidaL_UvsX-2 | MIEEKKVTVQELRELYLSGEYTIEIDTPDGYQTIGKWFDKGVLSMV RVATATYETVCAFNHMIQLADNTWVQACELDVGVDIQTAAGIQPV MLVEDTSDAECYDFEVMHPNHRYYGDGIVSHN |
| 124 | Int^{N} VidaL_UvsX-2 flanking sequence | sgksy |
| 125 | Int^{C} VidaL_UvsX-2 flanking sequence | agesg |
| 126 | DNA Int^{N} VidaL_UvsX-2 | GCTGGCGAAAGTGGCTGTTTGCCAAAAGAAGCAGTAGTACAGA TTCGATTAACAAAAAAAGGCTAG |
| 127 | DNA Int^{C} VidaL_UvsX-2 | ATGATTGAAGAAAAGAAAGTAACAGTACAAGAGCTTAGAGAGC TATATCTCAGCGGCGAGTATACTATTGAGATTGACACACCGGAC GGATATCAGACTATCGGAAAATGGTTTGACAAAGGGGTATTGTC CATGGTTAGAGTTGCCACAGCCACTTACGAAACAGTGTGTGCAT TTAATCATATGATTCAACTGGCTGACAATACGTGGGTACAAGCC TGTGAGTTAGATGTAGGAGTAGATATACAAACGGCGGCAGGCA TCCAGCCTGTTATGTTAGTCGAAGATACAAGTGATGCAGAGTGT TACGATTTTGAAGTCATGCATCCGAATCATAGATATTACGGTGA CGGAATTGTAAGCCATAACTCGGGGAAAAGTTAT |
| 128 | Int^{N} VidaL_TerL-6-1 | SLAHETIVSINDNNTLTSMCIGDLYDYM |
| 129 | Int^{C} VidaL_TerL-6-1 | MDYHSNQVSRIFGVGMSKVHLGFKKNTKNLKVLTPNGHEEFYGIN KIRVDEYIRIKFKEHKEIRCSIDHPFIQENDLPIKAKHIDKSKHIKCID GFTTLEYSHVVNKQIELYDIVNSGSEYIYFSNGILSHN |
| 130 | Int^{N} VidaL_TerL-6-1 2 flanking sequence | sveye |
| 131 | Int^{C} VidaL_TerL-6-1 2 flanking sequence | ckfmg |
| 132 | DNA Int^{N} VidaL_TerL-6-1 | TCCGTGGAATATGAGAGTTTGGCACACGAAACTATAGTAAGTAT AAATGATAATAACACACTAACAAGTATGTGCATTGGAGATTTAT ATGACTATATGTAA |
| 133 | DNA Int^{C} VidaL_TerL-6-1 | TTGGATTACCACTCTAATCAAGTGTCTCGAATTTTGGAGTTGGT ATGAGCAAAGTACATCTAGGGTTTAAAAAGAACACTAAAAATTT AAAAGTGTTAACACCAAATGGACACGAAGAATTCTACGGAATA AACAAAATACGTGTCGATGAATATATACGAATAAAATTCAAAG AACATAAAGAAATACGTTGCTCGATTGACCACCCGTTTATACAA GAAAATGATTTACCAATAAAAGCAAAACATATTGATAAAAGCA AACATATAAAATGTATTGATGGATTTACTACTTTAGAGTATTCG CATGTTGTTAATAAACAAATTGAACTATATGATATTGTAAACTC TGGTAGTGAGTACATATATTTTTCTAATGGGATATTAAGTCACA ACTGTAAATTCATGGGT |
| 134 | Int^{N} TerL-7-1_VidaL | CLWGASTVNVFDSLTGKNIDIKLEDLYQKL |
| 135 | Int^{C} TerL-7-1_VidaL | MESYTFRKNTRYKIMTPAGYQNFGGIRKLNKNVHYIVELSNKKILK CSTTHPFIYNDREIFANKLKVGSLLDSTSKKKISVISIELDKSKIDLYD IVEVNNGNIFNVDGIVSHN |
| 136 | Int^{N} TerL-7-1_VidaL flanking sequence | sqecd |
| 137 | Int^{C} TerL-7-1_VidaL 2 flanking sequence | c |
| 138 | DNA Int^{N} TerL-7-1_VidaL | TCCCAAGAATGCGATTGTTGTGGGCGCATCTACTGTAAATGT ATTTGATAGTTTAACTGGAAAAAACATTGATATAAAACTCGAAG ATTTGTATCAAAAACTTTAA |
| 139 | DNA Int^{C} TerL-7-1_VidaL | ATGGAATCATATACTTTTAGAAAAACACAAGATATAAAATAAT GACACCAGCAGGATATCAAAACTTTGGTGGTATTAGAAAATTGA ATAAAAATGTACATTATATAGTTGAATTATCCAATAAAAAAATA TTAAAATGTTCAACTACACATCCATTTATTTATAATGATAGAGA GATATTTGCAAATAAATTAAAAGTCGGTAGTTTACTTGATAGTA CTAGTAAAAAGAAAATTTCAGTAATATCAATTGAATTAGATAAA TCAAAAATAGATTTATATGATATAGTAGAAGTAAATAATGGTAA TATTTTTAATGTAGATGGTATTGTTTCACATAATTGT |
| 140 | Int^{N} VidaL_TerL-3 | CVSASTIITLQDTHGNIFDSQIGDLYNTIGK |
| 141 | Int^{C} VidaL_TerL-3 | MSKIFKENTNGYKVLTPAGFQDFAGVSMMGIKPLLRLEFERGAYV ECTYDHKFYIDLETCKPAQDIAVGNTVVTSEGDIKLLNKIELGYSEP VYDLIQVEGGHRYYTNKILSSN |
| 142 | Int^{N} VidaL_TerL-3 flanking sequence | rreyg |
| 143 | Int^{C} VidaL_TerL-3 2 flanking sequence | ceflv |
| 144 | DNA Int^{N} VidaL_TerL-3 | CGTCGTGAGTACGGTTGTGTGAGCGCATCTACAATCATTACTCT CCAAGACACACACGGTAATATATTTGACTCACAAATAGGCGACT TGTACAATACGATAGGTAAATAA |
| 145 | DNA Int^{C} VidaL_TerL-3 | ATGAGCAAGATTTTTAAAGAGAATACTAATGGATATAAGGTGTT AACACCAGCGGGGTTTCAAGACTTTGCTGGTGTTAGCATGATGG GAATAAAACCGTTGCTTCGGCTAGAGTTCGAGCGAGGCGCCTAC GTCGAATGCACCTACGATCATAAATTTTACATAGACCTAGAAAC TTGTAAGCCAGCCCAAGACATTGCAGTAGGAAACACTGTGGTTA CTTCTGAGGGTGATATAAAATTACTCAACAAAATAGAACTGGGT TATTCAGAACCTGTTTATGATCTTATACAAGTTGAAGGCGGCCA CCGATATTACACAAACAAAATACTCAGCTCAAATTGCGAATTTT |
| | | TAGTA |
| 146 | Int^{N} VidaL_TerL-1 | CVOADTKYTIRNKISGDVLNVTAEEFHKMOKK |
| 147 | Int^{C} VidaL_TerL-1 | MKLSNFTNRKFIETIDASEWEVETCEGFKPIISSNKTIEYVVYKIELE NGLSIKCADTHILIDKNLQEIYAKDSFNKIIFTKFGNSKVISVETLNIS ENMYDLSVDSEDHTYYTDDILSHN |
| 148 | Int^{N} VidaL_TerL-1 flanking sequence | rqqgk |
| 149 | Int^{C} VidaL_TerL-1 2 flanking sequence | tttaa |
| 150 | DNA Int^{N} VidaL_TerL-1 | AGACAACAAGGTAAGTGCGTTCAAGCAGACACTAAATACACTA TAAGAAACAAAATTAGTGGTGATGTGTTAAATGTTACAGCAGAA GAATTCCACAAAATGCAGAAAAAATAA |
| 151 | DNA Int^{C} VidaL_TerL-1 | ATGAAGCTATCCAATTTCACCAATAGAAAATTTATAGAAACAAT TGATGCTAGTGAATGGGAAGTAGAAACATGCGAAGGTTTCAAA CCCATCATTAGTTCAAATAAAACTATTGAATATGTAGTCTATAA AATTGAACTAGAAAATGGATTATCTATTAAATGTGCAGACACTC ATATTTTAATAGATAAAAATTTGCAAGAAATTTATGCAAAAGAT AGTTTTAATAAAATAATATTTACAAAGTTCGGAAACTCAAAAGT TATTTCCGTAGAAACTTTAAATATATCTGAAAATATGTATGATCT TTCTGTTGATTCAGAAGATCACACATACTATACAGATGATATCTT ATCACATAATACCACGACCGCCGCA |
| 152 | Int^{N} VidaL_gp46-1 | CLCINTIVKVKNTKTGVIYETTIGELYNGAME |
| 153 | Int^{C} VidaL_gp46-1 | MSTISQTVNRKFVNSFSLVDLEIETDSGWQPVTDIHKTIPYTVWHIE TQSGLTLDCADTHILFDHNYNEIFVKDIIPNQTKIISKHGPELVLTVIE QSQQENMFDLTVDHPDHRFYSNNILSHN |
| 154 | Int^{N} VidaL_gp46-1 flanking sequence | ngtgk |
| 155 | Int^{C} VidaL_gp46-1 flanking sequence | ttvin |
| 156 | DNA Int^{N} VidaL_gp46-1 | AACGGAACGGGCAAGTGCCTTTGTATAAATACTATTGTAAAAGT AAAAAACACCAAAACTGGGGTAATTTACGAAACTACAATAGGA GAATTATACAATGGCGCGATGGAATAA |
| 157 | DNA Int^{C} VidaL_gp46-1 | ATGTCTACAATTTCTCAAACAGTAAATAGAAAATTTGTTAATAG TTTCAGCTTAGTTGATCTTGAAATCGAAACAGATTCTGGATGGC AGCCTGTTACTGACATACACAAGACTATTCCATACACAGTTTGG CATATCGAAACCCAAAGCGGGCTGACTCTTGACTGTGCCGACAC TCATATCTTGTTTGATCACAACTACAACGAGATCTTTGTCAAAG ATATAATACCTAACCAGACTAAGATAATATCTAAGCACGGTCCT GAATTAGTATTAACAGTAATCGAACAGTCTCAGCAAGAAAATAT GTTTGATCTAACAGTTGATCATCCTGATCATCGTTTTTACTCAAA CAATATCTTATCTCACAATACCACAGTGATAAAT |
| 158 | Int^{N} VidaL_gp41-1 | CVIAETEVKIIELYNIDSFIKTGILNSQGVVSWEETSSHGRTR |
| 159 | Int^{C} VidaL_gp41-1 | MNLLDKRVEWLSKWYPVDKLQQLSEDKLVLLYNNSQPKKVRMG SLEGVPTSSYRISSPDGYVTAHAWRNKGTKECVTLTTDSGNSITAST DHFFEMSDGKWKYAGCLFPGQCISTESGTETVTSVVAAGKHTVYD FYIDHENHRYYTNGISSHN |
| 160 | Int^{N} VidaL_gp41-1 flanking sequence | ifagg |
| 161 | Int^{C} VidaL_gp41-1 flanking sequence | sgagk |
| 162 | DNA Int^{N} VidaL_gp41-1 | ATATTTGCCGGCGGATGTGTAATTGCCGAAACTGAGGTAAAAT AATTGAATTATACAACATTGACAGCTTTATTAAAACAGGAATAC TTAACAGCCAAGGGGTCGTTTCGTGGGAAGAAACTTCTTCCCAC GGTCGTACTCGATAG |
| 163 | DNA Int^{C} VidaL_gp41-1 | ATGAATTTACTAGATAAGAGAGTTGAGTGGTTATCTAAGTGGTA CCCAGTAGACAAGTTACAACAATTATCAGAAGACAAGTTGGTAC TCCTATATAACAATAGTCAGCCAAAAAAAGTCCGCATGGGATCG TTGGAAGGTGTTCCGACCAGCAGTTATCGGATCAGCAGCCCCGA CGGGTATGTTACCGCACATGCCTGGCGAAATAAAGGAACTAAA GAGTGTGTTACTCTAACCACAGACTCTGGAAATTCTATAACTGC TAGCACAGATCATTTTTTCGAAATGTCCGACGGCAAGTGGAAAT ATGCAGGCTGTTTGTTTCCAGGACAGTGCATTAGCACAGAATCC GGCACCGAAACAGTGACCAGCGTAGTAGCAGCAGGTAAGCACA CAGTATACGACTTCTACATCGATCATGAAAATCACAGATACTAT ACCAATGGAATAAGCAGTCATAACTCTGGTGCAGGCAAA |
| 164 | Int^{N} GS013_ter-3 | CLGGDTFTFTTDDNGIVQKTSMENLYERL |
| 165 | Int^{C} GS013_ter-3 | MFKINKNIKVKTPDGFKDFSGIQKVYKPFYHWIIFDDGSEIKCSDNH SFGKEKIKASTIKVDDILQEKKVLYNEIVEEGIYLYDLLDVGEDNLY |
| | | YSNNIVSHN |
| 166 | Int^{N} GS013_ter-3 flanking sequence | rvefe |
| 167 | Int^{C} GS013_ter 3 flanking sequence | ceflg |
| 168 | DNA Int^{N} GS013_ter-3 | CGGGTTGAGTTTGAATGTTTGGGTGGTGATACAGAGATTGAAAT TTTGGATGATAATGGAATTGTACAAAAAACTTCTATGGAAAATT TATATGAACGATTGTGA |
| 169 | DNA Int^{C} GS013_ter-3 | ATGTTTAAGATTAATAAAAATATTAAAGTAAAAACACCTGATGG ATTTAAAGATTTTTCAGGAATACAAAAAGTTTATAAACCTTTTTA CCATTGGATAATATTTGATGACGGATCAGAAATAAAATGCTCCG ATAATCATTCTTTCGGAAAAGAAAAAATTAAGGCATCAACAATT AAAGTTGATGATATTTTACAAGAAAAGAAAGTATTATATAATGA AATAGTAGAAGAAGGAATTTATCTTTATGATTTACTTGATGTTG GCGAAGACAaTCTTTACTATTCAAACAATATAGTATCACACAAC TGCGAGTTCTTGGGT |
| 170 | Int^{N} GS013_ter-2 | CLGGDTEIEILDDNGIVQKTSMENLYERL |
| 171 | Int^{C} GS013_ter-2 | MSVGKMFKINKNIKVKTPDGFKDFSGIQKVYKPFYHWIIFDDGSEIK CSDNHSFGKEKIKASTIKVDDILQEKKVLYNEIVEEGIYLYDLLDVG EDNLYYSNNIVSHN |
| 171 | Int^{N} GS013_ter-2 flanking sequence | rvefe |
| 173 | Int^{C} GS013_ter-2 flanking sequence | ceflg |
| 174 | DNA Int^{N} GS013_ter-2 | CGTGTTGAGTTTGAATGTTTGGGTGGTGATACAGAGATTGAAAT TTTGGATGATAATGGAATAGTACAAAAAACTTCTATGGAAAATT TATATGAACGATTGTGA |
| 175 | DNA IInt^{C} GS013_ter-2 | ATGAGTGTTGGAAAAATGTTTAAGATTAATAAAAATATTAAAGT AAAAACACCTGATGGATTTAAAGATTTTTCAGGAATACAAAAAG TTTATAAACCTTTTTACCATTGGATAATATTTGATGACGGATCAG AAATAAAATGCTCCGATAATCATTCTTTCGGAAAAGAAAAAATT AAGGCATCAACAATTAAAGTTGATGATATTTTACAAGAAAAGA AAGTATTATATAATGAAATAGTAGAAGAAGGAATTTATCTTTAT GATTTACTTGATGTTGGCGAAGACAATCTTTACTATTCAAACAA TATAGTATCACACAACTGCGAATTCTTAGGT |
| 176 | Int^{N} GS013_ter-1 | CFNTNTTVRLRNKLTGEIIEVTIGEFYEKIKIKKESNTDLP |
| 177 | Int^{C} GS013_ter-1 | MSKFIEEXXTDEWEVETPSGWQSFSGVGKTIEYEEWEVVTETGKSL ICADKHILLNDKWQEVYCEDCSIDDCIQTKNXAEKILQLKKTSRIXN MYDLLDVDNGNIFYSNEIVSHN |
| 178 | Int^{N} GS013_ter-1 flanking sequence | rqtgk |
| 179 | int^{C} GS013_ter-1 flaking sequence | sttvv |
| 180 | DNA Int^{N} GS013_ter-1 | CGTCAGACGGGTAAATGTTTTAATACAAATACAACGGTAAGGTT AAGGAATAAACTTACTGGAGAAATTATTGAAGTGACTATTGGAG AATTTTATGAAAAAATCAAGAAAGAAAGTAATACTGATTTGCCT TGA |
| 181 | DNA Int^{C} GS013_ter-1 | ATGTCTAAATTTATTGAAGAArTAmAAACTGATGAATGGGAAGT AGAAACTCCTTCTGGATGGCAATCTTTTTCTGGGGTAGGAAAAA CTATAGAATATGAAGAATGGGAGGTTGTAACCGAAACTGGAAA ATCTCTTATATGTGCAGATAAACACATCTTATTAAATGATAAAT GGCAAGAAGTTTATTGTGArGATTGTTCCATTGATGACTGTATAC AAACAAAAAATkGCGCAGAAAAAATATTACaATTAAAAAAAAC ATCAAGAATTyyTAATATGTATGATCTTCTTGATGTTGATAATGG TAATATATTTTACAGTAATGAAATAGTTTCACACAATTCTACAA CTGTTGTC |
| 182 | Int^{N} GS020_ter-7 | CVDGSSIITIKNKETNLIEKITIEELYNKLL |
| 183 | Int^{C} GS020_ter 7 | MKTNTKYEILGPEGFVDFKGIQKLKKKTRQIFFECGLTLRASYNHKI YDYFGDEIIIKDVVIGSKIKSHNGYLIVNSIKDFDYESDVYDVIDSGD SHLYYTNNIVSHN |
| 184 | Int^{N} GS020_ter-7 flanking sequence | sqele |
| 185 | Int^{C} GS020_ter-7 flanking sequence | cnflg |
| 186 | DNA Int^{N} GS020_ter-7 | TCGCAgGAATTAGAgtGTgtTGATGGTTCCTCAatTATaACTATAAA AAACAAAGAGACAAATTTAATAGAAAAAATAACAATAGAAGAA TTATACAATAAATTGTTATAG |
| 187 | DNA Int^{C} GS020_ter-7 | ATGAAAACTAACACAAAATATGAAATTTTAGGTCCTGAAGGATT CGTCGATTTCAAAGGTATTCAAAAATTAAAAAAGAAAACTAGA CAAATTTTTTTGAGTGTGGACTAACATTACGAGCAAGTTATAA CCACAAGATTTACGATTATTTTGGGGATGAAATTATAATTAAAG ACGTAGTTATTGGTAGTAAAATCAAATCACATAATGGTTATTTA ATTGTTAATAGTATCAAGGATTTTGATTATGAAAGTGACGTATA TGACGTTATTGATTCAGGTGATTCACATTTATACTACACAAACA ACATTGTTTCTCATAATTGTAATTTTCTTGGG |
| 188 | Int^{N} WT AceL-TerL-11 original DNA sequence | TGTGTTTATGGTGATACAATGGTTGAAACAGAAGATGGTAAAAT AAAAATAGAAGATTTATATAAAGGTTGGCA |
| 189 | Int^{C} WT AceL-TerL-11 original DNA sequence | ATGTTTAGAACTAATACAAATAATATAAAAATATTAAGTCCAAA TGGATTTTCTAATTTTAATGGTATTCAAAAGGTTGAAAGAAACC TTTATCAACACATTATCTTTGATGATGATACTGAAATAAAAACTT CCATTAATCATCCTTTTGGTAAAGATAAAATATTAGCAAGAGAT GTAAAAGTAGGAGATTATTTAAATAGTAAAAAGGTATTATATAA TGAGTTGGTTAATGAAAATATATTTTTATATGATCCTATAAATGT AGAAAAAGAAAGTTTATATATTACTAATGGTGTTGTTTCTCATA ATTGT |
| 190 | Int^{N} WT AceL-TerL-11 codon optimised DNA sequence | TGCGTGTATGGCGATACTATGGTGGAAACCGAAGATGGCAAAA TTAAAATTGAAGATCTGTATAAACGTCTGGCC |
| 191 | Int^{C} WT AceL-TerL-11 codon optimised DNA sequence | GGCATGTTTCGTACCAACACCAACAACATTAAAATTCTGAGCCC GAACGGCTTTAGCAACTTTAACGGCATTCAGAAAGTGGAACGTA ACCTGTATCAGCATATTATTTTTGATGATGATACCGAAATTAAA ACCAGCATTAACCATCCGTTTGGCAAAGATAAAATTCTGGCGCG TGATGTGAAAGTGGGCGATTATCTGAACAGCAAAAAAGTGCTGT ATAACGAACTGGTGAACGAAAACATTTTTCTGTATGATCCGATT AACGTGGAAAAAGAAAGCCTGTATATTACCAACGGCGTGGTGA GCCATAAC |
| 192 | DNA Int^{N} GS033_TerA-6 codon optimised DNA sequence | AGCATTAGCCAGGAATCCTATATCAACATTGAGGTGAACGGGA AAGTGGAAACCATCAAAATCGGCGACCTGTATAAAAAACTGTC CTTCAACGAGCGTAAATTCAACGAG |
| 193 | DNA Int^{C} GS033_TerA-6 codon optimised DNA sequence | ATGAAACTGCCGGAGAGCGTGGTGAAAAACAACATCAACCTGA AAATCGAAACCCCGTATGGCTTTGAGAACTTCTATGGTGTGAAC AAAATCAAAAAAGACAAATATATCCACCTGGAGTTTACCAACG GCGAAAAACTGAAATGCTCCCTGGATCATCCTCTGTCTACCATT GACGGCATCGTTAAAGCGAAAGATCTGGACAAATATACCGAGG TCTATACGAAATTTGGTGGCTGCTTTCTGAAAAAATCCAAAGTG ATCAACGAGTCCATCGAGCTGTATGATATCGTGAACTCTGGGCT GAAACACCTGTATTATTCCAACAATATTATCAGTCACAAC |
| 194 | Int^{C} WTAceL-TerL-3 | MFITNTDNIKILSPSGFSNFNGIQKVERNLYQHIIFDDESEIKTSINHPF GKNKILARNVKVGDYLSSKKVLYNELVNEKIFLYDPINVEKENLYI TNGVVSHN |
| 195 | Int^{C} WTAceL-TerL-4 | MFRTNTDNIKILSPSGFSIFNGIQKVERDLYQHIIFDDKSEIKTSINHPF GKDKILARNIKVGDYLNSKKVLYNELVAEKITLYDPINVEKENLYIT NGVISHN |
| 196 | Int^{C} WTAceL-TerL-5 | MFRTNTDNIKILSPSGFSNFNGIQKVERDLYQHIIFDDKSEIKTSINHP FGKDKILARNIKVGDYLNSKKVLYNELVNEKITLYDPINVEKENLYI TNGVISHN |

## Claims

1. Isolated polypeptide comprising at least one intein or at least one intein fragment of said intein, wherein said intein is a naturally split intein with a N-terminal intein fragment split after 14-60 amino acids from the intein' s N-terminal position.

2. The isolated polypeptide of claim 1 wherein said at least one intein fragment, is selected from the group comprising:
a) a N-terminal intein fragment having 100% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, or,
b) a N-terminal intein fragment having at least 70%, or 80% or 85% or 90% or 95% sequence identity with SEQ ID NO: 2, 3, 4, 5, 28, 38, 44, 50, 56, 62, 68, 74, 80, 86, 92, 98, 104, 110, 116, 122, 128, 134, 140, 146, 152, 158, 164, 170, 176 or 182, and/or
c) a C-terminal intein fragment having 100% sequence identity with SEQ ID NO: 6, 27, 39, 45, 51, 57, 63, 69, 75, 81, 87, 93, 99, 105, 111, 117, 123, 129, 135, 141, 147, 153, 159, 165, 171, 177, 183, 194, 195 or 196 or,
d) a C-terminal intein fragment having at least 70%, or 80% or 85% or 90% or 95% sequence identity with SEQ ID NO: 6, 27, 39, 45, 51, 57, 63, 69, 75, 81, 87, 93, 99, 105, 111, 117, 123, 129, 135, 141, 147, 153, 159, 165, 171, 177, 183, 194, 195 or 196, or
e) wherein the at least one intein has at least 70%, or 80% or 85% or 90% or 95% sequence identity with SEQ ID NO:1 or 26.

3. Isolated polypeptide according to claims 1 or 2, wherein said polypeptide comprises at least one N-terminal intein fragment and at least one C-terminal intein fragment, wherein
1) if said at least one N-terminal intein fragment is selected from SEQ ID NO: 5 then said at least one C-terminal intein fragment is selected from SEQ ID NO: 6, or
2) if said at least one N-terminal intein fragment is selected from SEQ ID NO: 5, 28-33 then said at least one C-terminal intein fragment is selected from SEQ ID NO: 27, or
3) if said at least one N-terminal intein fragment is selected from SEQ ID NO: 38 then said at least one C-terminal intein fragment is selected from SEQ ID NO: 39, or
4) if said at least one N-terminal intein fragment is selected from SEQ ID NO: 44 then said at least one C-terminal intein fragment is selected from SEQ ID NO: 45, or
5) if said at least one N-terminal intein fragment is selected from SEQ ID NO: 50 then said at least one C-terminal intein fragment is selected from SEQ ID NO: 51, or
6) if said at least one N-terminal intein fragment is selected from SEQ ID NO: 56 then said at least one C-terminal intein fragment is selected from SEQ ID NO: 57, or
7) if said at least one N-terminal intein fragment is selected from SEQ ID NO: 62 then said at least one C-terminal intein fragment is selected from SEQ ID NO: 63, or
8) if said at least one N-terminal intein fragment is selected from SEQ ID NO: 68 then said at least one C-terminal intein fragment is selected from SEQ ID NO: 69, or
9) if said at least one N-terminal intein fragment is selected from SEQ ID NO: 74 then said at least one C-terminal intein fragment is selected from SEQ ID NO: 75, or
10) if said at least one N-terminal intein fragment is selected from SEQ ID NO: 80 then said at least one C-terminal intein fragment is selected from SEQ ID NO: 81, or
11) if said at least one N-terminal intein fragment is selected from SEQ ID NO: 86 then said at least one C-terminal intein fragment is selected from SEQ ID NO: 87, or
12) if said at least one N-terminal intein fragment is selected from SEQ ID NO: 92 then said at least one C-terminal intein fragment is selected from SEQ ID NO: 93, or
13) if said at least one N-terminal intein fragment is selected from SEQ ID NO: 98 then said at least one C-terminal intein fragment is selected from SEQ ID NO: 99, or
14) if said at least one N-terminal intein fragment is selected from SEQ ID NO: 104 then said at least one C-terminal intein fragment is selected from SEQ ID NO: 105, or
15) if said at least one N-terminal intein fragment is selected from SEQ ID NO: 110 then said at least one C-terminal intein fragment is selected from SEQ ID NO: 111, or
16) if said at least one N-terminal intein fragment is selected from SEQ ID NO: 116 then said at least one C-terminal intein fragment is selected from SEQ ID NO: 117, or
17) if said at least one N-terminal intein fragment is selected from SEQ ID NO: 122 then said at least one C-terminal intein fragment is selected from SEQ ID NO: 123, or
18) if said at least one N-terminal intein fragment is selected from SEQ ID NO: 128 then said at least one C-terminal intein fragment is selected from SEQ ID NO: 129, or
19) if said at least one N-terminal intein fragment is selected from SEQ ID NO: 134 then said at least one C-terminal intein fragment is selected from SEQ ID NO: 135, or
20) if said at least one N-terminal intein fragment is selected from SEQ ID NO: 140 then said at least one C-terminal intein fragment is selected from SEQ ID NO: 141, or
21) if said at least one N-terminal intein fragment is selected from SEQ ID NO: 146 then said at least one C-terminal intein fragment is selected from SEQ ID NO: 147, or
22) if said at least one N-terminal intein fragment is selected from SEQ ID NO: 152 then said at least one C-terminal intein fragment is selected from SEQ ID NO: 153, or
23) if said at least one N-terminal intein fragment is selected from SEQ ID NO: 158 then said at least one C-terminal intein fragment is selected from SEQ ID NO: 159, or
24) if said at least one N-terminal intein fragment is selected from SEQ ID NO: 164 then said at least one C-terminal intein fragment is selected from SEQ ID NO: 165, or
25) if said at least one N-terminal intein fragment is selected from SEQ ID NO: 170 then said at least one C-terminal intein fragment is selected from SEQ ID NO: 171, or
26) if said at least one N-terminal intein fragment is selected from SEQ ID NO: 176 then said at least one C-terminal intein fragment is selected from SEQ ID NO: 177, or
27) if said at least one N-terminal intein fragment is selected from SEQ ID NO: 182 then said at least one C-terminal intein fragment is selected from SEQ ID NO: 183, or
28) if said at least one N-terminal intein fragment is selected from SEQ ID NO: 2 then said at least one C-terminal intein fragment is selected from SEQ ID NO: 194, or
29) if said at least one N-terminal intein fragment is selected from SEQ ID NO: 3 then said at least one C-terminal intein fragment is selected from SEQ ID NO: 195, or
30) if said at least one N-terminal intein fragment is selected from SEQ ID NO: 3 then said at least one C-terminal intein fragment is selected from SEQ ID NO: 196.

4. Isolated polypeptide according to anyone of the preceding claims, wherein said polypeptide at the N-terminal end and/or at the C-terminal end of said at least one intein fragment further comprises a sequence selected from:
1) in the case of SEQ ID NO: 5 and/or 6; SEQ ID NO: 25 and/or 26, respectively,
2) in the case of SEQ ID NO: 5, 28-33 and/or 27 SEQ ID NO: 34 and/or 35, respectively,
3) in the case of SEQ ID NO:38 and/or 39; SEQ ID NO: 40 and/or 41, respectively,
4) in the case of SEQ ID NO:44 and/or 45 ;SEQ ID NO: 46 and/or 47, respectively,
5) in the case of SEQ ID NO:50 and/or 51; SEQ ID NO: 52 and/or 53, respectively,
6) in the case of SEQ ID NO:56 and/or 57; SEQ ID NO: 58 and/or 59, respectively,
7) in the case of SEQ ID NO:62 and/or 63; SEQ ID NO: 64 and/or 65, respectively,
8) in the case of SEQ ID NO:68 and/or 69; SEQ ID NO: 70 and/or 71, respectively,
9) in the case of SEQ ID NO:74 and/or 75; SEQ ID NO: 76 and/or 77, respectively,
10) in the case of SEQ ID NO:80 and/or 81; SEQ ID NO: 82 and/or 83, respectively,
11) in the case of SEQ ID NO:86 and/or 87; SEQ ID NO: 88 and/or 89, respectively,
12) in the case of SEQ ID NO:92 and/or 93; SEQ ID NO: 94 and/or 95, respectively,
13) in the case of SEQ ID NO: 98 and/or 99; SEQ ID NO: 100 and/or 101, respectively,
14) in the case of SEQ ID NO: 104 and/or 105; SEQ ID NO: 106 and/or 107, respectively,
15) in the case of SEQ ID NO: 110 and/or 111; SEQ ID NO: 112 and/or 113, respectively,
16) in the case of SEQ ID NO:116 and/or 117; SEQ ID NO: 118 and/or 119, respectively,
17) in the case of SEQ ID NO:122 and/or 123; SEQ ID NO: 124 and/or 125, respectively,
18) in the case of SEQ ID NO:128 and/or 129; SEQ ID NO: 130 and/or 131, respectively,
19) in the case of SEQ ID NO:134 and/or 135; SEQ ID NO: 136 and/or 137, respectively,
20) in the case of SEQ ID NO:140 and/or 141; SEQ ID NO: 142 and/or 143, respectively,
21) in the case of SEQ ID NO:146 and/or 147; SEQ ID NO: 148 and/or 149, respectively,
22) in the case of SEQ ID NO:152 and/or 153; SEQ ID NO: 154 and/or 155, respectively,
23) in the case of SEQ ID NO:158 and/or 159; SEQ ID NO: 160 and/or 161, respectively,
24) in the case of SEQ ID NO:164 and/or 165; SEQ ID NO: 166 and/or 167, respectively,
25) in the case of SEQ ID NO:170 and/or 171; SEQ ID NO: 172 and/or 173, respectively,
26) in the case of SEQ ID NO:176 and/or 177; SEQ ID NO: 178 and/or 179, respectively,
27) in the case of SEQ ID NO:182 and/or 183; SEQ ID NO: 184 and/or 185, respectively.

5. Isolated polypeptide according to anyone of the preceding claims, wherein the N-terminal intein fragment has 100% sequence identity with a sequence selected from sequences comprising SEQ ID NO: 5, 11 or 12 or has at least 90% or 95% sequence identity with a sequence selected from sequences comprising SEQ ID NO: 5, 11 or 12 and/or,
wherein the C-terminal intein fragment has 100% sequence identity with a sequence selected from sequences comprising SEQ ID NO:6 or 13-18 or has at least 90% or 95% sequence identity with a sequence selected from sequences comprising SEQ ID NO:6 or 13-18.

6. Isolated polypeptide according to anyone of the preceding claims wherein the N-terminal intein fragment is selected from sequences comprising SEQ ID NO: 5, 11 or 12 and/or,
wherein the C-terminal intein fragment is selected from sequences comprising SEQ ID NO:6 or 13-18.

7. Isolated polypeptide according to anyone of the preceding claims, wherein the polypeptide comprises the N-terminal intein fragment with SEQ ID NO: 5 or 12 and the C-terminal intein fragment with SEQ ID NO: 13.

8. Isolated polypeptide according to anyone of the preceding claims, wherein the N-terminal intein fragment has 100% sequence identity with a sequence selected from sequences comprising SEQ ID NO: 5, 28-33 or has at least 90% or 95% sequence identity with a sequence selected from sequences comprising SEQ ID NOs: 28-33 and/or,
wherein the C-terminal intein fragment has 100% sequence identity with SEQ ID NO: 27 or has at least 90% or 95% sequence identity with SEQ ID NO: 27.

9. Isolated polypeptide according to anyone of the preceding claims, wherein the polypeptide further comprises at least one C-terminal extein and/or at least one N-terminal extein sequence.

10. Two isolated polypeptides according to anyone of the preceding claims,
wherein one of the isolated polypeptides comprises least one heterologous N-terminal extein fused to a N-terminal intein fragment having at least 70%, or 80% or 85% or 90% or 95% or 99% sequence identity with SEQ ID NO: 5, 28, 38, 44, 50, 56, 62, 68, 74, 80, 86, 92, 98, 104, 110, 116, 122, 128, 134, 140, 146, 152, 158, 164, 170, 176 or 182 and,
wherein the second one of the isolated polypeptides comprises at least one C-terminal extein sequence fused to a C-terminal intein fragment having at least 70%, or 80% or 85% or 90% or 95% or 99% sequence identity with SEQ ID NO: 6, 27, 39, 45, 51, 57, 63, 69, 75, 81, 87, 93, 99, 105, 111, 117, 123, 129, 135, 141, 147, 153, 159, 165, 171, 177 or 183 .

11. Isolated polypeptide according to anyone of the preceding claims, wherein the polypeptide further comprises at least one component selected from a solubility factor, a marker, a linker, an epitope, an affinity tag, a fluorophore or a fluorescent protein, a toxic compound or protein and a small-molecule or a small-molecule binding protein.

12. Isolated nucleic acid molecule comprising a nucleotide sequence encoding for an isolated polypeptide of claims 1 to 11 or a homolog, variant or complement thereof.

13. Method using a polypeptide according to claims 1-11 or a nucleic acid molecule according to claim 12, wherein the method is selected from the group consisting of modification of a protein, protein lipidation, protein immobilization, protein backbone semi-synthesis, regioselective protein side chain modification and artificial control of protein splicing by light.

14. Use of a polypeptide according to claims 1-11 or a nucleic acid molecule according to claim 12, wherein the use is selected from the group consisting of modification of a protein, protein lipidation, protein immobilization, protein backbone semisynthesis and use as molecular switch.

15. Kit comprising at least one polypeptide according to claims 1-11 or a nucleic acid molecule according to claim 12.
